# EUROPEAN PATENT SPECIFICATION

(11) **EP 2 660 249 B1**
(45) Date of publication and mention of the grant of the patent: **10.07.2019**
(21) Application number: 11830776.8
(22) Date of filing: 07.10.2011
(51) Int. Cl.: C07K 14/46, G01N 33/53, G01N 33/536

(54) **PEPTIDE DERIVATIVE AND USE THEREOF**
PEPTIDDERIVAT UND SEINE VERWENDUNG
DÉRIVÉ PEPTIDIQUE ET SON UTILISATION

(30) Priority: 14.06.2011 US 201161496831 P; 08.10.2010 US 391374 P; 08.10.2010 JP 2010229009
(43) Date of publication of application: 06.11.2013
(73) Proprietor: Kyoto University, Sakyo-ku Kyoto-shi Kyoto 606-8501 (JP); ARKRAY, Inc., Minami-ku Kyoto-shi Kyoto 601-8045 (JP)
(72) Inventor: SAJI, Hideo, Kyoto-shi, Kyoto 606-8501 (JP); INAGAKI, Nobuya, Kyoto-shi, Kyoto 606-8501 (JP); TOYODA, Kentaro, Kyoto-shi, Kyoto 606-8501 (JP); KIMURA, Hiroyuki, Kyoto-shi, Kyoto 606-8501 (JP); HIRAO, Konomu, Kamigyo-ku, Kyoto-shi, Kyoto 602-0008 (JP); MATSUDA, Hirokazu, Kamigyo-ku, Kyoto-shi, Kyoto 602-0008 (JP)
(74) Representative: Dehns
(86) International application number: PCT/JP2011/073234
(87) International publication number: WO 2012/046845

(56) References cited:
- EP-A1- 2 418 216
- EP-A1- 2 484 386
- WO-A2-2010/107256
- WICKI ANDREAS ET AL: "[Lys40(Ahx-DTPA-111In)NH2]-Exendin-4 is a highly efficient radiotherapeutic for glucagon-like peptide-1 receptor-targeted therapy for insulinoma", CLINICAL CANCER RESEARCH, THE AMERICAN ASSOCIATION FOR CANCER RESEARCH, US, vol. 13, no. 12, 15 June 2007 (2007-06-15) , pages 3696-3705, XP002561708, ISSN: 1078-0432, DOI: 10.1158/1078-0432.CCR-06-2965
- DATABASE MICAD [Online] 06 April 2010 LEUNG K.: '3-(4-Hydroxy-3-[125I]iodophenyl) propionate-exendin(9-39). (http://ncbi.nlm.nih.gov/books/NBK44818)', XP003032711 Retrieved from NCBI (bookshelf) Database accession no. NBK44818
- MUKAI E. ET AL.: 'GLP-1 receptor antagonist as a potential probe for pancreatic beta-cell imaging.' BIOCHEM. BIOPHYS. RES. COMMUN. vol. 389, no. 3, 20 November 2009, pages 523 - 526, XP055086413
- WILD D. ET AL.: 'Exendin-4-based radiopharmaceuticals for glucagonlike peptide-1 receptor PET/CT and SPECT/CT.' J. NUCL. MED. vol. 51, no. 7, July 2010, pages 1059 - 1067, XP055037208
- DATABASE MICAD [Online] 20 February 2008 CHENG K.T.: '[18F]FB-NH-mini-PEG-E{E[c(RGDyK)]2}2. (http://www.ncbi.nlm.nih.gov/books/NBK23235 )', XP003032712 Retrieved from NCBI (bookshelf) Database accession no. NBK23235
- LIU Z. ET AL.: '(68)Ga-labeled cyclic RGD dimers with Gly3 and PEG4 linkers: promising agents for tumor integrin ALPHAvBETA3 PET imaging' EUR. J. NUCL. MED. MOL. IMAGING vol. 36, no. 6, June 2009, pages 947 - 957, XP019706091
- MELENDEZ-ALAFORT L. ET AL.: 'Detection of sites of infection in mice using 99mTc-labeled PN(2)S-PEG conjugated to UBI and 99mTc-UBI: a comparative biodistribution study.' NUCL. MED. BIOL. vol. 36, no. 1, January 2009, pages 57 - 64, XP025923965
- BLAKELY B. ET AL.: 'Formulation and characterization of radio-opaque conjugated in situ gelling materials.' J. BIOMED. MATER. RES. B APPL. BIOMATER. vol. 93B, no. 1, April 2010, pages 9 - 17, XP055086420
- Insoo Kim ET AL: "Synthesis and Evaluation of Human Serum Albumin-Modified Exendin-4 Conjugate via Heterobifunctional Polyethylene Glycol Linkage with Protracted Hypoglycemic Efficacy", Bioconjugate Chemistry, vol. 21, no. 8, 18 August 2010 (2010-08-18), pages 1513-1519, XP055049140, ISSN: 1043-1802, DOI: 10.1021/bc100143c

## Description

### Technical Field

The present invention relates to a peptide derivative, and to the use of the same.

### Background Art

The estimated number of type-II diabetics in Japan exceeds 8,800,000 according to the statistic in fiscal 2007, and has been increasing further continuously, as compared with fiscal 2002. As a measure against this increase, interventions for preventing diabetes from developing have been made based on the glucose tolerance test, resulting, however, in unsatisfactory effects. The cause is as follows: at such a borderline stage that functional abnormalities are found by the glucose tolerance test, disorders of pancreatic islets have already advanced to a high degree, and this stage possibly is too late as a time for starting interventions.

Recently, it has been reported at home and overseas that even in type-II diabetics the amount of pancreatic islets has already decreased upon development of the disease, and it has been considered that a further decrease in pancreatic β-cells after development is one of the resistance factors against treatment for type-II diabetics. Therefore, the detection of the amount of pancreatic islets and/or the amount of pancreatic β-cells, enables the possibility of clarification of pathogenesis, ultra-early diagnosis, and prevention of development of type-I and type-II diabetes. For this purpose, molecular probes for imaging that allow determination of the amount of pancreatic islets and/or the amount of pancreatic β-cells have been researched and developed.

In designing a molecular probe for imaging, various target molecules in pancreatic cells, particularly functional proteins specific in β-cells, are being researched. Among these, GLP-1R (glucagon-like peptide-1 receptor) is being researched as a target molecule; GLP-1R is distributed in pancreatic β-cells, and is a seven-transmembrane G protein coupled receptor.

The following molecular probes for imaging that use GLP-1R as a target molecule, have been researched: a peptide derivative of GLP-1 having a C-terminus to which a labeling molecule is bound; a peptide derivative of exendin-3 having a C-terminus to which a labeling molecule is bound; and a peptide derivative of exendin-4 having a C-terminus to which a labeling molecule is bound (e.g., Patent Document 1; Non-Patent Document 1; and Non-Patent Document 2). In addition, a derivative of exendin(9-39) is proposed (e.g., Patent Document 2; Non-Patent Document 2; and Non-Patent Document 3).

### Prior Art Document

### Patent Document

[Patent Document 1] JP 2008-511557 A
[Patent Document 2] WO 2010/032509

### Non-Patent Document

[Non-Patent Document 1]: M. Gotthardt et al., "A new technique for in vivo imaging of specific GLP-1 binding sites": First results in small rodents, Regulatory Peptides 137 (2006) 162-267
[Non-Patent Document 2]:M. Beche et al., "Are radiolabeled GLP-1 receptor antagonists useful for scintigraphy?": 2009 SNM Annual Meeting, abstract, Oral Presentations No.327
[Non-Patent Document 3]:H. Kimura et al. Development of in vivo imaging agents targeting a glucagons-like peptide-1 receptor (GLP-1R) in pancreatic islets. 2009 SNM Annual Meeting, abstract, Oral Presentations No.326

### Disclosure of Invention

### Problem to be Solved by the Invention

The present invention provides a peptide derivative useful for imaging pancreatic β-cells.

The present invention relates to a peptide derivative represented by the following general formula (I): 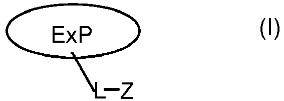 where ExP represents a polypeptide that is represented by an amino acid sequence of the following formula (1) and contains completely or partially the amino acid sequence of exendin-4 (HGEGTFTSDLSKQMEEEAVRLFIEWLKNGGPSSGAPPPS (SEQ ID NO.1)),

Ex4(x-y)-Kₙ (1)

where Ex4(x-y) represents the amino acid sequence of SEQ ID No.1 between positions x and y, x is an integer from 1 to 9, y is an integer from 30 to 39, K represents lysine, and n is 0 or 1,
the α-amino group at the N-terminus of the polypeptide ExP is unmodified or is modified with a modifying group having no electric charge,
the carboxyl group at the C-terminus of the polypeptide ExP is amidated, and
the -L-Z group represents a group represented by the following formula (II) that is bound to an amino acid side chain of a lysine that corresponds to position 12 or 40 of the polypeptide represented by the amino acid sequence of formula (1), 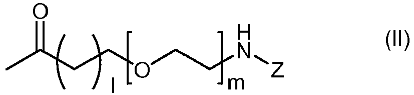 where l is 0,1 or 2 and m is an integer from 3 to 30 when n in formula (1) is 1,1 is an integer from 0 to 8 and m is an integer from 2 to 8 or 4 to 30 when n in formula (1) is 0, and Z represents a labeling group containing a radionuclide or isotope thereof.

Also disclosed herein is a peptide derivative as the labeling precursor of the peptide derivative of the present invention (hereinafter simply also referred to as the "labeling precursor"), which is represented by the following general formula (IV) 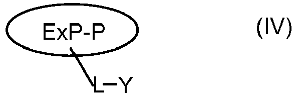 where ExP-P represents a polypeptide that is represented by an amino acid sequence of the following formula (1) and contains completely or partially the amino acid sequence of exendin-4 (HGEGTFTSDLSKQMEEEAVRLFIEWLKNGGPSSGAPPPS (SEQ ID NO.1)),

Ex4(x-y)-Kₙ (1)

where Ex4(x-y) represents the amino acid sequence of SEQ ID No.1 between positions x and y, x is an integer from 1 to 9, y is an integer from 30 to 39, K represents lysine, and n is 0 or 1,
the α-amino group at the N-terminus of the polypeptide ExP-P is bound to a protecting group, modified with a modifying group having no electric charge, or not modified,
the carboxyl group at the C-terminus of the polypeptide ExP-P is amidated, and
the -L-Y group represents a group represented by the following formula (V) that is bound to an amino acid side chain of a lysine that corresponds to position 12 or 40 of the polypeptide represented by formula (1), 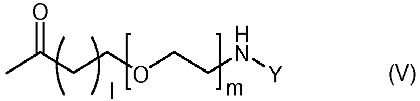 where is 0,1 or 2 and m is an integer from 3 to 30 when n in formula (1) is 1,1 is an integer from 0 to 8 and m is an integer from 2 to 8 or 4 to 30 when n in formula (1) is 0, and Y represents a hydrogen atom or radioactive labeling introduction group.

### Effects of the Invention

According to the present invention, it is possible to provide a peptide derivative useful for imaging of pancreatic β-cells, preferably imaging of GLP-1R of pancreatic β-cells.

### Brief Description of Drawings

FIG. 1 is a graph showing exemplary time-variations of biodistribution of a peptide derivative of Example 1.
FIG. 2 is a graph showing exemplary time-variations of biodistribution of a peptide derivative of Reference Example 1.
FIG. 3 shows images showing exemplary results of a two-dimensional imaging analysis using the peptide derivative of Example 1.
FIG. 4 shows images showing exemplary results of SPECT imaging using a peptide derivative of Example 2.
FIG. 5 is a graph showing exemplary time-variations of biodistribution of a peptide derivative of Example 3.
FIG. 6 is a graph showing exemplary time-variations of biodistribution of a peptide derivative of Reference Example 2.
FIG. 7 shows images showing exemplary results of a two-dimensional imaging analysis using the peptide derivative of Example 3.
FIG. 8 shows images showing exemplary results of SPECT imaging using a peptide derivative of Example 4.
FIG. 9 is a graph showing exemplary time-variations of biodistribution in Example 5.
FIG. 10 shows images showing exemplary results of a two-dimensional imaging analysis in Example 5.
FIG. 11 shows an image showing exemplary results of SPECT imaging in Example 6.
FIG. 12 is a graph showing exemplary time-variations of biodistribution in Example 7.
FIG. 13 shows images showing exemplary results of a two-dimensional imaging analysis in Example 7.
FIG. 14 shows an image showing exemplary results of SPECT imaging in Example 8.
FIG. 15 is a graph showing exemplary time-variations of biodistribution in Example 9.
FIG. 16 shows images showing exemplary results of a two-dimensional imaging analysis in Example 9.
FIG. 17 is a graph showing exemplary time-variations of biodistribution in Example 10.
FIG. 18 is a graph showing, by way of example, a percentage of radioactive accumulation per gram of each organ in Example 11.

### Description of the Invention

The diameter of a pancreatic islet is, for example, approximately 50 to 500 µm in the case of a human. In order to non-invasively image or quantify such pancreatic islets *in vivo,* a molecular probe, for example, is considered to be necessary that can accumulate specifically in pancreatic islets, thereby providing contrast between pancreatic islets and surrounding tissues. The aforementioned various research and development of molecular probes has been performed, However, to allow clearer imaging or more accurate quantification, a new molecular probe that accumulates more specifically in the pancreas and provides a desired contrast (S/N ratio) with respect to the organs surrounding the pancreas is desirable.

The present invention is based on the finding that, with the peptide derivative represented by general formula (I), it is possible to provide a molecular probe that exhibits improved specificity for pancreatic β-cells, preferably for GLP-1R of pancreatic β-cells, and/or an improved blood clearance, provides a desired S/N ratio and is useful for imaging by positron emission tomography (PET) or single photon emission computed tomography (SPECT).

Also, the present invention is based on the finding that, with use of the peptide derivative represented by general formula (IV) as the labeling precursor, the above-described peptide derivative represented by general formula (I) can be produced with ease, and moreover, yields at the time of labeling can be improved and the time involved in the labeling can be reduced.

In other words, the present invention relates to the following:
[1] A peptide derivative represented by the following general formula (I) 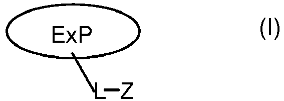 where ExP represents a polypeptide that is represented by an amino acid sequence of the following formula (1) and contains completely or partially the amino acid sequence of exendin-4 (HGEGTFTSDLSKQMEEEAVRLFIEWLKNGGPSSGAPPPS (SEQ ID NO.1)),

   Ex4(x-y)-Kₙ (1)

   where Ex4(x-y) represents the amino acid sequence of SEQ ID No.1 between positions x and y, x is an integer from 1 to 9, y is an integer from 30 to 39, K represents lysine, and n is 0 or 1,
   the α-amino group at the N-terminus of the polypeptide ExP is not modified or is modified with a modifying group having no electric charge,
   the carboxyl group at the C-terminus of the polypeptide ExP is amidated, and
   the -L-Z group represents a group represented by the following formula (II) that is bound to an amino acid side chain of a lysine that corresponds to position 12 or 40 of the polypeptide represented by the amino acid sequence of formula (1), 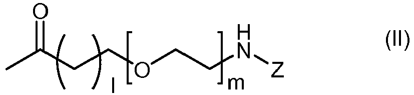 where is 0,1 or 2 and m is an integer from 3 to 30 when n in formula (1) is 1,1 is an integer from 0 to 8 and m is an integer from 2 to 8 or 4 to 30 when n in formula (1) is 0, and Z represents a labeling group containing a radionuclide or isotope thereof.
[2] The peptide derivative according to [1], wherein Ex4(x-y)-Kₙ is any of the amino acid sequences of the following formulae (2) to (5):
   HGEGTFTSDLSKQMEEEAVRLFIEWLKNGGPSSGAPPPS (2) (SEQ ID NO.2)
   HGEGTFTSDLSKQMEEEAVRLFIEWLKNGGPSSGAPPPSK (3) (SEQ ID NO.3)
   DLSKQMEEEAVRLFIEWLKNGGPSSGAPPPS (4) (SEQ ID NO.4)
   DLSKQMEEEAVRLFIEWLKNGGPSSGAPPPSK (5) (SEQ ID NO. 5).
[3] The peptide derivative according to [1] or [2], wherein Z is a group represented by the following formula (III), 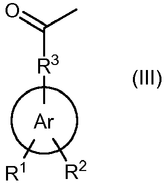 where Ar represents an aromatic hydrocarbon group or aromatic heterocyclic group, R¹ represents a substituent containing a radionuclide or isotope thereof, R² represents a hydrogen atom or one or more substituents different from the substituent represented by R¹, and R³ represents a bond, alkylene group having 1 to 6 carbon atoms or oxyalkylene group having 1 to 6 carbon atoms.
[4] The peptide derivative according to any one of [1] to [3], where Z represents a labeling group containing a radionuclide.
[5] The peptide derivative according to any one of [1] to [4], wherein n in formula (1) is 0, and is an integer from 0 to 8, preferably 0 to 5, and m is an integer from 2 to 8 or 4 to 30.
[6] A composition comprising the peptide derivative according to any one of [1] to [5].
[7] Use of the peptide derivative according to any one of [1] to [5] for imaging of pancreatic islets, preferably for imaging pancreatic β-cells.
[8] A method for imaging pancreatic β-cells, comprising detecting a signal of a radionuclide of the peptide derivative according to [4] from an analyte to which the peptide derivative has been administered in advance.
[9] The method according to [8], further comprising reconfiguring the detected signal to convert the signal into an image, and displaying the image.
[10] A method for determining the amount of pancreatic islets, the method comprising:
   detecting a signal of the peptide derivative according to [4] from an analyte to which the peptide derivative has been administered;
   and calculating the amount of pancreatic islets from the detected signal of the peptide derivative.
[11] The method according to [10], further comprising presenting the calculated amount of pancreatic islets.
[12] The peptide derivative according to [4] for use in diagnosis of diabetes, wherein in said diagnosis said peptide derivative is used in imaging of pancreatic islets.
[13] A method for producing a peptide derivative as defined in any one of [1] to [5], said method comprising labelling a peptide derivative represented by the following general formula (IV), 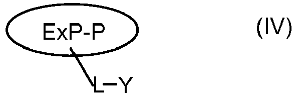 where ExP-P represents a polypeptide that is represented by an amino acid sequence of the following formula (1) and contains completely or partially the amino acid sequence of exendin-4 (HGEGTFTSDLSKQMEEEAVRLFIEWLKNGGPSSGAPPPS (SEQ ID NO.1)),

   Ex4(x-y)-Kₙ (1)

   where Ex4(x-y) represents the amino acid sequence of SEQ ID No.1 between positions x and y, x is an integer from 1 to 9, y is an integer from 30 to 39, K represents lysine, and n is 0 or 1,
   the α-amino group at the N-terminus of the polypeptide ExP-P is bound to a protecting group, modified with a modifying group having no electric charge, or not modified,
   the carboxyl group at the C-terminus of the polypeptide ExP-P is amidated, and
   the -L-Y group represents a group represented by the following formula (V) that is bound to an amino acid side chain of a lysine that corresponds to position 12 or 40 of the polypeptide represented by formula (1), 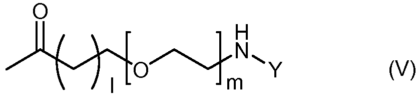 where 1 is 0,1 or 2 and m is an integer from 3 to 30 when n in formula (1) is 1,1 is an integer from 0 to 8 and m is an integer from 2 to 8 or 4 to 30 when n in formula (1) is 0, and Y represents a hydrogen atom or radioactive labeling introduction group.
[14] The method according to [13], wherein the radioactive labeling group is a chelating site selected from the group consisting of diethylenetriaminepentaacetic acid (DTPA), 6-hydrazinopyridine-3-carboxylic acid (HYNIC), tetraazacyclododecanetetraacetic acid (DOTA), dithisosemicarbazone (DTS), diaminedithiol (DADT), mercaptoacetylglycylglycylglycine (MAG3), monoamidemonoaminedithiol (MAMA), diamidedithiol (DADS), and propylene diamine dioxime (PnAO), or a group represented by formula (VI), 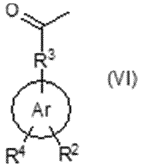 where Ar represents an aromatic hydrocarbon group or aromatic heterocyclic group, R⁴ represents a substituent having a mesylate group, tosylate group, triflate group, ⁸⁰Br, ¹²⁷I, chlorine atom, nitro group, trimethylammonium group, tin atom, alkyltin group, or alkylsilicon group, R² represents a hydrogen atom or one or more substituents different from the substituent represented by R⁴, and R³ represents a bond, alkylene group having 1 to 6 carbon atoms or oxyalkylene group having 1 to 6 carbon atoms.
[15] The method according to [13] or [14], wherein Ex4(x-y)-Kₙ is any of the amino acid sequences of the following formulae (2) to (5):
   HGEGTFTSDLSKQMEEEAVRLFIEWLKNGGPSSGAPPPS (2) (SEQ ID NO.2)
   HGEGTFTSDLSKQMEEEAVRLFIEWLKNGGPSSGAPPPSK (3) (SEQ ID NO.3)
   DLSKQMEEEAVRLFIEWLKNGGPSSGAPPPS (4) (SEQ ID NO.4)
   DLSKQMEEEAVRLFIEWLKNGGPSSGAPPPSK (5) (SEQ ID NO. 5).
[16] A kit for producing the peptide derivative according to [4], comprising the peptide derivative as defined in [13] to [15], and a substance selected from the group consisting of a labeling compound for labeling the peptide derivative and compounds which are starting material of the labeling compound, wherein the labeling compound contains a radionuclide.

The present invention achieves the effect of providing a molecular probe useful for imaging that accumulates specifically in pancreatic β-cells and has excellent blood clearance. Further, the present invention can also achieve the effect of, for example, detecting the amount of pancreatic β-cells, and moreover, allowing the clarification of pathogenesis, the ultra-early diagnosis, and/or the prevention of development of diseases such as type-I and type-II diabetes.

Further, the present invention achieves the effect of providing a molecular probe useful for imaging at low production cost in an efficient manner because of its high labeling yields at the time of radioactive labeling and the reduced time involved in the labeling.

### [Peptide Derivative]

The peptide derivative of the present invention is represented by the following general formula (I).

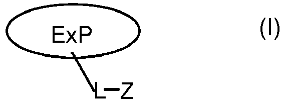

For example, the peptide derivative according to the present invention has excellent specificity for pancreatic β-cells, preferably for GLP-1R of pancreatic β-cells. Also, the peptide derivative of the present invention has excellent blood clearance. For these reasons, the peptide derivative of the present invention can be used for imaging of pancreatic β-cells, preferably for quantification of pancreatic β-cells and/or imaging of GLP-1R of pancreatic β-cells.

In general formula (I), ExP represents a polypeptide that is represented by the amino acid sequence of the following formula (1) and contains completely or partially the amino acid sequence of exendin-4 (SEQ ID NO.1):

Ex4(x-y)-Kₙ (1).

Ex4(x-y) is the amino acid sequence of SEQ ID No.1 between positions x and y. Both x and y represent the number of amino acid residues counted from the N-terminus side of the amino acid sequence of SEQ ID No.1. x is an integer from 1 to 9 and y is an integer from 30 to 39. Although combinations of x and y are not particularly limited and can be selected appropriately, x is preferably an integer from 1 or 9 and y is preferably 39. For example, Ex4(x-y) is preferably Ex4(1-39) or Ex4 (9-39). When x is 9 and y is 39, the amino acid sequence of Ex4(x-y) corresponds to the amino acid sequence of exendin(9-39) as the GLP-1R antagonist.

K of Kₙ represents a lysine residue that can be bound to the C-terminus of Ex4(x-y) and n is 0 or 1. That is, when n is 1 (i.e., K₁), lysine is bound to the C-terminus of Ex4(x-y) and when n is 0 (i.e., K₀), no lysine is bound to the C-terminus of Ex4(x-y). Although the lysine to be bound to the C-terminus may be in L-form (L-lysine) or D-form (D-lysine), the lysine is preferably in D-form to suppress peptide decomposition *in vivo* from the C-terminus side, so as to prevent detection of signals from the decomposition products.

An -L-Z group represents a group represented by the following formula (II) that is bound to an amino acid side chain of a lysine that corresponds to position 12 or 40 of the polypeptide ExP (the polypeptide having the amino acid sequence of formula (1)). Specifically, an -L- group is represented by the following formula (II').

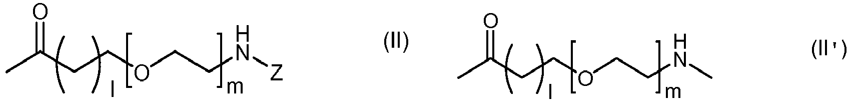

In formula (II), 1 represents the number of methylene groups and m represents the number of oxyethylene groups. When n in formula (1) is 1,1 is 0,1 or 2, and preferably 0 or 1. In the same instance, m is an integer from 3 to 30. To suppress the accumulation of the peptide derivative in liver, kidney, lungs and intestines, and improve the ratio of pancreas/liver and the ratio of pancreas/kidney, m is preferably an integer of 4 or more, 6 or more, 8 or more, 10 or more or 12 or more. An upper limit of m is preferably an integer of 28 or less, 26 or less, 24 or less, 22 or less, 20 or less, 18 or less, 16 or less, 14 or less or 12 or less. Further, to improve accumulation in the pancreas, m may be, for example, an integer from 1 to 14, preferably an integer from 2 to 12 and more preferably an integer from 2 to 8, but is from 3 to 30 in accordance with the invention. Further, when n in formula (1) is 0,1 is an integer from 0 to 8, preferably an integer from 0 to 5, more preferably an integer from 0 to 3, and even more preferably 0 or 1. In the same instance, m is an integer from 2 to 8 or 4 to 30. To suppress the accumulation of the peptide derivative in liver, kidney, lungs and intestines, and improve the ratio of pancreas/liver and the ratio of pancreas/kidney, m is preferably an integer from 4 to 30 and more preferably an integer of 6 or more, 8 or more, or 10 or more. An upper limit of m is preferably an integer of 28 or less, 26 or less, 24 or less, 22 or less, 20 or less, 18 or less, 16 or less, or 14 or less. Further, to improve the accumulation in pancreas, m is, for example, an integer from 0 to 14, preferably an integer from 0 to 12, and more preferably an integer from 2 to 8, but is from 2 to 8 or 4 to 30 in accordance with the invention.

Z represents a labeling group containing a radionuclide or isotope thereof. In the present specification, the "labeling group containing a radionuclide or isotope thereof" includes radionuclides and isotopes thereof. The isotopes include radioisotopes and non-radioisotopes of radionuclides. The labeling group containing a radionuclide or isotope thereof is not particularly limited as long as it is a group that can be bound to an amino group as shown in formula (II), and any of various known labeling groups can be used.

Examples of the radionuclide include ¹¹C, ¹³N, ¹⁵O, ¹⁸F, ⁶⁴Cu, ⁶⁷Ga, ⁶⁸Ga, ⁷⁵Br, ⁷⁶Br, ⁷⁷Br, ⁸²Rb, ^{99m}Tc, ¹¹¹In, ¹²³I, ¹²⁴I, ¹²⁵I, ¹³¹I, and ¹⁸⁶Re. From the viewpoint of performing PET, the radionuclide is preferably a positron emission nuclide such as ¹¹C, ¹³N, ¹⁵O, ¹⁸F, ⁶²Cu, ⁶⁴Cu, ⁶⁸Ga, ⁷⁵Br, ⁷⁶Br, ⁸²Rb, or ¹²⁴I. From the viewpoint of performing SPECT, the radionuclide is preferably a γ-emission nuclide such as ⁶⁷Ga, ^{99m}Tc, ⁷⁷Br, ¹¹¹In, ¹²³I, or ¹²⁵I, and more preferably is ⁷⁷Br, ^{99m}Tc, ¹¹¹In, ¹²³I, or ¹²⁵I. Among these, radioactive halogen nuclides such as ¹⁸F, ⁷⁵Br, ⁷⁶Br, ⁷⁷Br, ¹²³I, and ¹²⁴I are more preferred, and ¹⁸F, ¹²³I, and ¹²⁴I are particularly preferred as the radionuclide. The non-radioisotope of the radionuclide is any non-radioisotope of the above-mentioned radionuclides, and examples of the same include ¹³C, ¹⁴N, ¹⁶O, ¹⁷F, ⁶³Cu, ⁷⁰Ga, ⁸⁰Br, ^{99m}Tc, ¹¹⁵In and ¹²⁷I.

To improve the affinity between the peptide derivative and pancreatic β-cells, preferably the affinity between the peptide derivative and GLP-1R of pancreatic β-cells, the labeling group containing a radionuclide or isotope thereof is preferably a group represented by the following formula (III).

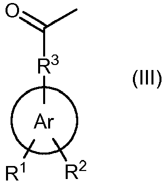

Ar represents an aromatic hydrocarbon group or aromatic heterocyclic group. The aromatic hydrocarbon group is preferably an aromatic hydrocarbon group having a carbon number from 6 to 18, and examples of the same include a phenyl group, o-tolyl group, m-tolyl group, p-tolyl group, 2,4-xylyl group, p-cumenyl group, mesityl group, 1-naphthyl group, 2-naphthyl group, 1-anthryl group, 2-anthryl group, 9-anthryl group, 1-phenanthryl group, 9-phenanthryl group, 1-acenaphthyl group, 2-azulenyl group, 1-pyrenyl group, 2-triphenylenyl group, o-biphenylyl group, m-biphenylyl group, p-biphenylyl group, and terphenyl group. The aromatic heterocyclic group is preferably a 5 to 10-membered heterocyclic group having one or two nitrogen atoms, oxygen atoms, or sulfur atoms, and examples of the same include a triazolyl group, 3-oxadiazolyl group, 2-furyl group, 3-furyl group, 2-thienyl group, 3-thienyl group, 1-pyrrolyl group, 2-pyrrolyl group, 3-pyrrolyl group, 2-pyridyl group, 3-pyridyl group, 4-pyridyl group, 2-pyradyl group, 2-oxazolyl group, 3-isoxyazolyl group, 2-thiazolyl group, 3-isothiazolyl group, 2-imidazolyl group, 3-pyrazolyl group, 2-quinolyl group, 3-quinolyl group, 4-quinolyl group, 5-quinolyl group, 6-quinolyl group, 7-quinolyl group, 8-quinolyl group, 1-isoquinolyl group, 2-quinoxalynyl group, 2-benzofuryl group, 2-benzothienyl group, N-indolyl group, and N-carbazolyl group. Ar is preferably, among these, a phenyl group, triazolyl group, or pyridyl group, and more preferably, a phenyl group.

R¹ represents a substituent containing a radionuclide or isotope thereof, and examples of the same include radionuclides, radionuclide-substituted C₁-C₃ alkyl groups, radionuclide-substituted C₁-C₃ alkoxy groups, isotopes of radionuclides, radionuclide-isotope-substituted C₁-C₃ alkyl groups, and radionuclide-isotope-substituted C₁-C₃ alkoxy groups.

In the present specification, the "C₁-C₃ alkyl groups" refer to alkyl groups that have 1 to 3 carbon atoms, and examples of the same include a methyl group, ethyl group, and propyl group. In the present specification, the "radionuclide-substituted or radionuclide-isotope-substituted C₁-C₃ alkyl groups" refer to alkyl groups that have 1 to 3 carbon atoms and in which the aforementioned radionuclide or isotope thereof substitutes for a hydrogen atom. In the present specification, the "C₁-C₃ alkoxy groups" refer to alkoxy groups that have 1 to 3 carbon atoms, and examples of the same include a methoxy group, ethoxy group, and propoxy group. In the present specification, the "radionuclide-substituted or radionuclide-isotope-substituted C₁-C₃ alkoxy groups" refer to alkoxy groups that have 1 to 3 carbon atoms and in which the aforementioned radionuclide or isotope thereof substitutes for a hydrogen atom.

R¹ preferably represents a substituent containing a radioactive halogen nuclide, and more preferably a substituent containing ¹⁸F, ^{75/76/77}Br, or ^{123/124/125/131}I, for example. From the viewpoint of performing PET, R¹ preferably represents a substituent containing a radioactive halogen nuclide that emits a positron, that is, for example, a substituent containing ¹⁸F, ⁷⁵Br, ⁷⁶Br, or ¹²⁴I. From the viewpoint of performing SPECT, R¹ preferably represents a substituent containing a radioactive halogen nuclide that emits γ-rays, that is, for example, a substituent containing ⁷⁷Br, ¹²³I, or ¹²⁵I. R¹ may be at any of an ortho-position, a meta-position, and a para-position. The meta-position is preferred when R¹ represents ^{123/124/125/131}I or an isotope thereof, and the para-position is preferred when R¹ represents ¹⁸F or an isotope thereof.. It should be noted that, in the present specification, ^{"75/76/77}Br" refers to ⁷⁵Br, ⁷⁶Br or ⁷⁷Br and "^{123/124/125/131}I" refers to ¹²³I, ¹²⁴I, ¹²⁵I or ¹³¹I.

R² represents a hydrogen atom or one or more substituents different from the substituent represented by R¹. Although R² can be a hydrogen atom or substituents, a hydrogen atom is preferred. That is, in formula (I), Ar is preferably not substituted with a substituent other than the substituent represented by R¹. When R² represents two or more substituents, the substituents may all be the same or they may be different from each other. Examples of the substituent include a hydroxyl group, electron attracting groups, electron donating groups, C₁-C₆ alkyl groups, C₂-C₆ alkenyl groups, and C₂-C₆ alkynyl groups. Examples of electron attracting groups include a cyano group, nitro group, halogen atoms, carbonyl group, sulfonyl group, acetyl group, and phenyl group. Examples of halogen atoms include a fluorine atom, chlorine atom, bromine atom, and iodine atom. In the present specification, the "C₁-C₆ alkyl group" refers to an alkyl group having 1 to 6 carbon atoms, and examples of the same include a methyl group, ethyl group, propyl group, isopropyl group, butyl group, isobutyl group, sec-butyl group, tert-butyl group, pentylene group, isopentyl group, and hexylene group. In the present specification, the "C₂-C₆ alkenyl group" refers to an alkenyl group having 2 to 6 carbon atoms, and examples of the same include a vinyl group, 1-propenyl group, 2-propenyl group, isopropenyl group, 1-butenyl group, 2-butenyl group, and 3-butenyl group. In the present specification, the "C₂-C₆ alkynyl group" refers to an alkynyl group having 2 to 6 carbon atoms, and examples of the same include an ethynyl group, 1-propynyl group, 2-propynyl group, 1-butynyl group, 2-butynyl group, and 3-butynyl group. Among these, the substituent is preferably a hydroxyl group or electron attracting group.

R³ preferably represents a bond, alkylene group having 1 to 6 carbon atoms, or oxyalkylene group having 1 to 6 carbon atoms. Examples of the alkylene group having 1 to 6 carbon atoms include straight-chain or branched-chain alkylene groups, such as a methylene group, ethylene group, propylene group, butylene group, pentyl group, and hexyl group. Examples of an oxyalkylene group having 1 to 6 carbon atoms include an oxymethylene group, oxyethylene group, oxypropylene group, oxybutylene group, and oxypentylene group. R³ is preferably a bond, methylene group, or ethylene group, and more preferably, a bond, to improve the affinity between the peptide derivative and pancreatic islets, preferably the affinity between the peptide derivative and pancreatic β-cells, and more preferably the affinity between the peptide derivative and GLP-1R of pancreatic β-cells.

To improve the affinity between the peptide derivative and pancreatic β-cells, preferably the affinity between the peptide derivative and GLP-1R of pancreatic β-cells, the group represented by formula (III) is preferably a group represented by formula (IIIa), and more preferably a group represented by formula (IIIb) or (IIIc). In formula (IIIa), R¹ is as described above. Further, the group represented by formula (III) is preferably a group represented by formula (IIId) for general versatility.

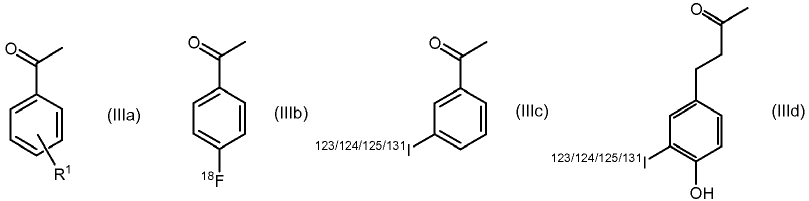

From the viewpoint of performing labeling with a metal radioisotope (radioactive metal nuclide), the labeling group containing a radionuclide or isotope thereof may contain a radioactive metal nuclide and a chelating site that can chelate the radioactive metal nuclide. Examples of compounds that can form a chelating site include diethylenetriaminepentaacetic acid (DTPA), 6-hydrazinopyridine-3-carboxylic acid (HYNIC), tetraazacyclododecanetetraacetic acid (DOTA), dithisosemicarbazone (DTS), diaminedithiol (DADT), mercaptoacetylglycylglycylglycine (MAG3), monoamidemonoaminedithiol (MAMA), diamidedithiol (DADS), and propylene diamine dioxime (PnAO).

The carboxyl group at the C-terminus of the polypeptide ExP is amidated with an amino group to improve binding to pancreatic β-cells and/or stability *in vivo.* Although the amino acid positioned at the C-terminus of the polypeptide ExP can be in L-form (L-amino acid) or D-form (D-amino acid), D-form is preferred to suppress peptide decomposition *in vivo* from the C-terminus side.

When the polypeptide ExP is a polypeptide represented by Ex4(x-y)-K₁ and the -L-Z group is bound to K (lysine) at the C-terminus, the lysine to which the -L-Z group is bound is preferably D-lysine to suppress peptide decomposition *in vivo* from the C-terminus side, so as to prevent detection of signals from the decomposition products. In this case, although the amino acid next to the lysine at the C-terminus to which the -L-Z group is bound, namely, the amino acid at position (y-(x-1)) may be either in L-form or D-form, D-form is more preferable to suppress peptide decomposition *in vivo* from the C-terminus side so as to prevent detection of signals from the decomposition products. For example, when y is 39, the serine at position (40x) may be either L-serine or D-serine, but D-serine is preferable for the same reason as mentioned above.

The α-amino group at the N-terminus of the polypeptide ExP is unmodified or modified, to cancel the positive electric charge of the α-amino group at the N-terminus to prevent the accumulation of the peptide derivative in the kidney, with a modifying group having no electric charge.

Examples of modifying groups having no electric charge include a 9-fluorenylmethyloxycarbonyl group (Fmoc), tert-butoxycarbonyl group (Boc), benzyloxycarbonyl group (Cbz), 2,2,2-trichloroethoxycarbonyl group (Troc), allyloxycarbonyl group (Alloc), 4-methoxytrityl group (Mmt), amino group, alkyl groups having a carbon number of 3 to 20, 9-fluoreneacetyl group, 1-fluorenecarboxylic acid group, 9-fluorenecarboxylic acid group, 9-fluorenone-1-carboxylic acid group, benzyloxycarbonyl group, xanthyl group (Xan), trityl group (Trt), 4-methyltrityl group (Mtt), 4-methoxy2,3,6-trimethyl-benzenesulfonyl group (Mtr), mesitylene-2-sulfonyl group (Mts), 4,4-dimethoxybenzohydryl group (Mbh), tosyl group (Tos), 2,2,5,7,8-pentamethylchroman-6-sulfonyl group (Pmc), 4-methylbenzyl group (MeBzl), 4-methoxybenzyl group (MeOBzl), benzyloxy group (BzlO), benzyl group (Bzl), benzoyl group (Bz), 3-nitro-2-pyridinesulfenyl group (Npys), 1-(4,4-dimethyl-2,6-diaxocyclohexylidene)ethyl group (Dde), 2,6-dichlorobenzyl group (2,6-DiCl-Bzl), 2-chlorobenzyloxycarbonyl group (2-Cl-Z), 2-bromobenzyloxycarbonyl group (2-Br-Z), benzyloxymethyl group (Bom), cyclohexyloxy group (cHxO), t-butoxymethyl group (Bum), t-butoxy group (tBuO), t-butyl group (tBu), acetyl group (Ac), trifluoroacetyl group (TFA), o-bromobenzyloxycarbonyl group, t-butyldimethylsilyl group, 2-chlorobenzyl (Cl-z) group, cyclohexyl group, cyclopentyl group, isopropyl group, pivalyl group, tetrahydropyran-2-yl group, and trimethylsilyl group. Among these, preferably, the modifying group is an acetyl group, benzyl group, benzyloxymethyl group, o-bromobenzyloxycarbonyl group, t-butyl group, t-butyldimethylsilyl group, 2-chlorobenzyl group, 2,6-dichlorobenzyl group, cyclohexyl group, cyclopentyl group, isopropyl group, pivalyl group, tetrahydropyran-2-yl group, tosyl group, trimethylsilyl group, or trityl group. More preferably, the modifying group is an acetyl group.

For example, Ex4(x-y)-Kₙ is preferably Ex4(1-39), Ex4(1-39)-K Ex4(9-39), or Ex4(9-39)-K. Specifically, Ex4(x-y)-Kₙ is preferably any of the amino acid sequences of the following formulae (2) to (5):
HGEGTFTSDLSKQMEEEAVRLFIEWLKNGGPSSGAPPPS (2) (SEQ ID NO.2)
HGEGTFTSDLSKQMEEEAVRLFIEWLKNGGPSSGAPPPSK (3) (SEQ ID NO.3)
DLSKQMEEEAVRLFIEWLKNGGPSSGAPPPS (4) (SEQ ID NO.4)
DLSKQMEEEAVRLFIEWLKNGGPSSGAPPPSK (5) (SEQ ID NO. 5).

When Ex4(x-y)-Kₙ is the amino acid sequence of formula (2), the -L-Z group is bound to the amino group of the side chain of lysine at position 12 (hereinafter also referred to as "Lys12") of the amino acid sequence of formula (2). When Ex4(x-y)-Kₙ is the amino acid sequence of formula (3), the -L-Z group is preferably bound to the amino group of the side chain of lysine at position 40 of the amino acid sequence of formula (3) (hereinafter also referred to as "Lys40") and Lys40 is preferably D-lysine to suppress peptide decomposition *in vivo* from the C-terminus side so as to prevent detection of signals from the decomposition products. In this case, the serine at position 39 is preferably D-serine for the same reason as mentioned above.

When Ex4(x-y)-Kₙ is the amino acid sequence of formula (4), the -L-Z group is bound to, for example, the amino group of the side chain of lysine at position 4 (hereinafter also referred to as "Lys4") of the amino acid sequence of formula (4). When Ex4(x-y)-Kₙ is the amino acid sequence of formula (5), the -L-Z group is preferably bound to the amino group of the side chain of lysine at position 32 of the amino acid sequence of formula (5) (hereinafter also referred to as "Lys32") and Lys 32 is preferably D-lysine to suppress peptide decomposition *in vivo* from the C-terminus side so as to prevent detection of signals from the decomposition products. In this case, the serine at position 31 is preferably D-serine for the same reason as mentioned above. The lysine at position 4 of the amino acid sequences of formula (4) and formula (5) corresponds to the lysine at position 12 of the amino acid sequence of formula (1); the lysine at position 32 of the amino acid sequence of formula (5) corresponds to the lysine at position 40 of the amino acid sequence of formula (1).

The peptide derivative of the present invention can be used for imaging of pancreatic islets, preferably for imaging of pancreatic β-cells, and more preferably for a molecular probe for imaging GLP-1R of pancreatic β-cells. Further, the peptide derivative of the present invention can be used for imaging for prevention, treatment, or diagnosis of diabetes. Further, the peptide derivative of the present invention can be used for, for example, a composition, a reagent for imaging, a contrast medium and a diagnostic imaging agent that contains the peptide derivative of the present invention as an effective ingredient, and which are used for a variety of imagings as described above. Although the composition, the diagnostic imaging agent and the like may be in the form of a solution or powder, for example, they are preferably in the form of a solution, and more preferably in the form of a parenteral solution in view of the half-life and radioactive decay of the radionuclide.

### [Labeling Precursor]

Also disclosed herein is the peptide derivative represented by the following general formula (IV). Such a peptide derivative can be utilized as the labeling precursor of the peptide derivative of the present invention.

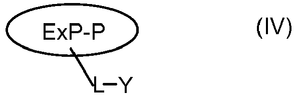

ExP-P represents a polypeptide that is represented by the amino acid sequence of the following formula (1) and contains completely or partially the amino acid sequence of exendin-4 (SEQ ID NO.1):

Ex4(x-y)-Kₙ (1).

Ex4(x-y)-Kₙ is the same as that in the peptide derivative of the present invention described above, and particularly, Ex4(1-39), Ex4(1-39)-K Ex4(9-39) or Ex4(9-39)-K is preferred. Specifically, it is preferable that Ex4(x-y)-Kₙ represents any of the amino acid sequences of formulae (2) to (5). Further, the carboxyl group at the C-terminus of the polypeptide ExP-P is amidated.

An -L-Y group represents a group represented by the following formula (V) that is bound to an amino acid side chain of a lysine that corresponds to position 12 or 40 of the polypeptide ExP-P represented by the amino acid sequence of formula (1). The -L- group, 1 and m are the same as those in the peptide derivative of the present invention described above.

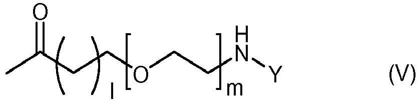

Y represents a hydrogen atom or radioactive labeling introduction group. Y preferably represents a hydrogen atom to allow the production of the peptide derivative of the present invention with ease, performance of labeling in a short time, and moreover the achievement of excellent labeling yields.

In the present specification, the "radioactive labeling introduction group" refers to a group capable of introducing a radionuclide or a radioactive labeling group containing a radionuclide. In the present specification, examples of being "capable of introducing a radionuclide or a radioactive labeling group containing a radionuclide" include a radioactive labeling introduction group or a specific functional group having a radioactive labeling introduction group being replaceable with a radionuclide, bindable to or chelatable with a radionuclide, and bindable to a radioactive labeling group containing a radionuclide.

From the viewpoint of performing labeling with a radioactive metal nuclide, the radioactive labeling introduction group may contain a radioactive metal nuclide and a chelating site that can chelate the radioactive metal nuclide. Chelate compounds that form a chelating site are as described above.

Examples of the radioactive labeling introduction group include a group represented by the following formula (VI) to improve the affinity between the peptide derivative to be obtained after the labeling and pancreatic β-cells, preferably the affinity between the peptide derivative and GLP-1R of pancreatic β-cells.

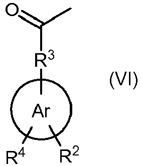

In formula (VI), Ar, R² and R³ are the same as those in formula (III). R⁴ represents a substituent having a mesylate (OMs) group, tosylate (OTs) group, triflate (OTf) group, [⁸⁰Br]bromine atom, [¹²⁷I]iodine atom, chlorine atom, nitro group, trimethylammonium group, tin atom, alkyltin group, or alkylsilicon group. An OMs group, OTs group, OTf group, [⁸⁰Br]bromine atom, [¹²⁷I]iodine atom, tin atom, alkyltin group or alkylsilicon group is preferred, and a [⁸⁰Br]bromine atom, [¹²⁷I]iodine atom or tin atom is more preferred. The alkyltin group may be a tin-atom-substituted C₁-C₆ alkyl group, preferably a tributyl tin group (Sn(C₄H₉)₃). The alkylsilicon group may be a silicon-atom-substituted C₁-C₆ alkyl group, preferably a tributyl silicon group. R⁴ may be at an ortho-position, meta-position, or para-position. The para-position is preferred for producing a labeling precursor in a fluorine-labeled form. R⁴ may be at an ortho-position, meta-position, or para-position for producing a labeling precursor in iodine-labeled form.

The group represented by formula (VI) is preferably a group represented by the following formula (VIa), and more preferably a group represented by formula (VIa) where R⁴ is a group representing a [⁸⁰Br]bromine atom, [¹²⁷I]iodine atom or tin atom. In formula (VIa), R⁴ is as described above.

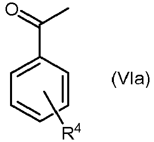

The α-amino group at the N-terminus of the polypeptide ExP-P may be bound to a protecting group, modified with a modifying group having no electric charge or not modified. To allow more selective labeling, the α-amino group at the N-terminus is preferably protected by a protecting group or modified with a modifying group having no electric charge. The modifying group having no electric charge is as described above.

When Ex4(x-y)-Kₙ is the amino acid sequence of formula (2), the -L-Y group is bound to the amino group of the side chain of Lysl2. When Ex4(x-y)-Kₙ is the amino acid sequence of formula (3), the -L-Y group is preferably bound to the amino group of the side chain of Lys40, and Lys 40 is preferably D-lysine to obtain a peptide derivative that is prevented from decomposing *in vivo* from the C-terminus side so as to prevent detection of signals from the decomposition products. In this case, the serine at position 39 is preferably D-serine for the same reason as mentioned above.

When Ex4(x-y)-Kₙ is the amino acid sequence of formula (4), the -L-Y group is bound to the amino group of the side chain of Lys4. When Ex4(x-y)-Kₙ is the amino acid sequence of formula (5), the -L-Y group is preferably bound to the amino group of the side chain of Lys32, and Lys32 is preferably D-lysine to obtain a peptide derivative that is prevented from decomposing *in vivo* from the C-terminus side so as to prevent detection of signals from decomposition products. In this case, the serine at position 31 is preferably D-serine for the same reason as mentioned above.

In the polypeptide ExP-P, amino acid side chains to which the -L-Y group is not bound may each be bound to a protecting group so as to be protected by it, or may not be protected. To allow more selective labeling, it is preferable that functional groups of amino acid side chains to which the -L-Y group is not bound are each bound to a protecting group so as to be protected by it, and it is more preferable that the amino groups of side chains of lysines to which the -L-Y group is not bound are each protected. To simplify the operation after labeling to reduce the production time, amino acid side chains to which the -L-Y group is not bound are preferably not protected and are free. For the protecting group, any known protecting groups capable of protecting functional groups of amino acid side chains to which the -L-Y group of the labeling precursor is not bound during labeling, preferably those capable of protecting amino groups to which the -L-Y group is not bound, can be used. The protecting group is not particularly limited, and examples of the same include Fmoc, Boc, Cbz, Troc, Alloc, Mmt, an amino group, alkyl group having a carbon number of 3 to 20, 9-fluoreneacetyl group, 1-fluorenecarboxylic acid group, 9-fluorenecarboxylic acid group, 9-fluorenone-1-carboxylic acid group, benzyloxycarbonyl group, Xan, Trt, Mtt, Mtr, Mts, Mbh, Ibs, Pmc, MeBzl, MeOBzl, BzlO, Bzl, Bz, Npys, Dde, 2,6-DiCl-Bzl, 2-Cl-Z, 2-Br-Z, Bom, cHxO, Bum, tBuO, tBu, Ac and TFA. Fmoc and Boc are preferred in terms of handling.

When Ex4(x-y)-Kₙ is the amino acid sequence of formula (2), the -L-Y group is bound to the amino group of the side chain of Lys12. To allow selective labeling, it is preferable that a protecting group is bound to the amino group of the side chain of the lysine to which the -L-Y group is not bound, i.e. to obtain a peptide derivative having excellent specificity for pancreatic β-cells, it is preferable that a protecting group is bound to the amino group of the side chain of the lysine at position 27 (Lys27). It is still more preferable that a protecting group is bound to each of the amino group of the side chain of Lys27 and the α-amino group at the N-terminus.

When Ex4(x-y)-Kₙ is the amino acid sequence of formula (3), it is preferable that the -L-Y group is bound to the amino group of the side chain of Lys40. To allow more selective labeling, it is more preferable that the -L-Y group is bound to the amino group of the side chain of Lys40 and a protecting group is bound to each of the amino groups of the side chains of Lys12 and that of Lys27, and it is still more preferable that the -L-Y group is bound to the amino group of the side chain of Lys40 and a protecting group is bound to each of the α-amino group at the N-terminus, the amino group of the side chain of Lys12 and that of Lys27.

When Ex4(x-y)-Kₙ is the amino acid sequence of formula (4), the -L-Y group is bound to the amino group of the side chain of Lys4. To allow selective labeling, it is preferable that the -L-Y group is bound to the amino group of the side chain of Lys4 and a protecting group is bound to the amino group of the side chain of the lysine to which the -L-Y group is not bound, i.e. to obtain a polypeptide derivative having excellent specificity for pancreatic β-cells, it is preferable that the -L-Y group is bound to the amino group of the side chain of Lys4 and a protecting group is bound to the amino group of the side chain of the lysine at position 19 (Lys19). It is still more preferable that the -L-Y group is bound to the amino group of the side chain of Lys4 and a protecting group is bound to each of the amino group of the side chain of Lys19 and the α-amino group at the N-terminus.

When Ex4(x-y)-Kₙ is the amino acid sequence of formula (5), the -L-Y group is preferably bound to the amino group of the side chain of Lys32. To allow more selective labeling, it is more preferable that the -L-Y group is bound to the amino group of the side chain of Lys32 and a protecting group is bound to each of the amino groups of the side chains of Lys4 and that of Lys19, and it is still more preferable that the -L-Y group is bound to the amino group of the side chain of Lys32 and a protecting group is bound to each of the α-amino group at the N-terminus, the amino group of the side chain of Lys4 and that of Lys19.

Examples of forms of the labeling precursor include a solution and a powder. The labeling precursor is preferably in the form of a powder, and more preferably in the form of a freeze-dried powder (freeze-dried formulation) for handling purposes.

The labeling precursor can be synthesized using, for example, an amino acid in which the α-amino group at the N-terminus is protected by a protecting group (hereinafter, an amino acid in which the α-amino group at the N-terminus is protected by a protecting group will be referred to as a "protected amino acid") and a protected amino acid whose side chain is bound to the -L-Y group. To synthesize a labeling precursor that can be labeled more selectively, the labeling precursor is preferably synthesized using a protected amino acid whose side chain is bound to the -L-Y group and a protected amino acid with the functional group of its side chain being protected by a protecting group. The synthesis of the labeling precursor preferably includes synthesizing the polypeptide represented by the amino acid sequence of formula (1), removing protecting groups which are protecting functional groups of amino acid side chains in the synthesized polypeptide to which the -L-Y group is not bound and that can be labeled, reprotecting the deprotected functional groups of the amino acids with protecting groups different from those removed, and removing protecting groups to deprotect functional groups of the amino acids other than those reprotected. When Y represents a hydrogen atom, the amino group at the end of the -L-Y group is preferably protected.

### [Method for Producing Peptide Derivative]

Another aspect of the present invention relates to a method for producing a peptide derivative of the invention, which includes labeling the labeling precursor defined above.

In the method for producing the peptide derivative of the present invention, to allow the production of the peptide derivative of the present invention with ease, preferably performing labeling in a short time, and moreover achieving excellent labeling yields, it is preferable that, in the labeling precursor represented by formula (IV), Y is a hydrogen atom. Specifically, a peptide derivative represented by the following formula (VII) is preferred. With the production method according to this aspect, labeling can be performed in a short time, and moreover, excellent labeling yields can be achieved when the peptide derivative represented by formula (VII) is used as the labeling precursor. Furthermore, with the production method according to this aspect, it is possible to preferentially suppress labeling by-products. In formula (VII), the polypeptide ExP-P, 1 and m are as described above.

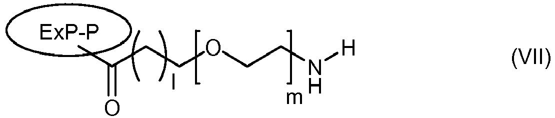

For example, a labeling compound having the group represented by formula (III) can be used to label the labeling precursor. The labeling compound having the group represented by formula (III) is preferably a succinimidyl ester compound in which the group represented by formula (III) is bound to succinimide via an ester bond, and more preferably, a succinimidyl ester compound represented by the following formula (VIII). In the succinimidyl ester compound represented by formula (VIII), it is preferable that Ar, R², and R³ represent a phenyl group, a hydrogen atom, and a bond, respectively, to improve the affinity between the peptide derivative to be produced and pancreatic β-cells, preferably the affinity between the peptide derivative and GLP-1R of pancreatic β-cells; and it is more preferable that Ar, R¹, R², and R³ represent a phenyl group, [¹⁸F]fluorine atom or [^{123/124/125/131}I]Iodo atom, a hydrogen atom, and a bond, respectively. The labeling compound having the group represented by formula (III) is preferably a compound represented by the following formula (VIIIa), and more preferably a compound represented by the following formula (VIIIb) ([¹⁸F]N-succinimidyl 4-fluorobenzoate) and a compound represented by the following formula (VIIIc) ([^{123/124/125/131}I]N-succimmidyl 3-iodobenzoate). The labeling compound having the group represented by formula (III) is preferably a compound represented by the following formula (VIIId) for general versatility.

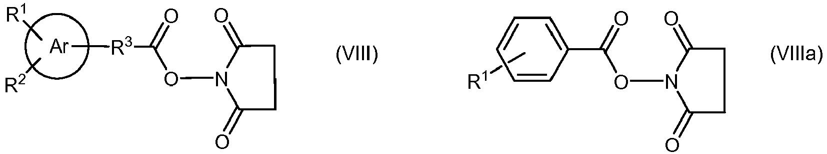

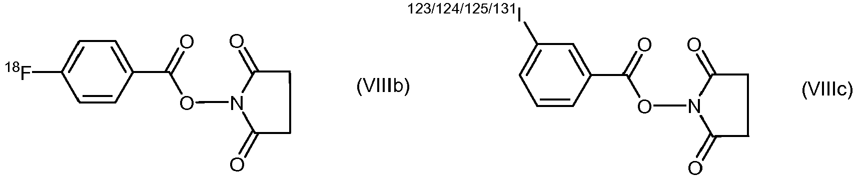

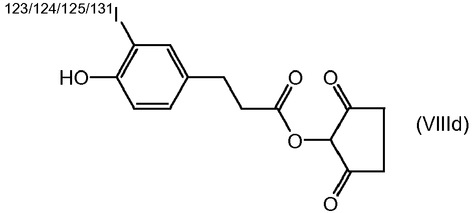

The method for producing the peptide derivative of the present invention may include removing protecting groups bound to the labeled peptide derivative to deprotect the peptide derivative. The deprotection can be performed by way of a known method in accordance with the type of the protective groups.

To produce a radioactively labeled peptide derivative with high purity, the method for producing the peptide derivative of the present invention may further include purifying the labeled peptide derivative. For purification, any known separation technique for purifying peptides or proteins can be used, for example. Examples of separation techniques include ion-exchange chromatography, hydrophobic chromatography, reverse-phase chromatography, and high-performance liquid chromatography (HPLC) and they can be used in combination as needed.

The method for producing the peptide derivative of the present invention may include synthesizing a labeling compound having the group represented by formula (III) used in the labeling and/or synthesizing the labeling precursor. In this case, the synthesis of the labeling compound and the labeling of the labeling precursor can be performed using a single automatic synthesizing device, or the synthesis of the labeling precursor, the synthesis of the labeling compound and the labeling of the labeling precursor can be performed using a single automatic synthesizing device.

Another aspect of the method for producing the peptide derivative of the present invention relates to a method for producing a peptide derivative, which includes labeling a labeling precursor in which Y in formula (IV) represents a radioactive labeling introduction group. In the method for producing a peptide according to this aspect, the labeling precursor may be, for example, a labeling precursor in which Y represents a chelating site or the group represented by formula (VI). Examples of the labeling precursor in which Y represents the group represented by formula (VI) include a labeling precursor represented by the following formula (IX). In formula (IX), the polypeptide ExP-P, l, m and R⁴ are as described above.

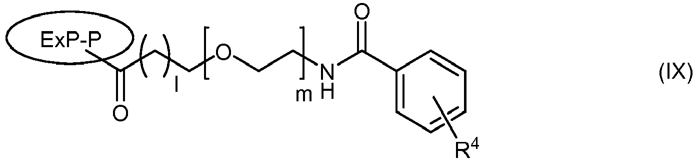

Examples of the labeling compound used in the labeling according to this aspect include compounds containing radioactive halogen nuclides such as [¹⁸F]F₂, [¹⁸F]KF, [^{123/124/125/131}I]NaI, and [^{123/124/125/131}I]NH₄I and compounds containing radioactive metal nuclides. The reaction used in the labeling is not particularly limited and electrophilic substitution, nucleophilic substitution, and the like can be used. Also in this aspect, the method may further include a deprotection process and/or a purification process as described above.

### [Reagent for Imaging]

Also disclosed herein is a reagent for imaging, which contains the peptide derivative represented by general formula (I).

The reagent for imaging disclosed herein contains, as an effective ingredient, the peptide derivative represented by general formula (I), preferably a peptide derivative in which Z in general formula (I) represents a labeling group containing a radionuclide for performing PET and SPECT imaging.

The form of the reagent for imaging is not particularly limited, and the reagent may be in the form of a solution or powder, for example. When Z in general formula (I) represents a labeling group containing a radionuclide, the reagent is preferably in the form of a solution, and more preferably in the form of a parenteral solution in terms of the half-life and radioactive decay of the radionuclide. When Z in general formula (I) represents a labeling group containing a non-radioisotope of a radionuclide, the reagent may be in the form of a solution or powder. However, the reagent is preferably in the form of a powder, and more preferably in the form of a freeze-dried powder (freeze-dried formulation) for handling and storage stability.

The reagent for imaging may contain medicinal additives such as a carrier. In the present specification, medicinal additives refer to compounds that have been approved as medicinal additives in the Japanese, US and/or European pharmacopoeia. Aqueous solvents and non-aqueous solvents can be used as the carrier. Examples of aqueous solvents include a potassium phosphate buffer solution, a physiological saline solution, a Ringer's solution, and distilled water. Examples of non-aqueous solvents include polyethylene glycol, vegetable oils, ethanol, glycerin, dimethylsulfoxide and propylene glycol.

### [Kit]

Also disclosed herein is a kit including the peptide derivative represented by general formula (I) and/or the labeling precursor represented by general formula (IV). Examples of the kit include a kit for producing the peptide derivative of the present invention, a kit for imaging pancreatic β-cells, a kit for imaging GLP-1R of pancreatic β-cells, a kit for determining the amount of pancreatic islets, and a kit for prevention, treatment, or diagnosis of diabetes. Preferably, the kit includes a suitable instruction manual. The instruction manual may be packed with the kit or may be provided on the web. The invention provides a kit for producing the peptide derivative of the invention, comprising the labeling precursor represented by general formula (IV), and a substance selected from the group consisting of a labeling compound for labeling the peptide derivative and compounds which are starting materials of the labeling compound, wherein the labeling compound contains a radionuclide.

The form of the peptide derivative represented by general formula (I) is not particularly limited, and it may be in the form of a solution or powder, for example. When Z in general formula (I) represents a labeling group containing a radionuclide, the peptide derivative is preferably in the form of a parenteral solution in terms of the half-life and radioactive decay of the radionuclide. When Z in general formula (I) represents a labeling group containing a non-radioisotope of a radionuclide, the peptide derivative may be in the form of a solution or powder. However, the peptide derivative is preferably in the form of a powder, and more preferably in the form of a freeze-dried powder (freeze-dried formulation) for handling and storage stability.

The form of the labeling precursor represented by general formula (IV) is not particularly limited, and it may be in the form of a solution or powder, for example. The labeling precursor is preferably in the form of a powder, and more preferably in the form of a freeze-dried powder (freeze-dried formulation) for handling.

The kit including the labeling precursor may contain a labeling compound used for labeling the labeling precursor, compounds to be starting materials of the labeling compound and other reagents used in radioactive labeling. In accordance with the invention, the kit contains a labeling compound for labeling the peptide derivative or compounds which are starting materials of the labeling compound, wherein the labeling compound contains a radionuclide. The labeling compound is as described above. In particular, the compound represented by formula (VIIIa) is preferred and the compound represented by formula (VIIIb) and the compound represented by formula (VIIIc) are more preferred. Examples of starting materials include starting materials of the labeling compound represented by formula (VIIIb) and those of the labeling compound represented by formula (VIIIc).

Examples of starting materials of the labeling compound represented by formula (VIIIb) include ester derivatives of 4-(trimethylammonium triflate) benzoic acid. Examples of ester derivatives of 4-(trimethylammonium triflate) benzoic acid include methyl ester, ethyl ester, t-butyl ester and pentamethyl ester. Examples of other starting materials of the labeling compound represented by formula (VIIIb) include ethyl 4-(trimethylammonium triflate) benzoate, ethyl 4-(tosyloxy)benzoate, and ethyl 4-(methylsulfonyloxy) benzoate. Examples of starting materials of the labeling compound represented by formula (VIIIc) include 2,5-dioxopyrrolidin-1-yl 3-(tributylstannyl)benzoate, 2,5-dioxopyrrolidin-1-yl 3-bromobenzoate, 2,5-dioxopyrrolidin-1-yl 3-chlorobenzoate and 2,5-dioxopyrrolidin-1-yl 3-iodobenzoate. Examples of other reagents used in radioactive labeling include reagents containing a radionuclide used for synthesizing the labeling compound.

The kit of the present invention and as described herein further may include a container for containing the peptide derivative represented by general formula (I) and/or the labeling precursor represented by general formula (IV). Examples of the container include a syringe and a vial.

The kit of the present invention and as described herein may further contain components for preparing a molecular probe, such as a buffer and an osmotic regulator, and an instrument used in administration of the peptide derivative, such as a syringe.

The kit including the labeling precursor may include, for example, an automatic synthesizing device for synthesizing the labeling compound. In addition to synthesizing the labeling compound, the automatic synthesizing device may also be capable of labeling the labeling precursor using the synthesized labeling compound, deprotecting the labeled peptide derivative, and synthesizing the labeling precursor.

### [Method for Imaging]

Still another aspect of the present invention relates to a method for imaging pancreatic β-cells, which includes imaging pancreatic β-cells using the peptide derivative represented by general formula (I). With the method for imaging of the present invention, it is possible to image pancreatic β-cells, preferably GLP-1R of pancreatic β-cells because the peptide derivative of the present invention is used. Examples of the analyte include humans and/or mammals other than humans. The peptide derivative is preferably a peptide derivative in which Z in general formula (I) represents a labeling group containing a radionuclide.

The method for imaging of the present invention includes detecting a signal of a radionuclide of the peptide derivative represented by general formula (I) from an analyte to which the peptide derivative has been administered in advance. The signal is preferably detected after a lapse of sufficient time from administration of the peptide derivative.

The method for imaging of the present invention may include, for example, reconfiguring the detected signal to convert the signal into an image, and displaying the image, and/or converting the detected signal into numbers and presenting an accumulation amount. Displaying includes, for example, displaying the image on a monitor and printing the same. Presenting includes, for example, storing the calculated accumulation amount and outputting the same to the outside.

The detection of the signal can be determined appropriately in accordance with the type of radionuclide of the peptide derivative to be used, and PET and SPECT can be used to perform the detection. SPECT includes, for example, determining, with use of a gamma camera, γ-rays emitted from an analyte to which the peptide derivative of the present invention has been administered. The determination with use of the gamma camera includes, for example, measuring radiation (γ-rays) emitted from the radionuclide of the peptide derivative during a certain time unit, and preferably includes determining the direction in which the radiation is emitted and the radiation dose during a certain time unit. The method for imaging of the present invention further may include presenting the determined distribution of the peptide derivative obtained by the measurement of the radiation as a cross-sectional image, and reconfiguring the obtained cross-sectional image.

PET includes, for example, simultaneously measuring γ-rays generated upon annihilation of positrons with electrons, with use of a detector for PET, from an analyte to which the peptide derivative has been administered, and further may include figuring a three-dimensional distribution of positions of radionuclides emitting positrons, based on the measurement results.

Determination by means of X-ray CT and/or MRI may be performed, together with determination by means of SPECT or PET. This makes it possible to obtain, for example, a fusion image obtained by fusion of an image obtained by SPECT or PET (functional image), with an image obtained by CT or MRI (morphological image).

Also disclosed herein is a method for imaging which includes administering the peptide derivative represented by general formula (I) to an analyte and detecting a signal of a radionuclide of the peptide derivative from the analyte to which the peptide derivative has been administered. The detection of the signal and the reconfiguration can be performed in the same manner as in the method for imaging according to the invention.

Administration of the peptide derivative to an analyte may be local administration or systemic administration. A path for administration may be determined appropriately according to the state of the analyte and the like, and it may be, for example, intravenous, intraarterial, intradermal, and intraabdominal injection or infusion. The administration amount (dosage) of the peptide derivative is not particularly limited and the peptide derivative may be administered in a sufficient amount to obtain a desired contrast for imaging, for example, not more than 1 µg. The peptide derivative is preferably administered with medicinal additives, such as a carrier. The medicinal additives are as described above. The time period from the administration to the determination may be decided appropriately according to, for example, the time that it takes for the molecular probe to be bound to pancreatic β-cells, the type of molecular probe, the decomposition time of the molecular probe, etc.

The method for imaging disclosed herein may include determining the state of pancreatic islets or pancreatic β-cells based on the obtained image using the peptide derivative of the present invention. Determining the state of pancreatic islets or pancreatic β-cells includes, for example, determining the presence/absence of pancreatic islets or pancreatic β-cells and determining an increase/decrease in the amount of pancreatic islets by analyzing an image of the pancreatic β-cells.

### [Method for Determining Amount of Pancreatic Islets]

Still another aspect of the present invention relates to a method for determining the amount of pancreatic islets using the peptide derivative represented by general formula (I). The method for determining the amount of pancreatic islets according to the present invention includes detecting a signal of the peptide derivative represented by general formula (I) from an analyte to which the peptide derivative has been administered, and calculating the amount of the pancreatic islets from the detected signal of the peptide derivative. The peptide derivative is preferably a peptide derivative in which Z in general formula (I) is a labeling group containing a radionuclide.

The calculation of the amount of pancreatic islets can be performed by, for example, analyzing the detected signal amount, and an image obtained by reconfiguration of the signal. Further, quantification of a subject of the imaging from results of the imaging can be performed easily by any person skilled in the art, using a calibration curve, an appropriate program, or the like. The subject of the imaging is, for example, pancreatic islets, preferably pancreatic β-cells, and more preferably GLP-1R of pancreatic β-cells. The method for determining the amount of pancreatic islets according to the present invention is preferably a method for determining the amount of pancreatic β-cells for use of the same in examination and diagnosis.

The method for determining the amount of pancreatic islets according to the present invention further may include presenting the calculated amount of pancreatic islets. Presenting the calculated amount of pancreatic islets includes, for example, storing the calculated amount of pancreatic islets or outputting the same to the outside. Outputting the same to the outside includes, for example, displaying the same on a monitor and printing the same.

### [Methods for Prevention, Treatment, and Diagnosis of Diabetes]

Also disclosed herein are methods for prevention, treatment, or diagnosis of diabetes. As described above, in the development of diabetes, the amount of pancreatic islets (particularly, the amount of pancreatic β-cells) decreases prior to the occurrence of glucose tolerance abnormalities, and therefore, when functional abnormalities are detected or there are subjective symptoms, diabetes has already reached a stage where it is too difficult to be treated. However, with the method for imaging using the peptide derivative of the present invention, and/or the method for determining the amount of pancreatic islets using the same, a decrease in the amount of pancreatic islets and/or the amount of pancreatic β-cells can be detected at an early stage, and further, new methods for prevention, treatment, and diagnosis of diabetes can be created. Examples of a subject (analyte) on which prevention, treatment, and diagnosis of diabetes may be carried out include humans and/or mammals other than humans.

The present invention thus provides a peptide derivative represented by general formula (I) for use in diagnosis of diabetes, wherein said diagnosis includes: imaging of pancreatic islets with said peptide derivative of the present invention; and preferably determining the state of the pancreatic islets based on the obtained image of the pancreatic islets and/or the obtained amount of the pancreatic islets; and further may include performing diagnosis of diabetes based on the determination results.
The determination of the state of pancreatic islets includes, for example, determining an increase/decrease, or a change, in the amount of pancreatic islets by comparing the obtained image of pancreatic islets with an image of pancreatic islets as a reference, or comparing the obtained amount of pancreatic islets with an amount of pancreatic islets as a reference. Further, the determination of the state of pancreatic islets may be carried out using an information processing device. When it is determined that the amount of pancreatic islets has decreased, preferably this information is presented, and when it is determined that the amount of pancreatic islets has increased or has been maintained, preferably this information is presented. The diagnosis of diabetes on the basis of the determination results includes, for example, determining the risk of development of diabetes, judging it to be diabetes, and determining the degree of development of diabetes.

The method for treatment of diabetes as disclosed herein includes, imaging of pancreatic islets with use of the peptide derivative of the present invention, performing diagnosis of diabetes based on the results of the imaging, and treating diabetes on the basis of the diagnosis. The imaging of pancreatic islets and the diagnosis of diabetes can be performed in the same manner as described above. The method for treatment of diabetes may include evaluating the effect of treatment such as medication and diet performed on a subject, focusing on the change in the amount of pancreatic islets. Further, the method for treatment of diabetes disclosed herein may include imaging pancreatic islets and/or determining the amount of pancreatic islets by the method of the present invention, and evaluating functional recovery of the pancreatic islets on the basis of the obtained image of the pancreatic islets and/or the determined amount of the pancreatic islets.

The method for prevention of diabetes as disclosed herein includes imaging of pancreatic islets by use of the peptide derivative of the present invention, and determining the state of the pancreatic islets based on the results of the imaging, so as to determine the risk of development of diabetes. The method for prevention of diabetes disclosed herein may include, for example, regularly determining the amount of pancreatic islets, and checking the presence/absence of a tendency for the amount of pancreatic islets to decrease.

Also disclosed herein is a method for ultra-early diagnosis of diabetes. The method for ultra-early diagnosis of diabetes may include, for example, imaging pancreatic islets and/or determining the amount of pancreatic islets in comprehensive or ordinary medical examination by the method of the present invention, and determining the state of the pancreatic islets on the basis of the obtained image of the pancreatic islets and/or the determined amount of the pancreatic islets.

### [Other Applications]

The peptide derivative of the present invention has the amino acid sequence of exendin-4(1-39) (SEQ ID NO.1) completely or partially. As described above, it is known that exendin-4(1-39) is an analog of GLP-1, and binds to GLP-1R expressed on the pancreatic β-cell. Therefore, the peptide derivative of the present invention can bind to GLP-1R, preferably to GLP-1R in a specific manner. Thus, it can be used in, for example, the imaging and quantification of GLP-1R-positive cells, and diagnosis and treatment of diseases involving the expression of GLP-1R. With the present invention, it is possible to perform imaging and quantification of GLP-1R-positive cells, e.g. imaging, quantification, and the like of pancreatic cells, as well as to perform the diagnosis and/or treatment of diseases involving the expression of GLP-1R. The disease involving the expression of GLP-1R is, for example, a neuroendocrine tumor (NET). Examples of the neuroendocrine tumor include insulinoma, small cell bronchial carcinoma, and pancreatic cancer.

Hereinafter, the present invention will be described further by way of Examples and Reference Examples. It should be noted that the present invention is, when interpreted, not limited to the following Examples.

In the description of the present specification, the following abbreviations are used.
IB: 3-iodobenzoyl
Rink Amide MBHA Resin (trade name, manufactured by Merck & Co., Inc): 4-(2', 4' -Dimethoxyphenyl-Fmoc-aminomethyl)-phenoxyacetamido-norleucyl-MBA
HBTu: 1-[bis(dmiethylamino)methylene]-1H-benzotriazohum-3-oxide-hexafluorophosphate
HOBt: 1-hydroxybenzotriazole
DMF: dimethyl formamide
Boc-mini-PEG-3™ (trade name, manufactured by Peptide International Inc):
Boc-11-Amino-3,6,9-trioxaundecanoic acid·DCHA
Boc: butoxy carbonyl group
DCHA: dicyclohexylamine
WSCD: water-soluble carbodiimide
TFA: tetrahydrofuran
HOSu: N-hydroxysuccinimide
IB-Cl: 3-iodobenzoyl chloride
DIEA: N,N-diisopropyl ethylamine
OBu: butyl ester group
Trt: trityl group
Pdf. 2,2,4,6,7-pentamethyldihydrobenzofaran-,5-sulfonyl group
Mmt: 4-methoxytrityl group
Fmoc: 9-fluorenylmethyloxycarbonyl group
PEG3: -C(O)-CH₂-(OC₂H₄)₃-NH-
ePEG12: -C(O)-C₂H₄-(OC₂H₄)₁₂-NH-
Note that amino acid in L-form was used unless mentioned otherwise.

### Examples

### (Production Example 1)

### Synthesis of Peptide Derivative represented by Formula (6): (IB-PEG₃)12-Ex(9-39)

A peptide derivative represented by the following formula (6) (SEQ ID NO. 6) (hereinafter also referred to as "(IB-PEG₃)12-Ex(9-39)") was prepared as follows. In (IB-PEG₃)12-Ex(9-39), [¹²⁵I]3-iodobenzoyl group ([¹²⁵I]IB) was bound to, through PEG3 as a linker, the amino group of the side chain of the lysine residue at position 4 of the amino acid sequence of SEQ ID NO.4 and the carboxyl group at the C-terminus was amidated.

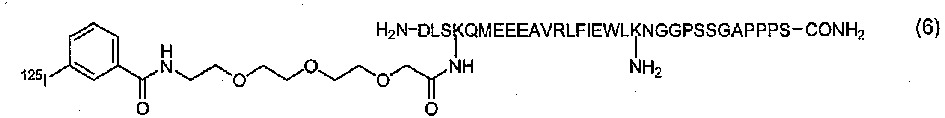

### (1) Synthesis of Protected Peptide ResinA

### Fmoc-DLSK(Boc-PEG3)QMEEEAVRLFIEWLK(Fmoc)NGGPSSGAPPPS-Rink Amide MBHA (7) (SEQ ID NO.7)

A protected peptide resin A represented by formula (7) was synthesized by solid phase synthesis using a peptide synthesizer (ACT90) manufactured by Advanced Chemtech. In formula (7), the protecting groups of the side chains other than Lys(Boc-PEG3) and Lys(Fmoc) are not recited.

Rink Amide MBHAResin (0.39 mol/g, 0.25 mmol scale) was used as a starting resin carrier. Fmoc-amino acid derivatives used in a general Fmoc-peptide synthesis were used as raw materials of amino acids. Among the Fmoc-amino acid derivatives used, Asp(OBu), Ser(OBu), Gln(Trt), Glu(OBu), Trp(Boc), Arg(Pbf) and Asn(Trt) were used for amino acids having functional groups on their side chains and Fmoc-Lys(Boc-PEG3) was used for the lysine at position 4 and Fmoc-Lys(Mmt) was used for the lysine at position 19. The Fmoc-amino acid derivatives as the raw materials were placed in the reaction container of the peptide synthesizer to dissolve them in HOBt and HBTu as activators and DMF, and put in a reaction vessel to let them react with each other. The obtained resin was stirred gently in piperidine-containing N-methylpyrolidone to eliminate the Fmoc group. After rinsing, the next amino acid derivative condensation was performed, where peptide chains were extended one after another in accordance with the peptide sequence, thus obtaining a protected peptide resin A1 represented by the following formula (8). Note that the protecting groups of the side chains other than Lys(Mmt) are not recited in formula (8).

### Fmoc-QMEEEAVRLFIEWLK(Mmt)NGGPSSGAPPPS- RinkAmide MBHA (8) (SEQ ID NO. 8)

Further, the amino acids were extended in accordance with the sequence using Fmoc-Lys(Boc-PEG3), Fmoc-Ser(OBu), Fmoc-Leu, and Fmoc-Asp(OBu) to obtain a protected peptide resin A2 represented by the following formula (9). Note that the protecting groups of the side chains other than Lys(Boc-PEG3) and Lys(Mmt) are not recited in formula (9).

### Fmoc-DLSK(Boc-PEG3)QMEEEAVELFIEWLK(Mmt)NGGPSSGAPPPS-Rink Amide MBHA (9) (SEQ ID NO.9)

Subsequently, the protecting group (Mmt) of the side chain of Lys at position 19 of the protected peptide resin A2 was removed by TFA-TIS-DCM (1.5:5:93.5 v/v), and then the amino group of the side chain of Lys at position 19 was Fmoc-protected using FmocOsu, thus obtaining the protected peptide resin A represented by formula (7).

The Fmoc-Lys(Boc-PEG3) was prepared as follows. Boc-mini-PEG-3™ was dissolved in THF and 0.5N of HCI/ethyl acetate was dropped thereto (pH: 3 to 5). After removing precipitated DCHA salt, the obtained Boc-PEG3 was converted to an activated ester body using WSCD·HCI and HOSu. The activated ester body and Fmoc-Lys were stirred in DMF at ambient temperature (pH: 7 to 8), thus obtaining Fmoc-Lys(Boc- PEG3).

### (2) Deprotection and Excision from Resin

The obtained protected peptide resin A was treated for 2 hours at ambient temperature under typical deprotection conditions using trifluoroacetate (TFA-TIS-H₂O-DT (95/2.5/2.5/2.5, v/v) to perform deprotection and excision of the peptide from the resin at the same time. After filtering the reaction solution to remove the carrier resin, TFA was distilled off. Ether was added to the residue to precipitate a crude product, and then the crude product was obtained by filtration.

### (3) Isolation and Purification of Crude Peptide

The obtained crude peptide was fractionated and purified in a system of water-acetonitrile containing 0.1 % of trifluoroacetate using a preparative high performance liquid chromatograph (HPLC) (trade name: LC-8A-2, manufactured by Shimadzu Corp, column: OSD 30 × 250 mm) to obtain the intended peptide fractions. After distilling off the acetonitrile, the peptide fractions were made into the form of freeze-dried powder, thus obtaining a peptide derivative represented by the following formula (10) (SEQ ID NO. 10) in the form of trifluoroacetate salt. The peptide derivative represented by formula (10) was the labeling precursor of (IB-PEG₃)12-Ex(9-39).

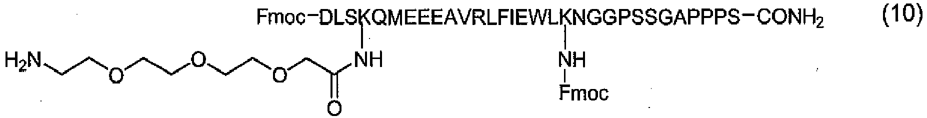

### (4) Labeling of Labeling Precursor

Labeling was performed as follows. The peptide derivative represented by formula (10) (410 µg) was dissolved in acetonitrile and a borate buffer (pH: 7.8), [¹²⁵I]N-succinimidyl 3-iodobenzoate ([¹²⁵I]SIB) was added thereto and the reaction solution was adjusted to have a pH of 8.5 to 9.0 and reacted for 30 minutes. Thereafter, DMF and piperidine were added thereto to carry out a deprotection reaction, thus obtaining the intended (IB-PEG₃)12-Ex(9-39) (the peptide derivative represented by formula (6)) (radiochemical yield: 48.6 %, radiochemical purity: > 99%). Further, the time involved in the labeling was 2.5 hours. Note that the time involved in the labeling refers to time until the intended labeled product was obtained by labeling the labeling precursor and the time includes the reaction time with the labeling compound, the deprotection reaction time after the reaction with the labeling compound, the LC purification time and the concentration time (the same applies to the following).

### (Reference Production Example 1)

A peptide derivative represented by the following formula (11) was prepared. In the peptide derivative represented by formula (11), [¹²⁵I]IB was directly bound to the amino group of the side chain of the lysine residue at position 4 of the amino acid sequence of SEQ ID NO. 4 and the carboxyl group at the C-terminus was amidated.

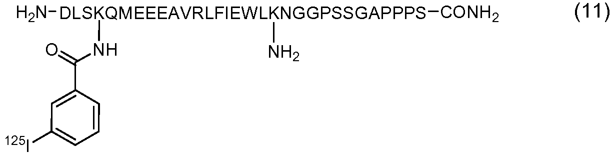

Labeling was performed in the same manner as in Production Example 1 except that a labeling precursor represented by the following formula (12) (1050 µg) was used in place of the peptide derivative represented by formula (10), thus obtaining the intended peptide derivative represented by formula (11) (radiochemical yield: 18.4 %, radiochemical purity: 96.4%). The time involved in the labeling was 5.5 hours.

### Fmoc-DLSKQMEEEAVRLFIEWLK(Fmoc)NGGPSSGAPPPS-CONH₂ (12) (SEQ ID NO. 12)

As Production Example 1 and Reference Production Example 1 show, due to performing the radioactive labeling using the peptide derivative represented by formula (10) in which PEG3 as a linker was bound to the amino group of the side chain of the lysine, the yield of the radioactively labeled peptide derivative significantly improved and the time involved in the labeling was significantly reduced in comparison to the case of using the labeling precursor represented by formula (12) in which PEG3 was not bound to the amino group of the side chain of the lysine. Specifically, the yield was improved by twice or more, from 18.4% to 48.6%. Further, the time involved in the labeling was reduced by 3 hours, from 5.5 hours to 2.5 hours, so that the labeling was performed in half the conventional time. Therefore, with the labeling precursor disclosed herein, labeling can be performed in an efficient manner, so that a radioactively labeled peptide derivative can be provided efficiently.

### (Production Example 2)

### Synthesis of Peptide Derivative represented by Formula (13): (IB-PEG₃)40-Ex(9-39)

A peptide derivative represented by the following formula (13) (SEQ ID NO. 13) (hereinafter also referred to as "(IB-PEG₃)40-Ex(9-39)") was prepared as follows. In (IB-PEG₃)40-Ex(9-39), [¹²⁵I]IB was bound to, through a PEG3 linker, the amino group of the side chain of the lysine residue at position 40 of the amino acid sequence of SEQ ID NO.5 and the carboxyl group at the C-terminus was amidated.

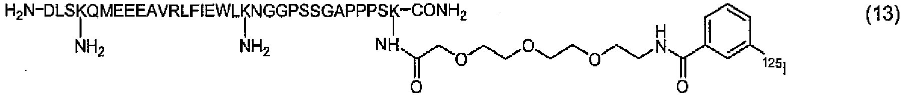

First, a protected peptide resin B represented by the following formula (14) (SEQ ID NO. 14) was synthesized. The protected peptide resin B was synthesized in the same manner as in Production Example 1 except that Fmoc-Lys(Mmt) was used for lysines at positions 4 and 19 and Fmoc-Lys(Boc-PEG3) was used for lysine at position 32. Note that the protecting groups of the side chains other than Lys(Boc-PEG3) and Lys(Fmoc) are not recited in formula (14).

### Fmoc-DLSK(Fmoc)QMEEEAVRLFIEWLK(Fmoc)NGGPSSGAPPPSK(Boc- PEG3)-Rink Amide MBHA (14) (SEQ ID NO. 14)

In the same manner as in Production Example 1, the obtained protected peptide resin B was deprotected and excised from the resin. Also, the peptide derivative was isolated and purified. Thus, the peptide derivative represented by the following formula (15) was obtained in the form of trifluoroacetate salt (freeze-dried powder). The peptide derivative represented by formula (15) was the labeling precursor of (IB-PEG₃)40-Ex(9-39).

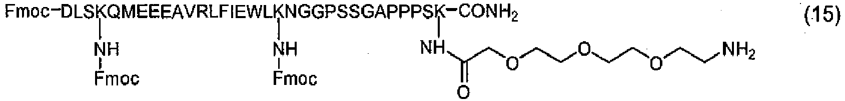

Then, the peptide derivative represented by formula (15) (580 pg) was labeled and deprotected in the same manner as in Production Example 1, thus obtaining the intended (IB-PEG₃)40-Ex(9-39) (the peptide derivative represented by formula (11)) (radiochemical yield: 30.1%, radiochemical purity: 96.7%). The time involved in the labeling was 4 hours.

### (Reference Production Example 2)

A peptide derivative represented by the following formula (16) (SEQ ID NO. 16) was prepared. In the peptide derivative represented by formula (16), [¹²⁵I]IB was directly bound to the amino group of the side chain of the lysine residue at position 32 of the amino acid sequence of SEQ ID NO. 5 and the carboxyl group at the C-terminus was amidated.

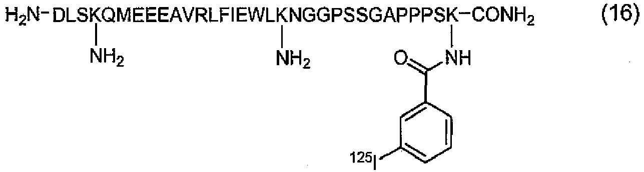

Labeling was performed in the same manner as in Production Example 1 except that a labeling precursor represented by the following formula (17) (400 pg) was used in place of the peptide derivative represented by formula (10), thus obtaining the intended peptide derivative represented by formula (16) (radiochemical yield: 33.6%, radiochemical purity: > 99%). Further, the time involved in the labeling was 6 hours.

### Fmoc-DLSK(Fmoc)QMEEEAVRLFIEWLK(Fmoc)NGGPSSGAPPPSK-CONH₂ (17) (SEQ ID NO. 17)

### (Example 1)

Biodistribution experiments and two-dimensional imaging analysis were performed using the peptide derivative represented by formula (6)
((IB-PEG₃)12-Ex(9-39)).

### [Biodistribution]

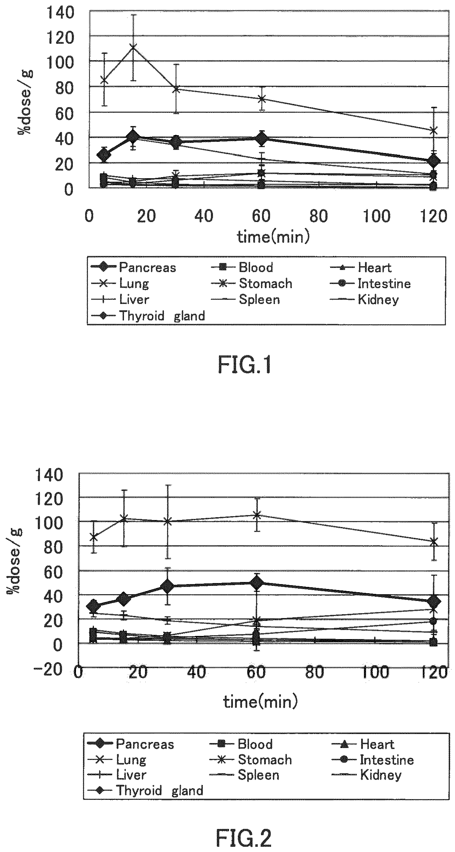

(IB-PEG₃)12-Ex(9-39) (0.93 µCi) was administered to unanesthetized 6-week-old ddY mice (male, weight: 30g) by intravenous injection (through the tail vein). After 5 minutes, 15 minutes, 30 minutes, 60 minutes, and 120 minutes from the administration, organs were dissected out of the mice (n=5). The weight and the radioactivity of each organ were determined, and an accumulation amount (%dose/g) of (IB-PEG₃)12-Ex(9-39) was calculated from the radioactivity per unit weight. The exemplary results are shown in Table 1 and FIG. 1. FIG. 1 is a graph showing, by way of example, how the accumulation of (IB-PEG₃)12-Ex(9-39) in each organ varied with time.

| (Table 1) | Time after administration | | | | |
|---|---|---|---|---|---|
| | 5min | 15min | 30min | 60min | 120min |
| Pancreas | 26.20 (5.96) | 40.21 (7.72) | 35.77 (5.23) | 38.70 (6.09) | 21.49 (7.71) |
| Blood | 8.35 (1.08) | 4.35 (0.56) | 2.64 (0.62) | 1.55 (0.14) | 0.68 (0.08) |
| Heart | 4.35 (0.61) | 2.52 (0.40) | 1.90 (0.34) | 1.33 (0.30) | 0.55 (0.18) |
| Lung | 85.31 (20.79) | 110.66 (25.84) | 77.81 (19.43) | 70.41 (9.15) | 45.36 (18.09) |
| Stomach | 3.77 (1.00) | 4.77 (1.13) | 9.52 (4.36) | 11.87 (5.65) | 8.74 (4.18) |
| Intestine | 2.83 (0.29) | 3.47 (0.48) | 6.12 (0.83) | 11.58 (1.24) | 10.74 (2.00) |
| Liver | 9.74 (0.93) | 7.26 (0.69) | 7.03 (0.65) | 5.57 (0.96) | 2.17 (0.38) |
| Spleen | 2.96 (0.46) | 2.43 (0.62) | 1.44 (0.11) | 1.11 (0.15) | 0.42 (0.06) |
| Kidney | 26.29 (2.64) | 38.98 (9.27) | 34.00 (2.23) | 22.83 (4.76) | 10.98 (2.61) |
| Thyroid gland | 5.24 (1.26) | 2.89 (1.12) | 2.05 (0.45) | 2.63 (0.32) | 2.93 (1.09) |

| | | | | | |
|---|---|---|---|---|---|
| Each numerical value indicates an average (SD) of 5 mice. | | | | | |

As shown in Table 1 and FIG. 1, the accumulation of (IB-PEG₃)12-Ex(9-39) in the pancreas reached a level exceeding 25 %dose/g at an early stage after administration, and the accumulation was maintained at a high level thereafter. In particular, during the time period between 15 minutes and 60 minutes after administration, the accumulation was greater than 35 %dose/g. In terms of the accumulation per unit weight, (IB-PEG₃)12-Ex(9-39) accumulated the most in the pancreas other than the lung during the time period from 15 minutes after administration. Further, no great change was seen in the accumulation of (IB-PEG₃)12-Ex(9-39) in the thyroid gland. This suggests that the peptide derivative was not subjected to deiodization metabolism *in vivo.* For these reasons, (IB-PEG₃)12-Ex(9-39) is considered suitable for non-invasive imaging, particularly non-invasive imaging of pancreatic β-cells.

### (Reference Example 1)

As Reference Example 1, the peptide derivative represented by formula (11) and prepared in Reference Production Example 1 was used to determine the biodistribution of the peptide derivative in mice in the same manner as in Example 1. The exemplary results are provided in Table 2 and FIG. 2.

| (Table 2) | Time after administration | | | | |
|---|---|---|---|---|---|
| | 5min | 15min | 30min | 60min | 120min |
| Pancreas | 30.26 (5.12) | 36.10 (3.53) | 46.87 (15.31) | 49.94 (7.37) | 34.36 (4.76) |
| Blood | 9.37 (0.26) | 6.72 (0.98) | 4.34 (0.77) | 2.57 (0.28) | 1.33 (0.20) |
| Heart | 4.89 (0.41) | 3.42 (0.65) | 2.39 (0.38) | 1.87 (0.36) | 0.99 (0.28) |
| Lung | 87.32 (13.16) | 102.62 (23.24) | 99.94 (30.05) | 105.47 (13.68) | 83.77 (15.02) |
| Stomach | 3.39 (1.37) | 4.23 (0.53) | 6.10 (2.34) | 18.45 (24.20) | 27.98 (27.99) |
| Intestine | 3.23 (0.44) | 3.53 (0.72) | 4.56 (0.51) | 7.66 (3.41) | 17.81 (19.02) |
| Liver | 11.34 (1.10) | 8.03 (1.29) | 5.83 (0.56) | 3.74 (0.64) | 2.22 (0.51) |
| Spleen | 4.14 (0.86) | 3.03 (0.47) | 2.37 (0.30) | 1.61 (0.26) | 0.85 (0.20) |
| Kidney | 24.77 (3.21) | 23.02 (3.51) | 18.58 (2.87) | 13.92 (1.70) | 9.14 (1.74) |
| Thyroid gland | 4.68 (0.98) | 3.95 (0.54) | 3.65 (0.62) | 3.14 (1.31) | 2.24 (0.60) |

| | | | | | |
|---|---|---|---|---|---|
| Each numerical value indicates an average (SD) of 5 mice. | | | | | |

As shown in Tables 1 and 2, in comparison to the peptide derivative represented by formula (11), the peptide derivative represented by formula (6) ((IB-PEG₃)12-Ex(9-39)) accumulated less in the liver as an adjacent organ to the pancreas at an early stage after administration as well as in blood.

Based on the accumulation amount in each organ during the biodistribution experiments in Example 1 and Reference Example 1, the ratio of pancreas/liver (accumulation amount in pancreas/accumulation amount in liver) for each peptide derivative is shown in Table 3, the ratio of pancreas/kidney (accumulation amount in pancreas/accumulation amount in kidney) for each peptide derivative is shown in Table 4, and the ratio of pancreas/blood (accumulation amount in pancreas/accumulation amount in blood) for each peptide derivative is shown in Table 5.

**(Table 3) Pancreas/Liver Ratio**

| | Time after administration | | | | |
|---|---|---|---|---|---|
| | 5min | 15min | 30min | 60min | 120min |
| Ex. 1 | 2.74 (0.84) | 5.61 (1.33) | 5.16 (1.12) | 7.00 (0.80) | 9.71 (1.94) |
| Ref Ex. 1 | 2.67 (0.45) | 4.56 (0.62) | 8.16 (2.81) | 13.47 (1.51) | 15.95 (3.54) |

**(Table 4) Pancreas/Kidney Ratio**

| | Time after administration | | | | |
|---|---|---|---|---|---|
| | 5min | 15min | 30min | 60min | 120min |
| Ex. 1 | 0.99 (0.13) | 1.07 (0.28) | 1.06 (0.17) | 1.75 (0.47) | 1.99 (0.65) |
| Ref. Ex.1 | 1.23 (0.19) | 1.60 (0.28) | 2.59 (1.01) | 3.61 (0.54) | 3.88 (0.94) |

**(Table 5) Pancreas / Blood Ratio**

| | Time after administration | | | | |
|---|---|---|---|---|---|
| | 5min | 15min | 30min | 60min | 120min |
| Ex. 1 | 3.19 (0.94) | 9.40 (2.29) | 14.57 (5.59) | 24.96 (2.96) | 31.08 (7.49) |
| Ref. Ex. 1 | 3.22 (0.47) | 5.44 (0.71) | 11.47 (4.97) | 19.49 (2.16) | 26.21 (4.42) |

As shown in Tables 3 and 4, the ratio of pancreas/liver and the ratio of pancreas/kidney for (IB-PEG₃)12-Ex(9-39) increased with time, and the ratio of pancreas/liver was greater than 2 at an early stage after administration. As shown in Table 5, the ratio of pancreas/blood for (IB-PEG₃)12-Ex(9-39) increased remarkably with time in comparison to that for the peptide derivative represented by formula (11). The ratio of pancreas/blood for (IB-PEG₃)12-Ex(9-39) was greater than 3 at an early stage after administration, and showed a satisfactory blood clearance. Thus, the results suggest that clear images of pancreatic β-cells can be obtained when performing imaging using (IB-PEG₃)12-Ex(9-39), which accumulates in the pancreas in a large amount while accumulating less in the organs surrounding the pancreas and has excellent blood clearance.

### [Two-Dimensional Imaging Analysis]

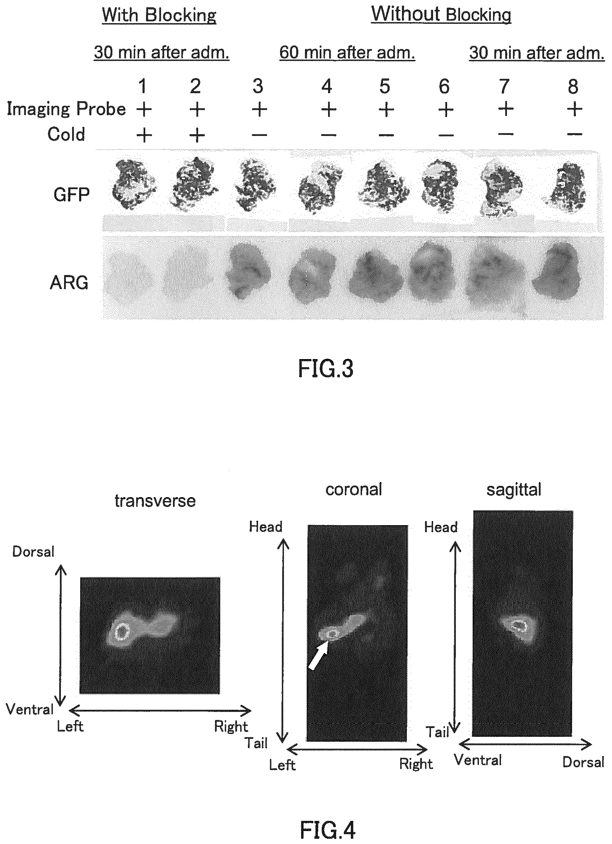

(IB-PEG₃)12-Ex(9-39) (5 µCi/100 µl) was administered to an unanesthetized MIP-GFP mouse (male, weight: 20g) by intravenous injection, and after 30 minutes and 60 minutes from the administration, the pancreas was dissected out of the mouse (n=1). Sections were cut out of the dissected pancreas, and each section was placed on a slide glass, covered with a cover glass. The fluorescence and radioactivity (autoradiography) of each section were determined using an image analyzer (trade name: Typhoon 9410, manufactured by GE Health Care Inc.) (exposure time: 21 hours). The exemplary results are shown in lanes 3 to 8 of FIG. 3.

Further, non-labeled exendin(9-39) (cold probe, SEQ ID NO. 21) was first administered to unanesthetized MIP-GFP mice (male, weight: 20g) by intravenous injection (50 µg/100 µl). After 30 minutes from the preliminary administration, (IB-PEG₃)12-Ex(9-39) (5 µCi/100 µl) was administered to the mice by intravenous injection, and after 30 minutes from the administration of (IB-PEG₃)12-Ex(9-39), the pancreases were dissected out of the mice (n=2). Sections were cut out of the dissected pancreases, and the fluorescence and radioactivity of each section were determined in the same manner as in the above. The results are shown in lanes 1 and 2 of FIG. 3.

FIG. 3 illustrates exemplary results of the imaging analysis of the pancreas sections of the MIP-GFP mice to which (IB-PEG₃)12-Ex(9-39) was administered. The images shown therein are images showing the fluorescence signal (upper row) and the radioactivity signal (lower row) of (IB-PEG₃)12-Ex(9-39). In FIG. 3, the wording "with blocking" indicates the results where the cold probe was administered prior to administration of (IB-PEG₃)12-Ex(9-39) and the wording "without blocking" indicates results where (IB-PEG₃)12-Ex(9-39) was administered without first administering the cold probe.

Here, all of ¹²⁵I, ¹²³I, and ¹³¹I were γ-ray emitting nuclides. Still further, ¹²⁵I and ¹²³I have the same numbers of nuclear spin. In view of this, it can be presumed that even when (IB-PEG₃)12-Ex(9-39) is modified to replace its radioactive iodine atom with an [¹²³I] iodine atom or an [¹³¹I] iodine atom, it will exhibit behaviors substantially identical to those of (IB-PEG₃)12-Ex(9-39). Further, it can also be presumed that even when (IB-PEG₃)12-Ex(9-39) is modified to replace its radioactive iodine atom with an [¹²⁴I] iodine atom, it will exhibit behaviors substantially identical to those of (IB-PEG₃)12-Ex(9-39). Thus, it was suggested that using (IB-PEG₃)12-Ex(9-39) obtained by replacing its [¹²⁵I] iodine atom with an [^{123/121/131}I] iodine atom, the non-invasive three-dimensional imaging of GLP-1R of pancreatic β-cells by SPECT, PET, or the like, for example, is enabled, and preferably, the quantification of GLP-1R of pancreatic β-cells is enabled.

### (Example 2)

Three-dimensional SPECT imaging was performed using a peptide derivative represented by the following formula (18) (SEQ ID NO. 18) as follows. In the peptide derivative represented by formula (18), [¹²³I]3-iodobenzoyl group ([¹²³I]IB) was bound, through a PEG3 linker, to the amino group of the side chain of the lysine residue at position 4 of the amino acid sequence of SEQ ID NO. 4 and the carboxyl group at the C-terminus was amidated.

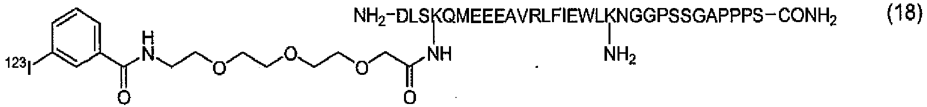

### [Preparation of Peptide Derivative]

The peptide derivative represented by formula (18) was prepared in the same manner as in Production Example 1 except that [¹²³I]SIB was used in place of [¹²⁵I]SIB.

### [Three-Dimensional Imaging]

With use of the peptide derivative represented by formula (18), SPECT imaging of mice was performed. The peptide derivative represented by formula (18) (491 µCi (18.2 MBq)/120 µl) was administered to 6-week-old ddY mice (male, weight: about 30g) by intravenous injection, and then after 20 minutes from the administration of the peptide derivative the mice were subjected to inhalation anesthesia with enflurane. After 30 minutes from the administration of the peptide derivative, the SPECT imaging was performed under the following imaging conditions with use of a gamma camera (product name: SPECT 2000H-40, manufactured by Hitachi Medical Corporation). Images obtained were reconfigured under the following reconfiguration condition.

### Imaging Conditions

| | |
|---|---|
| Collimator: | LEPH collimator |
| Collecting range: | 360° |
| Step angle: | 11.25° |
| Collecting time: | 60 sec per direction |
| | 1 × 32 frames per 60 sec (total: 32 min) |

### Reconfiguration Condition

| | |
|---|---|
| Preprocessing filter: | Butterworth filter (order: 10, cutoff frequency: 0.10) |

The exemplary results are shown in FIG. 4. The images shown in FIG. 4 were taken 30 minutes after the administration of the peptide derivative. Shown in FIG. 4 are, starting from the left, a transverse view, a coronal view and a sagittal view. In the coronal view of FIG. 4, the position of the pancreas is indicated by a white arrow.

As shown in FIG. 4, the position of the pancreas was confirmed non-invasively in mice as a result of SPECT imaging using the peptide derivative represented by formula (18). Thus, in view of the fact that the position of the pancreas was confirmed non-invasively in a mouse that has a pancreas of a smaller size than that of a human and in which the organs are present more densely than in a human, this suggests that in a human that has a pancreas of a greater size than that of a mouse and in which the organs are present not as densely as in a mouse, the position of the pancreas and the size of the pancreas can be determined more clearly, and moreover, the amount of the peptide derivative binding to GLP-1R of pancreatic β-cells can be determined.

These results suggest that the peptide derivative of the present invention allows non-invasive three-dimensional imaging of pancreatic islets, particularly three-dimensional imaging of pancreatic β-cells or non-invasive three-dimensional imaging of GLP-1R of pancreatic β-cells, in a human.

### (Example 3)

Biodistribution experiments and two-dimensional imaging analysis were performed using the peptide derivative represented by formula (13)
((IB-PEG₃)40-Ex(9-39)).

### [Biodistribution]

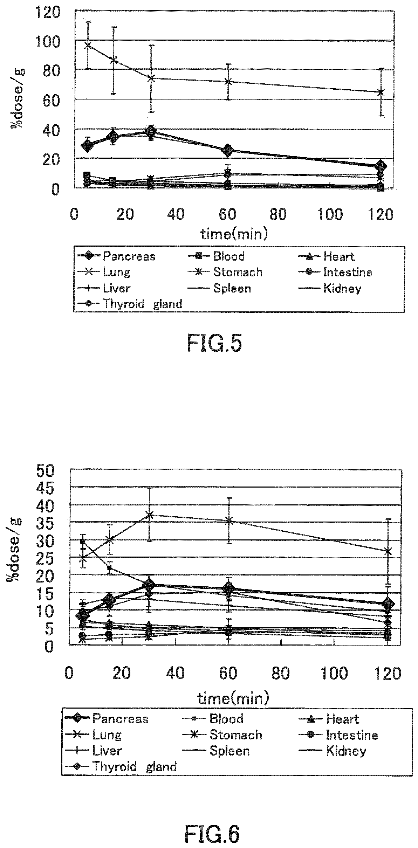

(IB-PEG₃)40-Ex(9-39) (0.69 µCi) was administered to unanesthetized 6-week-old ddY mice (male, weight: 30g) by intravenous injection (through the tail vein). After 5 minutes, 15 minutes, 30 minutes, 60 minutes, and 120 minutes from the administration, organs were dissected out of the mice (n=5). The weight and the radioactivity of each organ were determined, and an accumulation amount (%dose/g) of (IB-PEG₃)40-Ex(9-39) was calculated from the radioactivity per unit weight. The exemplary results are shown in Table 6 and FIG. 5. FIG. 5 is a graph showing, by way of example, how the accumulation of (IB-PEG₃)40-Ex(9-39) in each organ varied with time.

| (Table 6) | Time after administration | | | | |
|---|---|---|---|---|---|
| | 5min | 15min | 30min | 60min | 120min |
| Pancreas | 28.67 (2.94) | 34.58 (3.02) | 37.93 (4.22) | 25.46 (3.66) | 14.84 (3.10) |
| Blood | 8.15 (1.13) | 4.80 (0.40) | 3.19 (0.32) | 1.80 (0.29) | 0.93 (0.07) |
| Heart | 4.43 (0.60) | 2.70 (0.51) | 1.86 (0.26) | 1.11 (0.24) | 0.61 (0.10) |
| Lung | 96.22 (15.76) | 86.15 (22.61) | 73.84 (22.41) | 71.65 (12.12) | 64.92 (16.02) |
| Stomach | 3.24 (0.74) | 4.08 (1.57) | 5.96 (1.66) | 10.17 (5.71) | 6.99 (4.24) |
| Intestine | 2.65 (0.45) | 2.99 (0.89) | 4.45 (0.97) | 8.81 (3.51) | 9.13 (2.36) |
| Liver | 5.39 (0.46) | 4.76 (0.56) | 4.53 (0.16) | 2.99 (0.42) | 1.64 (0.14) |
| Spleen | 3.05 (0.50) | 1.91 (0.24) | 1.24 (0.10) | 0.81 (0.14) | 0.42 (0.07) |
| Kidney | 29.83 (4.46) | 35.04 (5.69) | 35.15 (2.65) | 25.89 (2.31) | 14.03 (2.84) |
| Thyroid gland | 4.61 (1.97) | 3.04 (0.48) | 2.51 (1.06) | 1.29 (0.67) | 2.07 (0.91) |

| | | | | | |
|---|---|---|---|---|---|
| Each numerical value indicates an average (SD) of 5 mice. | | | | | |

As shown in Table 6 and FIG. 5, the accumulation of (IB-PEG₃)40-Ex(9-39) in the pancreas reached a level exceeding 25 %dose/g at an early stage after administration, and the accumulation was maintained at a high level thereafter. Further, no great change was seen in the accumulation of (IB-PEG₃)40-Ex(9-39) in the thyroid gland. This suggests that the peptide derivative was not subjected to deiodization metabolism *in vivo.* For these reasons, (IB-PEG₃)40-Ex(9-39) is considered suitable for non-invasive imaging, particularly non-invasive imaging of GLP-1R of pancreatic β-cells.

### (Reference Example 2)

As Reference Example 2, the peptide derivative represented by formula (16) and produced in Reference Production Example 2 was used to determine the biodistribution of the peptide derivative in mice in the same manner as in Example 2. The exemplary results are provided in Table 7 and FIG. 6.

| (Table 7) | Time after administration | | | | |
|---|---|---|---|---|---|
| | 5min | 15min | 30min | 60min | 120min |
| Pancreas | 8.28 (0.96) | 12.80 (1.49) | 17.20 (1.22) | 16.18 (3.03) | 11.82 (5.00) |
| Blood | 29.39 (2.05) | 21.99 (1.54) | 17.24 (0.91) | 14.10 (1.41) | 9.62 (1.82) |
| Heart | 6.28 (0.37) | 6.27 (0.45) | 5.71 (0.20) | 4.99 (0.56) | 3.10 (0.52) |
| Lung | 24.64 (2.51) | 30.00 (4.21) | 37.09 (7.49) | 35.52 (6.50) | 26.83 (9.26) |
| Stomach | 1.61 (0.10) | 2.06 (0.32) | 2.29 (0.77) | 4.62 (2.89) | 4.23 (2.28) |
| Intestine | 2.57 (0.35) | 2.90 (0.29) | 3.06 (0.23) | 3.59 (0.80) | 3.77 (1.81) |
| Liver | 7.29 (0.75) | 5.73 (0.49) | 4.64 (0.81) | 4.04 (0.55) | 2.93 (0.83) |
| Spleen | 5.41 (0.98) | 4.71 (0.45) | 4.08 (0.36) | 3.32 (0.46) | 2.18 (0.54) |
| Kidney | 11.57 (0.51) | 13.39 (0.56) | 12.92 (1.96) | 11.14 (1.75) | 8.22 (2.18) |
| Thyroid gland | 9.61 (3.36) | 11.07 (2.78) | 14.63 (5.30) | 15.17 (2.54) | 6.55 (2.83) |

| | | | | | |
|---|---|---|---|---|---|
| Each numerical value indicates an average (SD) of 5 mice. | | | | | |

As shown in Tables 6 and 7, in comparison to the peptide derivative represented by formula (16), the accumulation of the peptide derivative represented by formula (13) ((IB-PEG₃)40-Ex(9-39)) in the pancreas was extremely large while the accumulation in the liver as an adjacent organ to the pancreas was small. Consequently, (IB-PEG₃)40-Ex(9-39) is considered to have excellent organ specificity for the pancreas in comparison to the peptide derivative represented by formula (16).

Based on the accumulation amount in each organ during the biodistribution experiments in Example 3 and Reference Example 2, the ratio of pancreas/liver for each peptide derivative is shown in Table 8, the ratio of pancreas/kidney for each peptide derivative is shown in Table 9, and the ratio of pancreas/blood for each peptide derivative is shown in Table 10.

**(Table 8) Pancreas/Liver Ratio**

| | Time after administration | | | | |
|---|---|---|---|---|---|
| | 5min | 15min | 30min | 60min | 120min |
| Ex. 3 | 5.34 (0.51) | 7.34 (1.02) | 8.37 (0.94) | 8.65 (1.84) | 9.04 (1.72) |
| Ref. Ex. 2 | 1.15 (0.24) | 2.25 (0.32) | 3.80 (0.70) | 4.01 (0.56) | 3.99 (1.18) |

**(Table 9) Pancreas/Kidney Ratio**

| | Time after administration | | | | |
|---|---|---|---|---|---|
| | 5min | 15min | 30min | 60min | 120min |
| Ex. 3 | 0.98 (0.18) | 1.01 (0.21) | 1.09 (0.19) | 0.99 (0.20) | 1.09 (0.32) |
| Ref. Ex. 2 | 0.72 (0.08) | 0.96 (0.12) | 1.36 (0.27) | 1.46 (0.20) | 1.39 (0.31) |

**(Table 10) Pancreas/Blood Ratio**

| | Time after administration | | | | |
|---|---|---|---|---|---|
| | 5min | 15min | 30min | 60min | 120min |
| Ex. 3 | 3.57 (0.58) | 7.24 (0.82) | 12.07 (2.29) | 14.49 (3.54) | 15.85 (2.46) |
| Ref. Ex. 2 | 0.28 (0.04) | 0.58 (0.08) | 1.00 (0.08) | 1.14 (0.15) | 1.22 (0.45) |

As shown in Table 8, the results show that the ratio of pancreas/liver for (IB-PEG₃)40-Ex(9-39) was greater than 5 at an early stage after administration. In particular, during the time period between 5 minutes and 30 minutes after administration, the ratio of pancreas/liver for (IB-PEG₃)40-Ex(9-39) was more than 5 times as much as the ratio of pancreas/liver for the peptide derivative represented by formula (16). Further, as shown in Table 9, the ratio of pancreas/kidney for (IB-PEG₃)40-Ex(9-39) reached approximately 1 at an early stage after administration. As shown in Table 10, the ratio of pancreas/blood for (IB-PEG₃)40-Ex(9-39) was considerably higher than that for the peptide derivative represented by formula (16), was greater than 3.5 at an early stage after administration, and showed a satisfactory blood clearance. Thus, the results suggest that clear images of pancreatic β-cells can be obtained when performing imaging using (IBPEG₃)40-Ex(9-39), which accumulates in the pancreas in a large amount while accumulating less in the organs surrounding the pancreas and has excellent blood clearance.

### [Two-Dimensional Imaging Analysis]

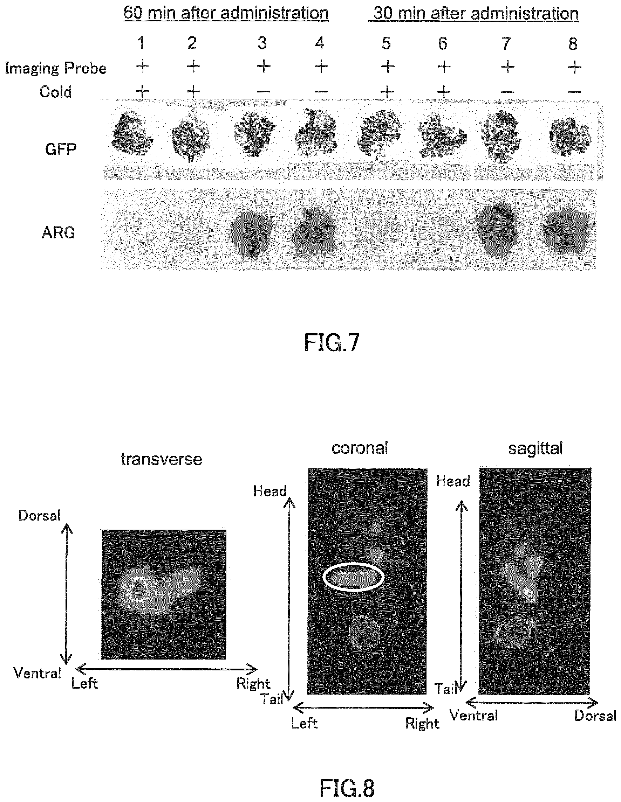

(IB-PEG₃)40-Ex(9-39) (5 µCi/100 µl) was administered to an unanesthetized MIP-GFP mouse (male, weight: 20g) by intravenous injection, and 30 minutes and 60 minutes after administration, the pancreas was dissected out of the mouse (n=1). Sections were cut out of the dissected pancreas, and each section was placed on a slide glass, covered with a cover glass. The fluorescence and radioactivity (autoradiography) of each section were determined using an image analyzer (trade name: Typhoon 9410, manufactured by GE Health Care Inc.) (exposure time: 19 hours). The exemplary results are shown in lanes 3,4,7 and 8 of FIG. 7.

Further, non-labeled exendin(9-39) (cold probe, SEQ ID NO. 20) was first administered to an unanesthetized MIP-GFP mouse (male, weight: 20g) by intravenous injection (50 µg/100 µl). After 30 minutes from the preliminary administration, (IB-PEG₃)40-Ex(9-39) (5 µCi/100 µl) was administered to the mouse by intravenous injection, and after 30 minutes from the administration of (IB-PEG₃)40-Ex(9-39), the pancreas was dissected out of the mouse (n=1). Sections were cut out of the dissected pancreas, and the fluorescence and radioactivity of each section were determined in the same manner as above. The exemplary results are shown in lanes 1, 2, 5 and 6 of FIG. 7.

FIG. 7 illustrates exemplary results of the imaging analysis of the pancreas sections of the MIP-GFP mice to which (IB-PEG₃)40-Ex(9-39) was administered. The images shown therein are images showing the fluorescence signal (upper row) and the radioactivity signal (lower row) of (IB-PEG₃)40-Ex(9-39). In FIG. 7, the wording "with blocking" indicates that the results are those in which the cold probe was administered prior to the administration of (IB-PEG₃)40-Ex(9-39) and the wording "without blocking" indicates that the results are those in which (IB-PEG₃)40-Ex(9-39) was administered without first administering the cold probe.

From the results described above, it was suggested that, similar to (IB-PEG₃)12-Ex(9-39), use of (IB-PEG₃)40-Ex(9-39) obtained by replacing its [¹²⁵I] iodine atom with an [^{123/124/131}I] iodine atom allows, for example, the non-invasive three-dimensional imaging of GLP-1R of pancreatic β-cells by SPECT, PET, or the like preferably, the quantification of GLP-1R of pancreatic β-cells.

### (Example 4)

Three-dimensional SPECT imaging was performed using a peptide derivative represented by the following formula (19) (SEQ ID NO. 19) as follows. In the peptide derivative represented by formula (19), [¹²³I]3-iodobenzoyl group ([¹²³I]IB) was bound, through a PEG3 linker, to the amino group of the side chain of the lysine residue at position 32 of the amino acid sequence of SEQ ID NO. 5 and the carboxyl group at the C-terminus was amidated.

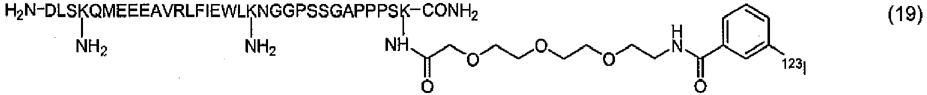

### [Preparation of Peptide Derivative]

The peptide derivative represented by formula (19) was prepared in the same manner as in Production Example 2 except that [¹²³I]SIB was used in place of [¹²⁵I] SIB.

### [Three-Dimensional Imaging]

With use of the peptide derivative represented by formula (19), SPECT imaging of mice was performed. The peptide derivative represented by formula (18) (500 µCi (18.2 MBq)) was administered to 6-week-old ddY mice (male, weight: about 30g) by intravenous injection, and then after 20 minutes from the administration of the peptide derivative the mice were subjected to inhalation anesthesia with enflurane. After 30 minutes from the administration of the peptide derivative, the SPECT imaging was performed under the following imaging conditions with use of a gamma camera (product name: SPECT 2000H-40, manufactured by Hitachi Medical Corporation). Images obtained were reconfigured under the following reconfiguration condition.

### Imaging Conditions

| | |
|---|---|
| Collimator: | LEPH collimator |
| Collecting range: | 360° |
| Step angle: | 11.25° |
| Collecting time: | 60 sec per direction |
| | 1 × 32 frames per 60 sec (total: 32 min) |

### Reconfiguration Condition

| | |
|---|---|
| Preprocessing filter: | Butterworth filter (order: 10, cutoff frequency: 0.14) |

The exemplary results are shown in FIG. 8. The images shown in FIG. 8 were taken 30 minutes after the administration of the peptide derivative. Shown in FIG. 8 are, starting from the left, a transverse view, a coronal view and a sagittal view. In the coronal view of FIG. 8, the portion circled with a white line indicates the position of the pancreas.

As shown in FIG. 8, the position of the pancreas was confirmed non-invasively in mice as a result of the SPEC imaging using the peptide derivative represented by formula (19). Thus, in view of the fact that the position of the pancreas was confirmed non-invasively in a mouse that has a pancreas of a smaller size than that of a human and in which the organs are present more densely than in a human, this suggests that in a human that has a pancreas of a greater size than that of a mouse and in which the organs are present not as densely as in a mouse, the position of the pancreas and the size of the pancreas can be determined more clearly, and moreover, the amount of the peptide derivative binding to GLP-1R of pancreatic β-cells can be determined.

These results suggest that the peptide derivative of the present invention allows non-invasive three-dimensional imaging of pancreatic islets, particularly three-dimensional imaging of pancreatic β-cells or non-invasive three-dimensional imaging of GLP-1R of pancreatic β-cells, in a human.

### (Production Example 3)

### Synthesis of Peptide Derivative represented by Formula (21): (FB-PEG₃)12-Ex4

A peptide derivative represented by the following formula (21) (SEQ ID NO. 21) (hereinafter also referred to as "(FB-PEG₃)12-Ex4") was prepared.

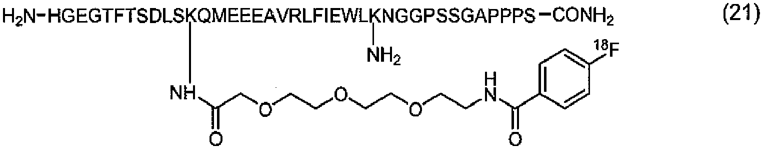

First, a peptide derivative represented by the following formula (22) (SEQ ID NO. 22, labeling precursor) was prepared using the same procedure as (1) to (3) of Production Example 1, except that the amino acid sequence of exendin4 (SEQ ID NO. 1) was adopted as the base sequence of the peptide to be synthesized.

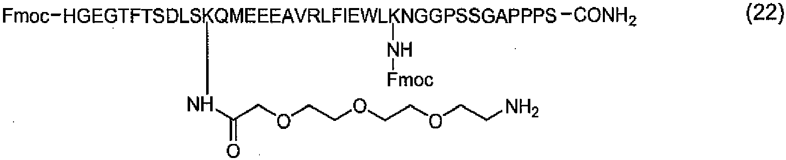

Next, labeling was performed as follows. The peptide derivative represented by formula (22) (470 pg) was dissolved in a borate buffer (pH: 7.8), [¹⁸F]N-succinimidyl 4-fluorobenzoate ([¹⁸F]SFB) was added thereto and the reaction solution was adjusted to have a pH of 8.5 to 9.0 and reacted for 40 minutes. Thereafter, DMF and piperidine were added thereto to carry out a deprotection reaction, thus obtaining the intended (FB-PEG₃)12-Ex4 (the peptide derivative represented by formula (20)) (radiochemical yield: 10.0 %, radiochemical purity: > 99%). Further, the time involved in the labeling was 2 hours.

### (Reference Production Example 3)

Labeling was performed in the same manner as in Production Example 3 except that a labeling precursor represented by the following formula (23) (220 pg) was used in place of the peptide derivative represented by formula (22) and the reaction time with [¹⁸F]SFB was changed to 73 minutes, thus obtaining the intended peptide derivative represented by formula (24) (SEQ ID NO. 24) (radiochemical yield: 1.3 %, radiochemical purity: > 99%). Further, the time involved in the labeling was 2.9 hours.

### Fmoc-HGEGTFTSDLSKQMEEEAVRLFIEWLK(Fmoc)NGGPSSGAPPPS-CONH₂ (23) (SEQ ID NO. 23)

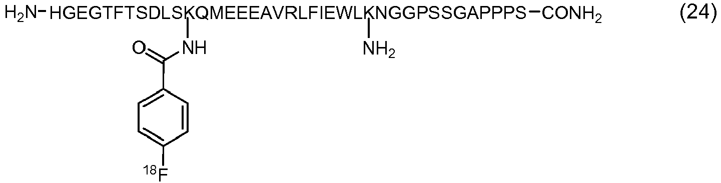

As Production Example 3 and Reference Production Example 3 show, as a result of radioactively labeling the peptide derivative (labeling precursor) in which PEG3 was bound to the amino group of the side chain of Lys12, the yield of the radioactively labeled peptide derivative improved by seven times or more and the time involved in the labeling was reduced by approximately one hour in comparison to the labeling precursor of formula (23) in which PEG3 was not bound to the amino group of the side chain of the lysine. Therefore, with the labeling precursor disclosed herein, it is possible to produce a peptide derivative that is radioactively labeled in an efficient manner.

### (Production Example 4)

### Synthesis of Peptide Derivative represented by Formula (25): (IB-PEG₃)12-Ex4

Labeling was performed in the same manner as in (4) of Production Example 1 except that the peptide derivative represented by formula (22) (240 µg) was used as the labeling precursor in place of the peptide derivative represented by formula (10), thus obtaining the intended (IB-PEG₃)12-Ex4 (a peptide derivative represented by formula (25), (SEQ ID NO. 25)) (radiochemical yield: 18.7 %, radiochemical purity: > 95.1%). Further, the time involved in the labeling was 3.5 hours. Note that the time involved in the labeling includes the reaction time with the labeling compound, the HPLC purification time, the deprotection reaction time after the reaction with the labeling compound, the LC purification time and the concentration time.

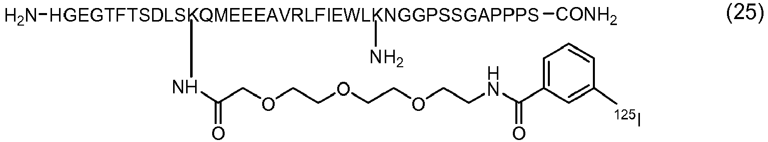

### (Reference Production Example 4)

Labeling was performed in the same manner as in Production Example 4 except that the labeling precursor represented by formula (23) (570 µg) was used in place of the peptide derivative represented by formula (22) and the reaction time with [¹²⁵I]SIB was changed to 174 minutes, thus obtaining an intended peptide derivative represented by formula (26) (SEQ ID NO. 26) (radiochemical yield: 18.4 %, radiochemical purity: > 97.2%). Further, the time involved in the labeling was 4.6 hours.

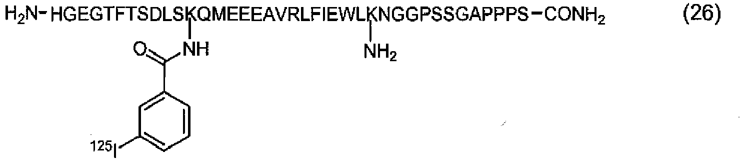

As Production Example 4 and Reference Production Example 4 show, as a result of radioactively labeling the peptide derivative of formula (22) in which PEG3 was bound to the amino group of the side chain of Lys12, the purity of the radioactively labeled peptide derivative obtained was improved and the time involved in the labeling was reduced by 1.1 hours in comparison to using the labeling precursor of formula (23) in which PEG3 was not bound to the amino group of the side chain of the lysine.

### (Production Example 5)

### Synthesis of Peptide Derivative represented by Formula (27): (IB-PEG₃)40-Ex4

Labeling was performed in the same manner as in (4) of Production Example 1 except that a peptide derivative represented by formula (28) (SEQ ID NO. 28) (320 pg) was used as the labeling precursor in place of the peptide derivative represented by formula (10) and the reaction time with [¹²⁵I]SIB was changed to 40 minutes, thus obtaining the intended (IB-PEG₃)40-Ex4 (a peptide derivative represented by formula (27), (SEQ ID NO. 27)) (radiochemical yield: 32.4 %, radiochemical purity: > 99%). Further, the time involved in the labeling was 4.3 hours.

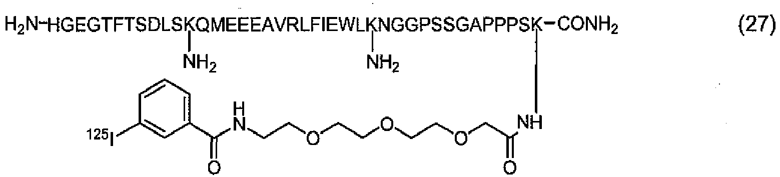

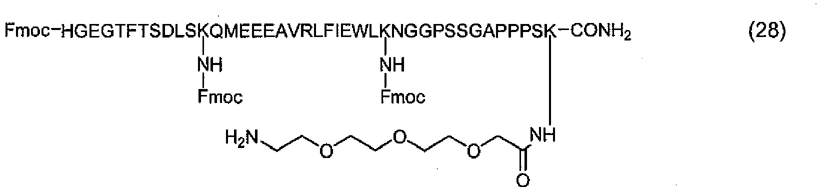

### (Reference Production Example 5)

Labeling was performed in the same manner as in Production Example 5 except that a labeling precursor represented by formula (29) (510 µg) was used in place of the peptide derivative represented by formula (28) and the reaction time with [¹²⁵I]SIB was changed to 61 minutes, thus obtaining an intended peptide derivative represented by formula (30) (SEQ ID NO. 30) (radiochemical yield: 28.3 %, radiochemical purity: > 99%). Further, the time involved in the labeling was 4.5 hours.

### Fmoc-HGEGTFTSDLSK(Fmoc)QMEEEAVRLFIEWLK(Fmoc)NGGPSSGAPPPSK-C ONH₂ (29) (SEQ ID NO. 29)

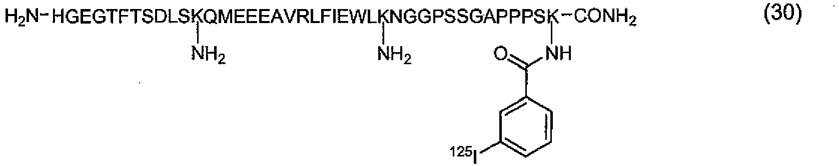

As Production Example 5 and Reference Production Example 5 show, as a result of performing radioactive labeling using, as the labeling precursor, the peptide derivative of formula (28) in which PEG3 was bound to the amino group of the side chain of the lysine, the yield of the radioactively labeled peptide derivative was improved in comparison to using the labeling precursor of formula (29) in which PEG3 was not bound to the amino group of the side chain of the lysine.

### [Binding Assay]

A binding assay was performed using four kinds of polypeptides (formulae (31) to (34); all cold probes) listed in Table 11 below.

### (Procedure for Binding Assay)

The binding assay was performed using the following procedure. First, pancreatic islets isolated from a mouse was recovered in a 50 ml-tube and after they were subjected to centrifugation (2000 rpm, 2 minutes), they were washed once with 20 ml of cold PBS. To this, 15 ml of trypsin-EDTA (which was prepared by adding 12 ml of PBS-containing 0.53mM EDTA (pH 7.4 (NaOH)) to 3 ml of trypsine-EDTA (0.05% / 0.53mM)) was added. This was incubated at 37°C for one minute while shaking, then immediately placed on ice. Subsequently, after it was subjected to pipetting vigorously 20 times with a 10 ml pipette dropper without being foamed, the cold PBS was added so that the final amount would be 30 ml. After centrifugation (3000 rpm, 2 minutes), it was washed twice with 30 ml of cold PBS. The supernatant was removed, whereby a sample of pancreatic islet cells was obtained. The obtained sample of pancreatic islet cells was reserved at -80°C.

The sample of pancreatic islet cells was suspended in a buffer (20mM HEPES (pH 7.4), 1 mM MgCl₂, 1 mg/ml bacitracin, 1 mg/ml BSA) so as to make 100 µL/tube. Then, 880 µL of the buffer and 10 µL of a solution including each polypeptide (final concentration of polypeptide: 0,1 × 10⁻⁶ to 1 × 10⁻¹² M), and 10 µL of a solution including [¹²⁵I] Bolton-Hunter labeled Exendin(9-39) (prepared by adding 90 µL of a buffer to 10 µL of [¹²⁵I]Bolton-Hunter labeled Exendin(9-39) (product code: NEX335, 1.85 MBq/ml = 50 µCi/ml, 22.73 pmol/ml = 76.57 ng/ml, manufactured by Perkin Elmer) were added thereto, and incubated for 60 minutes at room temperature. Here, the final concentration of the [¹²⁵I] Bolton-Hunter labeled Exendin(9-39) was set to 0.05 µCi/tube. Next, after B/F separation by aspirating with use of an aspirator to which a pre-moistened glass fiber filter (Whatman GF/C filter) was attached, the filter was washed three times with 5 ml of ice-cold PBS. The filter was set in the tube, and the radioactivity measurement was carried out with a gamma counter. Table 11 below provides the obtained results (IC₅₀ of each polypeptide).

| (Table 11) | IC₅₀(nM) |
|---|---|
| (¹²⁷IB-PEG₃)12-Ex(9-39) (formula (31), SEQ ID NO. 31) | 3.9 |
| (¹²⁷IB-PEG₃)40-Ex(9-39) (formula (32), SEQ ID NO. 32) | 5.7 |
| (¹²⁷IB-PEG₃)12-Ex4 (formula (33), SEQ ID NO. 33) | 2.6 |
| (¹²⁷IB-PEG₃)40-Ex4 (formula (34), SEQ ID NO. 34) | 2.7 |
| exendin(9-39) (SEQ ID NO. 20) | 1.4 |

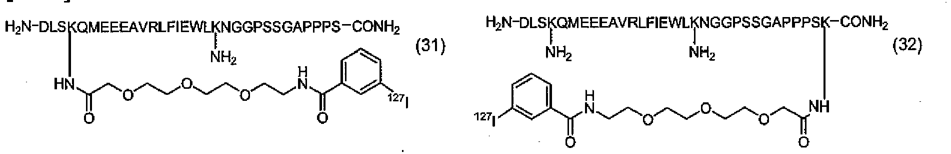

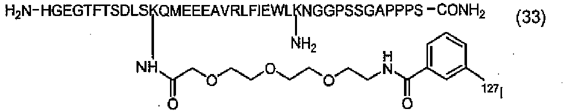

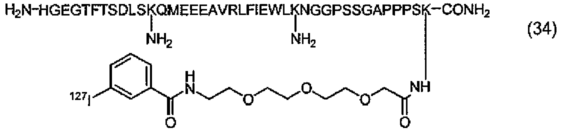

Each of the polypeptides of formulae (31) to (34) inhibited in a concentration-dependent manner the binding between the GLP-1R of pancreatic islets and the [¹²⁵I] Bolton-Hunter labeled Exendin(9-39). In particular, (¹²⁷IB-PEG₃)12-Ex4 and (¹²⁷IB-PEG₃)40-Ex4 exhibited a high affinity for the GLP-1R of pancreatic islets.

### (Example 5)

Biodistribution experiments and two-dimensional imaging analysis were performed using the peptide derivative represented by formula (25)
((IB-PEG₃)12-Ex4).

### [Biodistribution Experiment]

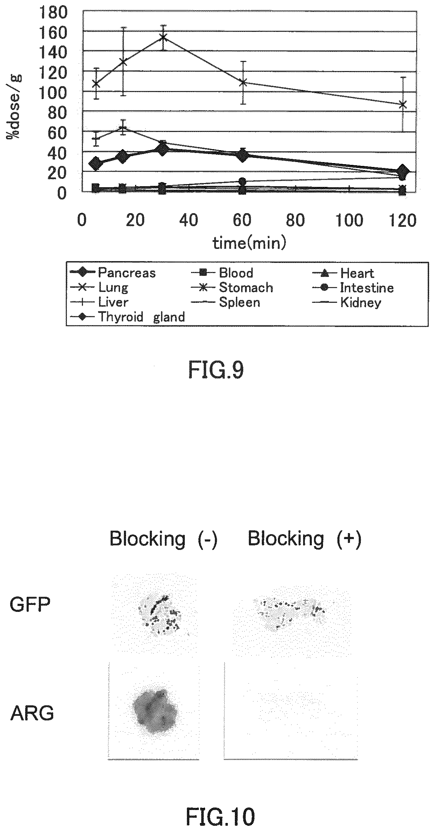

(IB-PEG₃)12-Ex4 (0.77 µCi) was administered to unanesthetized 6-week-old ddY mice (male, weight: 30g) by intravenous injection (through the tail vein). After 5 minutes, 15 minutes, 30 minutes, 60 minutes, and 120 minutes from the administration, organs were dissected out of the mice (n=5). The weight and the radioactivity of each organ were determined, and an accumulation amount (%dose/g) of (IB-PEG₃)12-Ex4 was calculated from the radioactivity per unit weight. The exemplary results are shown in Tables 12 and 13 and FIG. 9. FIG. 9 is a graph showing, by way of example, how the accumulation of (IB-PEG₃)12-Ex4 in each organ varied with time.

| (Table 12) | Time after administration | | | | |
|---|---|---|---|---|---|
| | 5min | 15min | 30min | 60min | 120min |
| Pancreas | 27.66 (5.61) | 34.63 (4.39) | 42.36 (3.77) | 35.94 (3.90) | 20.77 (2.97) |
| Blood | 4.39 (0.76) | 2.05 (0.27) | 1.10 (0.07) | 0.72 (0.09) | 0.37 (0.08) |
| Heart | 2.78 (0.56) | 1.92 (0.56) | 1.54 (0.43) | 1.18 (0.59) | 0.77 (0.11) |
| Lung | 107.21 (15.40) | 129.24 (33.76) | 153.27 (12.31) | 108.74 (21.29) | 87.27 (27.49) |
| Stomach | 3.47 (1.08) | 4.30 (0.90) | 5.04 (1.30) | 5.93 (3.28) | 3.49 (1.06) |
| Intestine | 3.07 (0.27) | 3.91 (0.48) | 5.85 (1.17) | 10.29 (2.45) | 14.56 (2.03) |
| Liver | 2.07 (0.29) | 3.10 (0.19) | 4.50 (0.58) | 4.63 (0.66) | 3.05 (0.68) |
| Spleen | 1.44 (0.21) | 1.03 (0.20) | 0.60 (0.12) | 0.51 (0.11) | 0.39 (0.21) |
| Kidney | 52.05 (7.05) | 63.78 (7.49) | 48.55 (2.44) | 37.34 (6.15) | 15.73 (2.11) |
| Thyroid gland | 2.66 (0.77) | 2.67 (0.74) | 4.11 (3.36) | 2.85 (1.07) | 3.15 (1.06) |

| | | | | | |
|---|---|---|---|---|---|
| Each numerical value indicates an average (SD) of 5 mice. | | | | | |

| (Table 13) | Time after administration | | | | |
|---|---|---|---|---|---|
| | 5min | 15min | 30min | 60min | 120mm |
| Pancreas/Liver Ratio | 13.64 (3.98) | 11.25 (1.93) | 9.54 (1.35) | 7.81 (0.72) | 6.95 (1.09) |
| Pancreas/Kidney Ratio | 0.54 (0.15) | 0.55 (0.12) | 0.88 (0.11) | 0.97 (0.11) | 1.35 (0.33) |
| Pancreas/Blood Ratio | 6.48 (1.76) | 17.27 (4.03) | 38.92 (6.07) | 50.07 (3.51) | 56.86 (7.06) |

As shown in Table 12 and FIG. 9, the accumulation of (IB-PEG₃)12-Ex4 in the pancreas reached a level exceeding 25 %dose/g at an early stage after administration, and the accumulation was maintained at a high level thereafter. Further, no great change was seen in the accumulation of (IB-PEG₃) 12-Ex4 in the thyroid gland. This suggests that (IB-PEG₃)12-Ex4 was not subjected to deiodization metabolism *in vivo.*

As shown in Table 13, the ratio of pancreas/liver for (IB-PEG₃)12-Ex4 was greater than 10 at an early stage after administration. Further, the ratio of pancreas/blood for (IB-PEG₃)12-Ex4 was greater than 5 at an early stage after administration, indicating a satisfactory blood clearance. In this way, the results suggest that clear images of pancreatic β-cells, preferably GLP-1R of pancreatic β-cells can be obtained when performing imaging using (IB-PEG₃)12-Ex4, which accumulates in the pancreas in a large amount while showing a high pancreas/liver ratio and has excellent blood clearance.

### [Two-Dimensional Imaging Analysis]

(IB-PEG₃)12-Ex4 (5.6 µCi/100 µl) was administered to an unanesthetized MIP-GFP mouse (male, weight: 20g) by intravenous injection, and after 30 minutes from the administration, the pancreas was dissected out of the mouse (n=1). Sections were cut out of the dissected pancreas, and each section was placed on a slide glass, covered with a cover glass. The fluorescence and radioactivity (autoradiography) of each section were determined using an image analyzer (trade name: Typhoon 9410, manufactured by GE Health Care Inc.) (exposure time: 24 hours). The exemplary results are shown in FIG. 10 under Blocking (-).

Further, non-labeled exendin(9-39) (cold probe, SEQ ID NO. 20) was first administered to an unanesthetized MIP-GFP mouse (male, weight: 20g) by intravenous injection (50 µg/100 µl). After 30 minutes from the preliminary administration, (IB-PEG₃)12-Ex4 (5.6 µCi/100 µl) was administered to the mouse by intravenous injection. Other than these, the fluorescence and radioactivity were determined in the same manner as in the above. The exemplary results are shown in FIG. 10 under Blocking (+).

FIG. 10 illustrates the exemplary results of the imaging analysis of the pancreas sections of the MIP-GFP mice to which (IB-PEG₃)12-Ex4 was administered. The images in the upper row show the fluorescence signal and the images on the lower row show the radioactivity signal. As shown in FIG. 10, the localization of the radioactivity signal was substantially consistent with that of the GFP signal. From this, it was confirmed that (IB-PEG₃)12-Ex4 accumulated specifically in the pancreatic β-cells.

### (Example 6)

With use of a peptide derivative represented by the following formula (35) (SEQ ID NO. 35), three-dimensional SPECT imaging was performed.

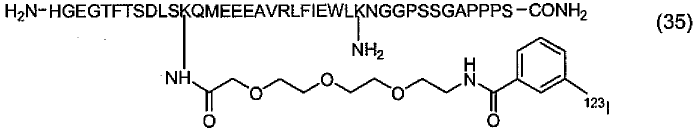

### [Preparation of Peptide Derivative]

The peptide derivative represented by formula (35) was prepared using the same procedure as the one used in Production Example 4 except that [¹²³I]SIB was used in place of [¹²⁵I]SIB.

### [Three-Dimensional Imaging]

The peptide derivative represented by formula (35) (153 µCi (5.66 MBq)/250 µl) was administered to 6-week-old ddY mice (male, weight: about 30g) by intravenous injection, and then after 20 minutes from the administration of the peptide derivative the mice were subjected to inhalation anesthesia with enflurane. After 30 minutes from the administration of the peptide derivative, SPECT imaging was performed under the following imaging conditions with use of a gamma camera (product name: SPECT 2000H-40, manufactured by Hitachi Medical Corporation). Images obtained were reconfigured under the following reconfiguration condition.

### Imaging Conditions

| | |
|---|---|
| Collimator: | LEPH collimator |
| Collecting range: | 360° |
| Step angle: | 11.25° |
| Collecting time: | 60 sec per direction |
| | 1 × 32 frames per 60 sec (total: 32 min) |

### Reconfiguration Condition

| | |
|---|---|
| Preprocessing filter: | Butterworth filter (order: 10, cutoff frequency: 0.10) |

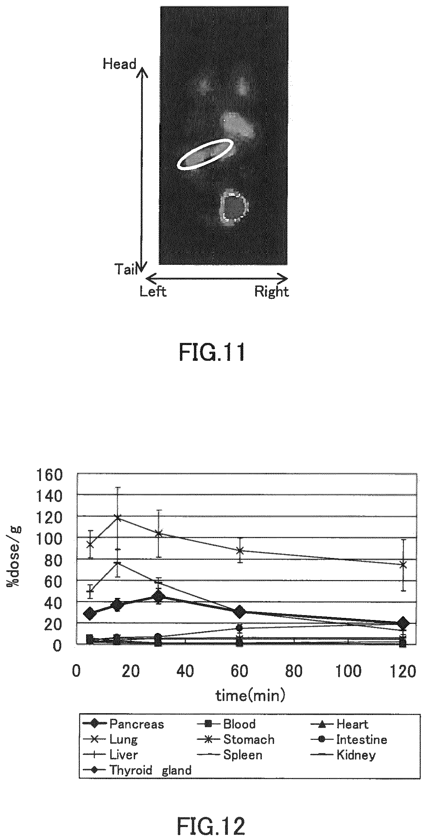

FIG. 11 shows an exemplary image obtained. The image shown in FIG. 11 is a coronal view taken 30 minutes after the administration of the peptide derivative. The portion circled with a white line indicates the position of the pancreas. As shown in FIG. 11, the position of the pancreas was confirmed non-invasively in mice as a result of the SPECT imaging using the peptide derivative represented by formula (35). In this way, the position of the pancreas was confirmed non-invasively in a mouse that has a pancreas of a smaller size than that of a human and in which the organs are present more densely than in a human. This suggests that in a human that has a pancreas of a greater size than that of a mouse and in which the organs are present not as densely as in a mouse, the position of the pancreas and the size of the pancreas can be determined more clearly, and moreover, the amount of the peptide derivative binding to GLP-1R of pancreatic β-cells can be determined.

### (Example 7)

Biodistribution experiments and two-dimensional imaging analysis were performed using the peptide derivative represented by formula (27)
((IB-PEG₃)40-Ex4).

### [Biodistribution Experiment]

(IB-PEG₃)40-Ex4 (0.74 µCi) was administered to unanesthetized 6-week-old ddY mice (male, weight: 30g) by intravenous injection (through the tail vein). After 5 minutes, 15 minutes, 30 minutes, 60 minutes, and 120 minutes from the administration, organs were dissected out of the mice (n=5). The weight and the radioactivity of each organ were determined, and an accumulation amount (%dose/g) of (IB-PEG₃)40-Ex4 was calculated from the radioactivity per unit weight. The exemplary results are shown in Tables 14 and 15 below and FIG. 12. FIG. 12 is a graph showing, by way of example, how the accumulation of (IB-PEG₃)40-Ex4 in each organ varied with time.

| (Table 14) | Time after administration | | | | |
|---|---|---|---|---|---|
| | 5min | 15min | 30min | 60min | 120min |
| Pancreas | 28.70 (4.48) | 36.93 (5.97) | 45.09 (7.01) | 30.76 (2.63) | 19.65 (2.21) |
| Blood | 5.71 (0.68) | 2.85 (0.25) | 1.32 (0.17) | 0.75 (0.08) | 0.35 (0.07) |
| Heart | 3.10 (0.41) | 2.41 (0.17) | 1.16 (0.26) | 1.14 (0.08) | 0.78 (0.26) |
| Lung | 93.55 (12.68) | 118.10 (29.03) | 103.68 (22.13) | 88.00 (11.65) | 74.43 (23.81) |
| Stomach | 3.61 (0.45) | 5.70 (0.51) | 4.83 (1.25) | 4.73 (1.68) | 4.67 (1.90) |
| Intestine | 4.02 (0.46) | 6.06 (0.92) | 6.89 (1.07) | 14.79 (3.91) | 19.17 (3.58) |
| Liver | 2.18 (0.26) | 3.68 (0.45) | 5.43 (0.78) | 5.96 (0.81) | 6.33 (7.23) |
| Spleen | 2.07 (0.21) | 1.23 (0.22) | 0.80 (0.23) | 0.46 (0.06) | 0.39 (0.18) |
| Kidney | 48.95 (6.16) | 75.81 (12.71) | 57.29 (5.02) | 30.78 (4.24) | 12.92 (3.81) |
| Thyroid gland | 2.45 (0.64) | 2.63 (2.01) | 1.25 (0.60) | 1.43 (1.02) | 2.69 (1.55) |

| | | | | | |
|---|---|---|---|---|---|
| Each numerical value indicates an average (SD) of 5 mice. | | | | | |

| (Table 15) | Time after administration | | | | |
|---|---|---|---|---|---|
| | 5min | 15min | 30min | 60min | 120min |
| Pancreas/Liver Ratio | 13.24 (2.08) | 10.25 (2.73) | 8.42 (1.54) | 5.25 (0.94) | 5.20 (2.39) |
| Pancreas/Kidney Ratio | 0.60 (0.14) | 0.49 (0.09) | 0.79 (0.08) | 1.01 (0.06) | 1.59 (0.34) |
| Pancreas/Blood Ratio | 5.08 (0.90) | 12.99 (2.00) | 35.04 (9.20) | 41.31 (6.23) | 56.74 (11.08) |

As shown in Table 14 and FIG. 12, the accumulation of (IB-PEG₃)40-Ex4 in the pancreas reached a level exceeding 25 %dose/g at an early stage after administration, and the accumulation was maintained at a high level thereafter. Further, no great change was seen in the accumulation of (IB-PEG₃)40-Ex4 in the thyroid gland. This suggests that (IB-PEG₃)40-Ex4 was not subjected to deiodization metabolism *in vivo.*

As shown in Table 15, the ratio of pancreas/liver for (IB-PEG₃)40-Ex4 was greater than 10 at an early stage after administration. Further, the ratio of pancreas/blood for (IB-PEG₃)40-Ex4 was greater than 5 at an early stage after administration, indicating a satisfactory blood clearance. In this way, the results suggest that clear images of pancreatic β-cells, preferably GLP-1R of pancreatic β-cells can be obtained when performing imaging using (IB-PEG₃)40-Ex4, which accumulates in the pancreas in a large amount while showing a high pancreas/liver ratio and has excellent blood clearance.

### [Two-Dimensional Imaging Analysis]

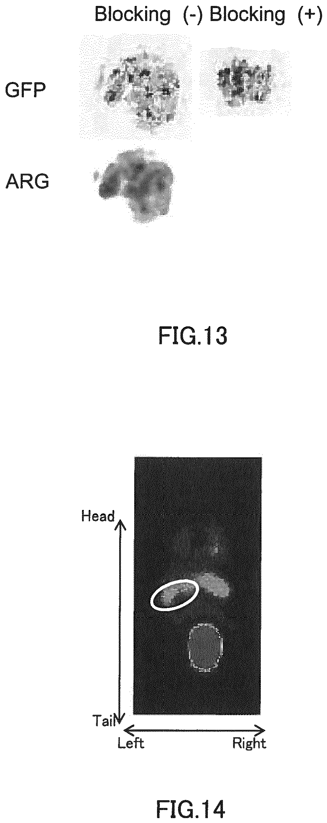

(IB-PEG₃)40-Ex4 (5.6 µCi/100 µl) was administered to an unanesthetized MIP-GFP mouse (male, weight: 20g) by intravenous injection, and after 30 minutes from the administration, the pancreas was dissected out of the mouse (n=1). Sections were cut out of the dissected pancreas, and each section was placed on a slide glass, covered with a cover glass. The fluorescence and radioactivity (autoradiography) of each section were determined using an image analyzer (trade name: Typhoon 9410, manufactured by GE Health Care Inc.) (exposure time: 48 hours). The exemplary results are shown in FIG. 13 under Blocking (-).

Further, non-labeled exendin(9-39) (cold probe, SEQ ID NO. 20) was first administered to an unanesthetized MIP-GFP mouse (male, weight: 20g) by intravenous injection (50 µg/100 µl). After 30 minutes from the preliminary administration, (IB-PEG₃)40-Ex4 (5.6 µCi/100 µl) was administered to the mouse by intravenous injection. Other than these, the fluorescence and radioactivity were determined in the same manner as above. The exemplary results are shown in FIG. 13 under Blocking (+).

FIG. 13 illustrates the exemplary results of the imaging analysis of the pancreas sections of the MIP-GFP mice to which (IB-PEG₃)40-Ex4 was administered. The images in the upper row show the fluorescence signal and the images on the lower row show the radioactivity signal. As the images under Blocking (-) in FIG. 13 show, the localization of the radioactivity signal was substantially consistent with that of the GFP signal. From this, it was confirmed that (IB-PEG₃)40-Ex4 accumulated specifically in the pancreatic β-cells. Further, as the images under Blocking (+) in FIG. 13 show, the radioactivity signal of (IB-PEG₃)40-Ex4 was hardly detected because the receptors were blocked. This suggests that (IB-PEG₃)40-Ex4 accumulated specifically in GLP-1R of the pancreatic β-cells.

### (Example 8)

With use of a peptide derivative represented by the following formula (36) (SEQ ID NO. 36), three-dimensional SPECT imaging was performed.

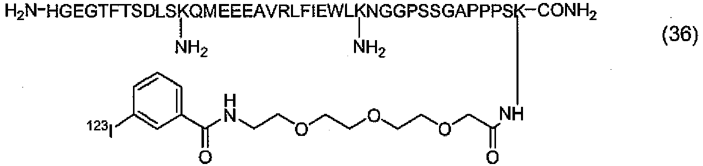

### [Preparation of Peptide Derivative]

The peptide derivative represented by formula (36) was prepared in the same manner as in Production Example 5 except that [¹²³I]SIB was used in place of [¹²⁵I]SIB.

### [Three-Dimensional Imaging]

The peptide derivative represented by formula (36) (154 µCi (5.7 MBq)/100 µl) was administered to 6-week-old ddY mice (male, weight: about 30g) by intravenous injection, and then after 20 minutes from the administration of the peptide derivative the mice were subjected to inhalation anesthesia with enflurane. After 30 minutes from the administration of the peptide derivative, the SPECT imaging was performed under the following imaging conditions with use of a gamma camera (product name: SPECT 2000H-40, manufactured by Hitachi Medical Corporation). Images obtained were reconfigured under the following reconfiguration condition.

### Imaging Conditions

| | |
|---|---|
| Collimator: | LEPH collimator |
| Collecting range: | 360° |
| Step angle: | 11.25° |
| Collecting time: | 60 sec per direction |
| | 1 × 32 frames per 60 sec (total: 32 min) |

### Reconfiguration Condition

| | |
|---|---|
| Preprocessing filter: | Butterworth filter (order: 10, cutoff frequency: 0.10) |

FIG. 14 shows an exemplary image obtained. The image shown in FIG. 14 is a coronal view taken 30 minutes after the administration of the peptide derivative. The portion circled with a white line indicates the position of the pancreas. As shown in FIG. 14, the position of the pancreas was confirmed non-invasively in mice as a result of the SPECT imaging using the peptide derivative represented by formula (36).

### (Example 9)

Biodistribution experiments and two-dimensional imaging analysis were performed using the peptide derivative represented by formula (24)
(FB-PEG₃)12-Ex4.

### [Biodistribution Experiment]

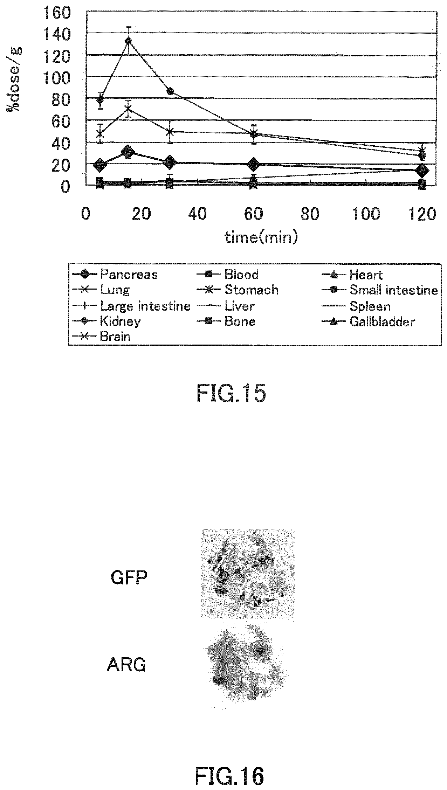

(FB-PEG₃)12-Ex4 (5 µCi) was administered to unanesthetized 6-week-old ddY mice (male, weight: 30g) by intravenous injection (through the tail vein). After 5 minutes, 15 minutes, 30 minutes, 60 minutes, and 120 minutes from the administration, organs were dissected out of the mice (n=5). The weight and the radioactivity of each organ were determined, and an accumulation amount (%dose/g) of (FB-PEG₃)12-Ex4 was calculated from the radioactivity per unit weight. The exemplary results are shown in Tables 16 and 17 below and FIG. 15. FIG. 15 is a graph showing, by way of example, how the accumulation of (FB-PEG₃)40-Ex4 in each organ varied with time.

| (Table 16) | Time after administration | | | | |
|---|---|---|---|---|---|
| | 5min | 15min | 30min | 60min | 120min |
| Pancreas | 18.70 (1.84) | 30.43 (5.56) | 21.31 (2.75) | 19.26 (3.06) | 14.06 (0.48) |
| Blood | 3.74 (0.48) | 1.81 (0.15) | 0.70 (0.04) | 0.26 (0.02) | 0.15 (0.01) |
| Heart | 2.46 (0.24) | 1.75 (0.23) | 0.82 (0.23) | 0.48 (0.09) | 0.29 (0.08) |
| Lung | 47.25 (8.82) | 70.49 (7.90) | 49.08 (10.75) | 47.88 (8.10) | 31.83 (7.09) |
| Stomach | 2.21 (0.57) | 2.95 (1.05) | 3.16 (0.68) | 2.61 (1.33) | 2.09 (1.33) |
| Small intestine | 3.00 (0.62) | 3.47 (0.35) | 3.12 (0.39) | 2.49 (0.43) | 3.29 (0.72) |
| Large intestine | 1.13 (0.14) | 1.16 (0.21) | 0.67 (0.07) | 0.49 (0.07) | 0.50 (0.07) |
| Liver | 1.88 (0.24) | 2.52 (0.12) | 4.73 (5.28) | 1.57 (0.31) | 1.17 (0.08) |
| Spleen | 2.00 (0.44) | 1.54 (0.59) | 0.64 (0.11) | 0.45 (0.23) | 0.19 (0.06) |
| Kidney | 78.02 (7.32) | 132.66 (12.35) | 86.15 (2.29) | 46.63 (8.42) | 27.73 (3.90) |
| Bone | 1.17 (0.23) | 0.87 (0.24) | 0.55 (0.05) | 0.43 (0.31) | 0.18 (0.10) |
| Gall-bladder | 1.22 (0.81) | 2.81 (2.43) | 2.98 (1.05) | 6.91 (3.32) | 14.53 (3.11) |
| Brain | 0.16 (0.03) | 0.10 (0.03) | 0.04 (0.01) | 0.03 (0.03) | 0.07 (0.10) |

| | | | | | |
|---|---|---|---|---|---|
| Each numerical value indicates an average (SD) of 5 mice. | | | | | |

| (Table 17) | Time after administration | | | | |
|---|---|---|---|---|---|
| | 5min | 15min | 30mm | 60min | 120min |
| Pancreas/Liver Ratio | 10.14 (1.98) | 12.10 (2.45) | 7.85 (3.94) | 12.52 (2.58) | 12.08 (0.66) |
| Pancreas/Kidney Ratio | 0.24 (0.04) | 0.23 (0.05) | 0.25 (0.03) | 0.42 (0.06) | 0.52 (0.09) |
| Pancreas/Blood Ratio | 5.08 (0.94) | 17.04 (4.47) | 30.73 (4.91) | 73.47 (7.06) | 92.83 (5.34) |

As shown in Table 16 and FIG. 15, the accumulation of (FBPEG₃)12-Ex4 in the pancreas reached a level exceeding 15 %dose/g at an early stage after administration, and the accumulation was maintained at a high level thereafter. Further, the radioactive accumulation of (FB-PEG₃) 12-Ex4 in bone was small, suggesting that (FB-PEG₃)12-Ex4 was not subjected to deiodization metabolism *in vivo.*

As shown in Table 17, the ratio of pancreas/liver for (FB-PEG₃)12-Ex4 was greater than 10 at an early stage after administration. Further, the ratio of pancreas/blood for (FB-PEG₃)12-Ex4 was greater than 5 at an early stage after administration, indicating a satisfactory blood clearance. In this way, the results suggest that clear images of pancreatic β-cells, preferably GLP-1R of pancreatic β-cells can be obtained when performing imaging using (FB-PEG₃)12-Ex4, which accumulates in the pancreas in a large amount while showing a high pancreas/liver ratio and has excellent blood clearance.

### [Two-Dimensional Imaging Analysis]

(FB-PEG₃)12-Ex4 (292 µCi/220 µl) was administered to an unanesthetized MIP-GFP mouse (male, weight: 20g) by intravenous injection, and after 15 minutes from the administration, the pancreas was dissected out of the mouse (n=1). Sections were cut out of the dissected pancreas, and each section was placed on a slide glass, covered with a cover glass. The fluorescence and radioactivity (autoradiography) of each section were determined using an image analyzer (trade name: Typhoon 9410, manufactured by GE Health Care Inc.) (exposure time: 24 hours). The exemplary results are shown in FIG. 16.

FIG. 16 illustrates the exemplary results of the imaging analysis of the pancreas sections of the MIP-GFP mice to which (FB-PEG₃)12-Ex4 was administered. The image in the upper row shows the fluorescence signal and the image on the lower row shows the radioactivity signal. As shown in FIG. 16, the localization of the radioactivity signal was substantially consistent with that of the GFP signal. From this, it was confirmed that (FB-PEG₃)12-Ex4 accumulated specifically in the pancreatic β-cells.

### (Production Example 6)

### Synthesis of Peptide Derivative represented by Formula (37): (IB-ePEG₁₂)12-Ex4

A peptide derivative represented by the following formula (38) (SEQ ID NO. 38) was prepared in the same manner as in Production Example 3 except that Fmoc-Lys (Boc-ePEG12) was used in place of Fmoc-Lys (Boc-PEG3). Next, labeling was performed in the same manner as in (4) of Production Example 1 except that the obtained peptide derivative represented by formula (38) (540 µg) was used in place of the peptide derivative represented by formula (10), thus obtaining the intended (IB-ePEG₁₂)12-Ex4 (peptide derivative represented by the following formula (37) (SEQ ID NO. 37) (radiochemical yield: 41.8 %, radiochemical purity: 95.7%). Further, the time involved in the labeling was 2.75 hours. Note that the time involved in the labeling includes the reaction time with the labeling compound, the HPLC purification time, the deprotection reaction time after the reaction with the labeling compound, the LC purification time and the concentration time.

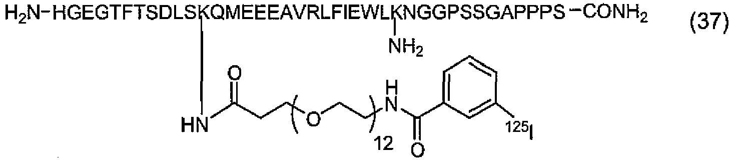

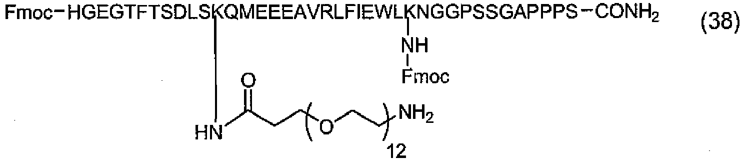

### (Example 10)

Biodistribution experiments were performed using the peptide derivative represented by formula (37) (dB-ePEG₁₂)12-Ex4).

### [Biodistribution Experiment]

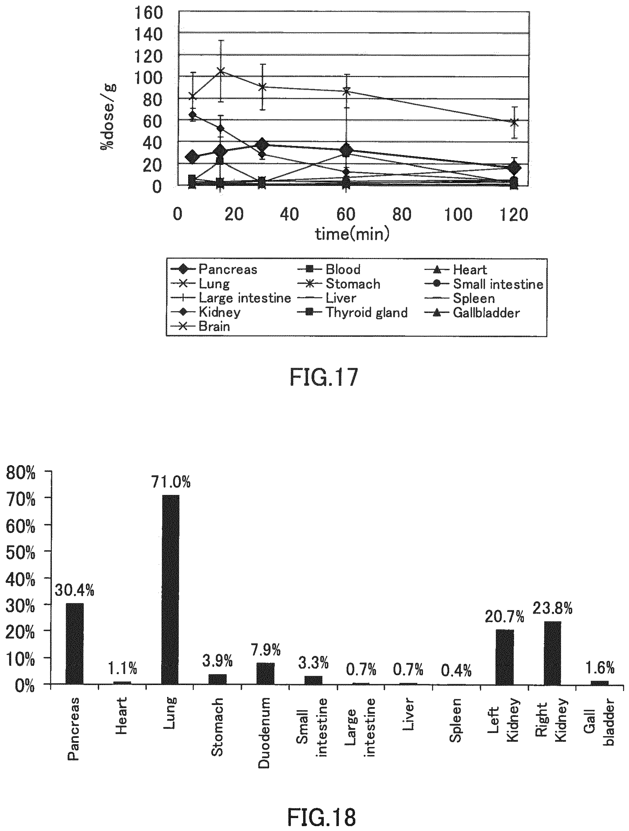

(IB-ePEG₁₂)12-Ex4 (0.21 µCi) was administered to unanesthetized 6-week-old ddY mice (male, weight: 30g) by intravenous injection (through the tail vein). After 5 minutes, 15 minutes, 30 minutes, 60 minutes, and 120 minutes from the administration, organs were dissected out of the mice (n=5). The weight and the radioactivity of each organ were determined, and an accumulation amount (%dose/g) of (IB-ePEG₁₂)12-Ex4 was calculated from the radioactivity per unit weight. The exemplary results are shown in Tables 18 and 19 and FIG. 17. FIG. 17 is a graph showing, by way of example, how the accumulation of (EB-ePEG₁₂)12-Ex4 in each organ varied with time.

| (Table 18) | Time after administration | | | | |
|---|---|---|---|---|---|
| | 5min | 15min | 30min | 60min | 120min |
| Pancreas | 25.87 (2.33) | 30.87 (1.82) | 36.95 (3.35) | 32.07 (5.74) | 16.47 (1.18) |
| Blood | 5.72 (0.67) | 2.36 (0.21) | 1,25 (0.22) | 0.70 (0.05) | 0.37 (0.04) |
| Heart | 3.07 (0.47) | 1.83 (0.15) | 1.48 (0.32) | 1.10 (0.13) | 0.73 (0.15) |
| Lung | 81.86 (21.39) | 104.67 (28.35) | 90.49 (21.11) | 86.48 (15.69) | 57.99 (14.71) |
| Stomach | 3.44 (1.76) | 3.23 (0.95) | 4.33 (1.68) | 2.92 (0.64) | 3.09 (1.42) |
| Small intestine | 3.13 (0.44) | 3.58 (0.57) | 3.77 (0.27) | 4.17 (0.40) | 5.56 (1.71) |
| Large intestine | 1.13 (0.25) | 0.95 (0.18) | 0.89 (0.09) | 1.77 (1.30) | 4.51 (1.25) |
| Liver | 1.96 (0.20) | 1.53 (0.14) | 1.37 (0.17) | 0.91 (0.12) | 1.25 (0.82) |
| Spleen | 1.44 (0.19) | 0.96 (0.47) | 0.61 (0.20) | 0.25 (0.08) | 0.24 (0.02) |
| Kidney | 64.47 (6.11) | 52.07 (7.89) | 28.22 (4.76) | 12.45 (3.84) | 4.47 (1.09) |
| Thyroid gland | 3.77 (3.06) | 21.86 (42.17) | 2.86 (3.24) | 28.74 (61.14) | 2.88 (2.72) |
| Gall-bladder | 0.55 (0.47) | 1.23 (0.78) | 3.96 (3.25) | 6.96 (1.24) | 16.73 (8.83) |
| Brain | 0.15 (0.03) | 0.08 (0.02) | 0.04 (0.02) | 0.03 (0.01) | 0.03 (0.02) |

| | | | | | |
|---|---|---|---|---|---|
| Each numerical value indicates an average (SD) of 5 mice. | | | | | |

| (Table 19) | Time after administration | | | | |
|---|---|---|---|---|---|
| | 5min | 15min | 30min | 60min | 120min |
| Pancreas/Liver Ratio | 13.32 (1.81) | 20.27 (2.30) | 27.39 (5.03) | 35.74 (7.00) | 17.98 (9.31) |
| Pancreas/Kidney Ratio | 0.40 (0.02) | 0.60 (0.08) | 1.35 (0.29) | 2.77 (0.94) | 3.80 (0.61) |
| Pancreas/Blood Ratio | 4.56 (0.58) | 13.18 (1.58) | 30.36 (6.54) | 45.68 (5.91) | 44.77 (5.91) |

As shown in Table 18 and FIG. 17, the accumulation of (IB-ePEG₁₂)12-Ex4 in the pancreas reached a level exceeding 25 %dose/g at an early stage after administration, and the accumulation was maintained at a high level thereafter. Further, no great change was seen in the accumulation of (IB-ePEG₁₂)12-Ex4 in the thyroid gland. This suggests that (IB-ePEG₁₂)12-Ex4 was not subjected to deiodization metabolism *in vivo.*

As shown in Table 19, the ratio of pancreas/liver for (IB-ePEG₁₂)12-Ex4 was greater than 10 at an early stage after administration and the ratio was maintained at a high level thereafter. Further, the ratio of pancreas/blood for (IB-ePEG₁₂)12-Ex4 was greater than 5 at an early stage after administration and the ratio was maintained at a high level thereafter, indicating a satisfactory blood clearance. In this way, the results suggest that clear images of pancreatic β-cells, preferably GLP-1R of pancreatic β-cells can be obtained when performing imaging using (IB-ePEG₁₂)12-Ex4, which accumulates in the pancreas in a large amount while showing a high pancreas/liver ratio and has excellent blood clearance.

### (Example 11)

With use of a peptide derivative represented by the following formula (39) (SEQ ID NO. 39), three-dimensional SPECT imaging was performed.

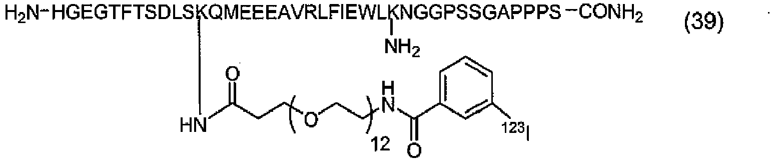

### [Preparation of Peptide Derivative]

The peptide derivative represented by formula (39) was prepared using the same procedure as in Production Example 6 except that [¹²³I]SIB was used in place of [¹²⁵I]SIB.

### [Three-Dimensional Imaging]

SPECT imaging of a mouse was performed in the same manner as in Example 8 except that the peptide derivative represented by formula (39) was used in place of the peptide derivative of formula (36) and the administration amount was changed to 233 µCi ((8.6 MBq)/120 µl). The exemplary results are shown in FIG. 18. FIG. 18 is a graph showing the percentage of administered radiation per gram of each organ of the mouse (percentage of radioactive accumulation per gram of organ (%dose/g)). As shown in FIG. 18, accumulation was hardly seen in the liver and the large intestine, indicating favorable results. Further, the position of the pancreas was confirmed non-invasively from the images obtained (data not shown).

As described above, the present invention is useful in, for example, the medical field, the molecular imaging field, and the field relating to diabetes.

### Sequence Listing Free Text

SEQ ID NO.1: the amino acid sequence of exendin-4
SEQ ID NO.2: an exemplary amino acid sequence of a peptide derivative of the present invention
SEQ ID NO.3: an exemplary amino acid sequence of a peptide derivative of the present invention
SEQ ID NO.4: an exemplary amino acid sequence of a peptide derivative of the present invention
SEQ ID NO. 5: an exemplary amino acid sequence of a peptide derivative of the present invention
SEQ ID NO.6: the amino acid sequence of the peptide derivative produced in Production Example 1
SEQ ID NO.7: the amino acid sequence of the protected peptide resin A produced in Production Example 1
SEQ ID NO.8: the amino acid sequence of the protected peptide resin A1 produced in Production Example 1
SEQ ID NO.9: the amino acid sequence of the protected peptide resin A2 produced in Production Example 1
SEQ ID NO. 10: the amino acid sequence of the labeling precursor produced in Production Example 1
SEQ ID NO. 11: the amino acid sequence of the peptide derivative produced in Reference Production Example 1
SEQ ID NO. 12: the amino acid sequence of the labeling precursor used in Reference Production Example 1
SEQ ID NO. 13: the amino acid sequence of the peptide derivative produced in Production Example 2
SEQ ID NO. 14: the amino acid sequence of the protected peptide resin B produced in Production Example 2
SEQ ID NO. 15: the amino acid sequence of the labeling precursor produced in Production Example 2
SEQ ID NO. 16: the amino acid sequence of the peptide derivative produced in Reference Production Example 2
SEQ ID NO. 17: the amino acid sequence of the labeling precursor used in Reference Production Example 2
SEQ ID NO. 18: the amino acid sequence of the peptide derivative used in Example 2
SEQ ID NO. 19: the amino acid sequence of the peptide derivative used in Example 4
SEQ ID NO. 20: the amino acid sequence of exendin(9-39)
SEQ ID NO. 21: the amino acid sequence of the peptide derivative produced in Production Example 3
SEQ ID NO. 22: the amino acid sequence of the labeling precursor produced in Production Example 3
SEQ ID NO. 23: the amino acid sequence of the labeling precursor produced in Reference Production Example 3
SEQ ID NO. 24: the amino acid sequence of the peptide derivative produced in Reference Production Example 3
SEQ ID NO. 25: the amino acid sequence of the peptide derivative produced in Production Example 4
SEQ ID NO. 26: the amino acid sequence of the peptide derivative produced in Reference Production Example 4
SEQ ID NO. 27: the amino acid sequence of the peptide derivative produced in Production Example 5
SEQ ID NO. 28: the amino acid sequence of the labeling precursor produced in Production Example 5
SEQ ID NO. 29: the amino acid sequence of the labeling precursor produced in Reference Production Example 5
SEQ ID NO. 30: the amino acid sequence of the peptide derivative produced in Reference Production Example 5
SEQ ID NO. 31: the amino acid sequence of a polypeptide used in a binding assay
SEQ ID NO. 32: the amino acid sequence of a polypeptide used in a binding assay
SEQ ID NO. 33: the amino acid sequence of a polypeptide used in a binding assay
SEQ ID NO. 34: the amino acid sequence of a polypeptide used in a binding assay
SEQ ID NO. 35: the amino acid sequence of the peptide derivative used in Example 6
SEQ ID NO. 36: the amino acid sequence of the peptide derivative used in Example 8
SEQ ID NO. 37: the amino acid sequence of the peptide derivative produced in Production Example 6
SEQ ID NO. 38: the amino acid sequence of the labeling precursor produced in Production Example 6
SEQ ID NO. 39: the amino acid sequence of the peptide derivative used in Example 10

## Claims

1. A peptide derivative represented by the following general formula (I) 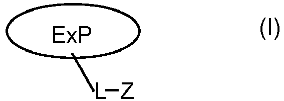 where ExP represents a polypeptide that is represented by an amino acid sequence of the following formula (1) and contains completely or partially the amino acid sequence of exendin-4 (HGEGTFTSDLSKQMEEEAVRLFIEWLKNGGPSSGAPPPS (SEQ ID NO.1)),
Ex4(x-y)-Kₙ (1)
where Ex4(x-y) represents the amino acid sequence of SEQ ID No.1 between positions x and y, x is an integer from 1 to 9, y is an integer from 30 to 39, K represents lysine, and n is 0 or 1,
the α-amino group at the N-terminus of the polypeptide ExP is unmodified or is modified with a modifying group having no electric charge,
the carboxyl group at the C-terminus of the polypeptide ExP is amidated, and
the -L-Z group represents a group represented by the following formula (II) that is bound to an amino acid side chain of a lysine that corresponds to position 12 or 40 of the polypeptide represented by the amino acid sequence of formula (1), 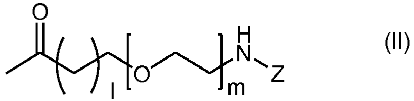 where 1 is 0, 1 or 2 and m is an integer from 3 to 30 when n in formula (1) is 1,1 is an integer from 0 to 8 and m is an integer from 2 to 8 or 4 to 30 when n in formula (1) is 0, and Z represents a labeling group containing a radionuclide or isotope thereof.

2. The peptide derivative according to claim 1, wherein Ex4(x-y)-Kₙ is any of the amino acid sequences of the following formulae (2) to (5):
HGEGTFTSDLSKQMEEEAVRLFIEWLKNGGPSSGAPPPS (2) (SEQ ID NO.2)
HGEGTFTSDLSKQMEEEAVRLFIEWLKNGGPSSGAPPPSK (3) (SEQ ID NO. 3)
DLSKQMEEEAVRLFIEWLKNGGPSSGAPPPS (4) (SEQ ID NO.4)
DLSKQMEEEAVRLFIEWLKNGGPSSGAPPPSK (5) (SEQ ID NO.5).

3. The peptide derivative according to claim 1 or 2, wherein Z is a group represented by the following formula (III), 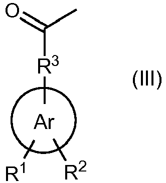 where Ar represents an aromatic hydrocarbon group or aromatic heterocyclic group, preferably an aromatic hydrocarbon group having a carbon number of 6 to 18, R¹ represents a substituent containing a radionuclide or isotope thereof, R² represents a hydrogen atom or one or more substituents different from the substituent represented by R¹, preferably a hydrogen atom, and R³ represents a bond, alkylene group having 1 to 6 carbon atoms or oxyalkylene group having 1 to 6 carbon atoms, preferably a bond, methylene group, or ethylene group.

4. The peptide derivative according to any one of claims 1 to 3, wherein Z represents a labeling group containing a radionuclide.

5. The peptide derivative according to any one of claims 1 to 4, wherein n in formula (1) is 0, and 1 is an integer from 0 to 8, preferably 0 to 5, and m is an integer from 2 to 8 or 4 to 30.

6. A composition comprising the peptide derivative according to any one of claims 1 to 5.

7. Use of the peptide derivative according to any one of claims 1 to 5 for imaging of pancreatic islets, preferably for imaging pancreatic β-cells.

8. A method for imaging pancreatic β-cells, comprising detecting a signal of a radionuclide of the peptide derivative according to claim 4 from an analyte to which the peptide derivative has been administered in advance, wherein preferably the analyte is a human.

9. The method according to claim 8, further comprising reconfiguring the detected signal to convert the signal into an image, and displaying the image.

10. A method for determining the amount of pancreatic islets, the method comprising:
detecting a signal of the peptide derivative according to claim 4 from an analyte to which the peptide derivative has been administered; and
calculating the amount of pancreatic islets from the detected signal of the peptide derivative, wherein preferably the analyte is a human.

11. The method according to claim 10, further comprising presenting the calculated amount of pancreatic islets.

12. The peptide derivative according to claim 4 for use in diagnosis of diabetes, wherein in said diagnosis said peptide derivative is used in imaging of pancreatic islets, wherein preferably said peptide derivative is used in imaging as described in claim 8 or 9.

13. A method for producing a peptide derivative as defined in any one of claims 1 to 5, said method comprising labelling a peptide derivative represented by the following general formula (IV), 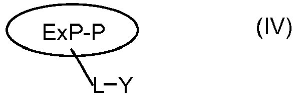 where ExP-P represents a polypeptide that is represented by an amino acid sequence of the following formula (1) and contains completely or partially the amino acid sequence of exendin-4 (HGEGTFTSDLSKQMEEEAVRLFIEWLKNGGPSSGAPPPS (SEQ ID NO.1)),
Ex4(x-y)-Kₙ (1)
where Ex4(x-y) represents the amino acid sequence of SEQ ID No.1 between positions x and y, x is an integer from 1 to 9, y is an integer from 30 to 39, K represents lysine, and n is 0 or 1,
the α-amino group at the N-terminus of the polypeptide ExP-P is bound to a protecting group, modified with a modifying group having no electric charge, or unmodified,
the carboxyl group at the C-terminus of the polypeptide ExP-P is amidated, and
the -L-Y group represents a group represented by the following formula (V) that is bound to an amino acid side chain of a lysine that corresponds to position 12 or 40 of the polypeptide represented by formula (1), 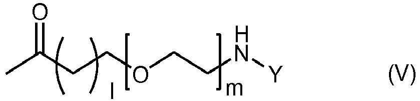 where 1 is 0, 1 or 2 and m is an integer from 3 to 30 when n in formula (1) is 1,1 is an integer from 0 to 8 and m is an integer from 2 to 8 or 4 to 30 when n in formula (1) is 0, and Y represents a hydrogen atom or radioactive labeling introduction group.

14. The method according to claim 13, wherein the radioactive labeling group is a chelating site selected from the group consisting of diethylenetriaminepentaacetic acid (DTPA), 6-hydrazinopyridine-3-carboxylic acid (HYNIC), tetraazacyclododecanetetraacetic acid (DOTA), dithisosemicarbazone (DTS), diaminedithiol (DADT), mercaptoacetylglycylglycylglycine (MAG3), monoamidemonoaminedithiol (MAMA), diamidedithiol (DADS), and propylene diamine dioxime (PnAO), or a group represented by formula (VI), 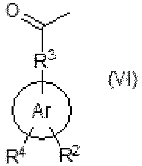 where Ar represents an aromatic hydrocarbon group or aromatic heterocyclic group, R⁴ represents a substituent having a mesylate group, tosylate group, triflate group, ⁸⁰Br, ¹²⁷I, chlorine atom, nitro group, trimethylammonium group, tin atom, alkyltin group, or alkylsilicon group, R² represents a hydrogen atom or one or more substituents different from the substituent represented by R⁴, and R³ represents a bond, alkylene group having 1 to 6 carbon atoms or oxyalkylene group having 1 to 6 carbon atoms.

15. The method according to claim 13 or 14, wherein Ex4(x-y)-Kₙ is any of the amino acid sequences of the following formulae (2) to (5):
HGEGTFTSDLSKQMEEEAVRLFIEWLKNGGPSSGAPPPS (2) (SEQ ID NO.2)
HGEGTFTSDLSKQMEEEAVRLFIEWLKNGGPSSGAPPPSK (3) (SEQ ID NO. 3)
DLSKQMEEEAVRLFIEWLKNGGPSSGAPPPS (4) (SEQ ID NO.4)
DLSKQMEEEAVRLFIEWLKNGGPSSGAPPPSK (5) (SEQ ID NO.5).

16. A kit for producing the peptide derivative according to claim 4, comprising the peptide derivative as defined in any one of claims 13 to 15, and
a substance selected from the group consisting of a labeling compound for labeling the peptide derivative and compounds which are starting materials of the labeling compound, wherein the labeling compound contains a radionuclide.

## Patentansprüche

1. Peptidderivat, das durch die folgende allgemeine Formel (I) dargestellt ist 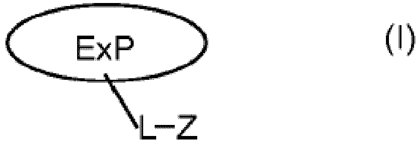 wo ExP ein Polypeptid darstellt, das durch eine Aminosäuresequenz der folgenden Formel (1) dargestellt ist und vollständig oder teilweise die Aminosäuresequenz von Exendin-4 (HGEGTFTSDLSKQMEEEAVRLFIEWLKNGGPSSGAPPPS (SEQ ID Nr. 1)), Ex4(x-y)-Kₙ (1) enthält,
wo Ex4(x-y) die Aminosäuresequenz von SEQ ID Nr. 1 zwischen Positionen x und y darstellt, x eine ganze Zahl von 1 bis 9 ist, y eine ganze Zahl von 30 bis 39 ist, K Lysin darstellt und n 0 oder 1 ist,
die α-Aminogruppe am N-Terminus des Polypeptids ExP unmodifiziert ist oder mit einer Modifizierungsgruppe modifiziert ist, die keine elektrische Ladung aufweist,
die Carboxylgruppe am C-Terminus des Polypeptis ExP amidiert ist, und
die -L-Z-Gruppe eine Gruppe darstellt, die durch die folgende Formel (II) dargestellt ist, die an eine Aminosäure-Seitenkette eines Lysins gebunden ist, die Position 12 oder 40 des Polypeptids entspricht, das durch die Aminosäuresequenz von Formel (1) dargestellt ist, 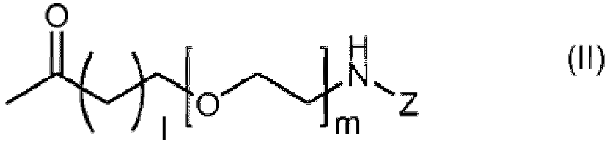 wo I 0, 1 oder 2 ist und m eine ganze Zahl von 3 bis 30 ist, wenn n in Formel (1) 1 ist, I eine ganze Zahl von 0 bis 8 ist und m eine ganze Zahl von 2 bis 8 oder 4 bis 30 ist, wenn n in Formel (1) 0 ist, und Z eine Kennzeichnungsgruppe darstellt, die ein Radionuklid oder Isotop davon enthält.

2. Peptidderivat nach Anspruch 1 wobei Ex4(x-y)-Kₙ eine beliebige der Aminosäuresequenzen der folgenden Formeln (2) bis (5) ist:
HGEGTFTSDLSKQMEEEAVRLFIEWLKNGGPSSGAPPPS (2) (SEQ ID Nr. 2)
HGEGTFTSDLSKQMEEEAVRLFIEWLKNGGPSSGAPPPSK (3) (SEQ ID Nr. 3)
DLSKQMEEEAVRLFIEWLKNGGPSSGAPPPS (4) (SEQ ID Nr. 4)
DLSKQMEEEAVRLFIEWLKNGGPSSGAPPPSK (5) (SEQ ID Nr. 5).

3. Peptidderivat nach Anspruch 1 oder 2, wobei Z eine Gruppe ist, die durch die folgende Formel (III) dargestellt ist, 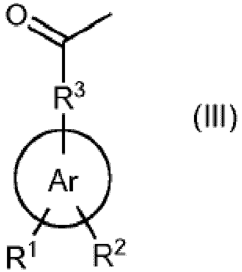 wo Ar eine aromatische Kohlenwasserstoffgruppe oder aromatische heterocyclische Gruppe, vorzugsweise eine aromatische Kohlenwasserstoffgruppe, die eine Kohlenstoffzahl von 6 bis 18 aufweist, darstellt, R¹ einen Substituenten darstellt, der ein Radionuklid oder Isotop davon enthält, R² ein Wasserstoffatom oder einen oder mehrere Substituenten, die sich vom Substituenten unterscheiden, der durch R¹ dargestellt ist, vorzugsweise ein Wasserstoffatom, darstellt, und R³ eine Bindung, Alkylengruppe, die 1 bis 6 Kohlenstoffatome aufweist, oder Oxyalkylengruppe, die 1 bis 6 Kohlenstoffatome aufweist, vorzugsweise eine Bindung, Methylengruppe oder Ethylengruppe, darstellt.

4. Peptidderivat nach einem der Ansprüche 1 bis 3, wobei Z eine Kennzeichnungsgruppe darstellt, die ein Radionuklid enthält.

5. Peptidderivat nach einem der Ansprüche 1 bis 4, wobei n in Formel (1) 0 ist, und I eine ganze Zahl von 0 bis 8, vorzugsweise 0 bis 5, ist, und m eine ganze Zahl von 2 bis 8 oder 4 bis 30 ist.

6. Zusammensetzung, umfassend das Peptidderivat nach einem der Ansprüche 1 bis 5.

7. Verwendung des Peptidderivats nach einem der Ansprüche 1 bis 5 zur Bildgebung von Pankreasinseln, vorzugsweise zur Bildgebung von pankreatischen β-Zellen.

8. Verfahren zur Bildgebung von pankreatischen β-Zellen, umfassend ein Erfassen eines Signals eines Radionuklids des Peptidderivats nach Anspruch 4 von einem Analyten, dem das Peptidderivat vorab verabreicht wurde, wobei vorzugsweise der Analyt ein Mensch ist.

9. Verfahren nach Anspruch 8, weiter umfassend ein Rekonfigurieren des erfassten Signals, um das Signal in ein Bild zu verwandeln, und ein Anzeigen des Bildes.

10. Verfahren zum Bestimmen der Menge an Pankreasinseln, wobei das Verfahren umfasst:
Erfassen eines Signals des Peptidderivats nach Anspruch 4 von einem Analyten, dem das Peptidderivat verabreicht wurde; und
Berechnen der Menge an Pankreasinseln vom erfassten Signal des Peptidderivats, wobei vorzugsweise der Analyt ein Mensch ist.

11. Verfahren nach Anspruch 10, weiter umfassend ein Darlegen der berechneten Menge an Pankreasinseln.

12. Peptidderivat nach Anspruch 4 zur Verwendung in einer Diabetesdiagnose, wobei in der Diagnose das Peptidderivat bei einer Bildgebung von Pankreasinseln verwendet wird, wobei vorzugsweise das Peptidderivat bei der Bildgebung, wie in Anspruch 8 oder 9 beschrieben, verwendet wird.

13. Verfahren zum Herstellen eines Peptidderivats wie in einem der Ansprüche 1 bis 5 definiert, wobei das Verfahren eine Kennzeichnung eines Peptidderivats umfasst, das durch die folgende allgemeine Formel (IV) dargestellt ist, 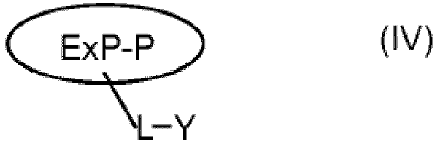 wo ExP-P ein Polypeptid darstellt, das durch eine Aminosäuresequenz der folgenden Formel (1) dargestellt ist und vollständig oder teilweise die Aminosäuresequenz von Exendin-4 (HGEGTFTSDLSKQMEEEAVRLFIEWLKNGGPSSGAPPPS (SEQ ID Nr. 1)), Ex4(x-y)-Kₙ (1) enthält,
wo Ex4(x-y) die Aminosäuresequenz von SEQ ID Nr. 1 zwischen Positionen x und y darstellt, x eine ganze Zahl von 1 bis 9 ist, y eine ganze Zahl von 30 bis 39 ist, K Lysin darstellt und n 0 oder 1 ist,
die α-Aminogruppe am N-Terminus des Polypeptids ExP-P an eine Schutzgruppe gebunden ist, mit einer Modifizierungsgruppe modifiziert ist, die keine elektrische Ladung aufweist, oder unmodifiziert ist,
die Carboxylgruppe am C-Terminus des Polypeptids ExP-P amidiert ist, und
die -L-Y-Gruppe eine Gruppe darstellt, die durch die folgende Formel (V) dargestellt ist, die an eine Aminosäure-Seitenkette eines Lysins gebunden ist, die Position 12 oder 40 des Polypeptids entspricht, das durch Formel (1) dargestellt ist, 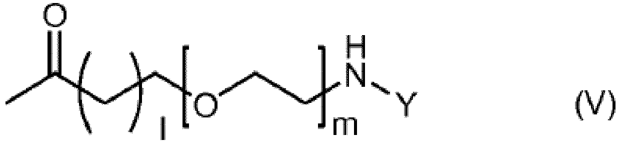 wo I 0, 1 oder 2 ist und m eine ganze Zahl von 3 bis 30 ist, wenn n in Formel (1) 1 ist, I eine ganze Zahl von 0 bis 8 ist und m eine ganze Zahl von 2 bis 8 oder 4 bis 30 ist, wenn n in Formel (1) 0 ist, und Y ein Wasserstoffatom oder eine radioaktive Kennzeichnung-Einführungsgruppe darstellt.

14. Verfahren nach Anspruch 13, wobei die radioaktive Kennzeichnung-Gruppe eine Chelatierungsstelle ist, die ausgewählt ist aus der Gruppe bestehend aus Diethylentriaminpentaessigsäure (DTPA), 6-Hydrazinopyridin-3-carboxylsäure (HYNIC), Tetraazacyclododecantetraessigsäure (DOTA), Dithisosemicarbazon (DTS), Diamindithiol (DADT), Mercaptoacetylglycylglycylglycin (MAG3), Monoamidmonoamindithiol (MAMA), Diamiddithiol (DADS), und Propylendiamindioxim (PnAO), oder eine Gruppe, die durch Formel (VI) dargestellt ist, 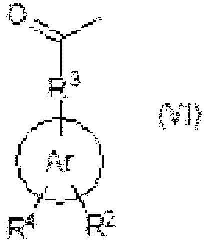 wo Ar eine aromatische Kohlenwasserstoffgruppe oder aromatische heterocyclische Gruppe darstellt, R⁴ einen Substituenten darstellt, der eine Mesylatgruppe, Tosylatgruppe, Triflatgruppe, ⁸⁰BR, ¹²⁷I, Chloratom, Nitrogruppe, Trimethylammoniumgruppe, Zinnatom, Alkylzinngruppe oder Alkylsiliziumgruppe aufweist, R² ein Wasserstoffatom oder einen oder mehrere Substituenten darstellt, die sich vom Substituenten unterscheiden, der durch R⁴ dargestellt ist, und R³ eine Bindung, Alkylengruppe, die 1 bis 6 Kohlenstoffatome aufweist, oder Oxyalkylengruppe, die 1 bis 6 Kohlenstoffatome aufweist, darstellt.

15. Verfahren nach Anspruch 13 oder 14, wobei Ex4(x-y)-Kₙ eine beliebige der Aminosäuresequenzen der folgenden Formeln (2) bis (5) ist:
HGEGTFTSDLSKQMEEEAVRLFIEWLKNGGPSSGAPPPS (2) (SEQ ID Nr. 2)
HGEGTFTSDLSKQMEEEAVRLFIEWLKNGGPSSGAPPPSK (3) (SEQ ID Nr. 3)
DLSKQMEEEAVRLFIEWLKNGGPSSGAPPPS (4) (SEQ ID Nr. 4)
DLSKQMEEEAVRLFIEWLKNGGPSSGAPPPSK (5) (SEQ ID Nr. 5).

16. Satz zum Herstellen eines Peptidderivats nach Anspruch 4, umfassend das Peptidderivat wie in einem der Ansprüche 13 bis 15 definiert, und
eine Substanz, die ausgewählt ist aus der Gruppe bestehend aus einer Kennzeichnungsverbindung zum Kennzeichnen des Peptidderivats und Verbindungen, die Ausgangsmaterialien der Kennzeichnungsverbindung sind, wobei die Kennzeichnungsverbindung ein Radionuklid enthält.

## Revendications

1. Dérivé de peptide représenté par la formule générale (I) suivante 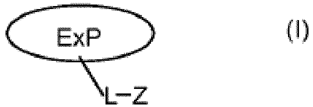 où ExP représente un polypeptide qui est représenté par une séquence d'acides aminés de la formule suivante (1) et qui contient complètement ou partiellement la séquence d'acides aminés d'exendin-4
(HGEGTFTSDLSKQMEEEAVRLFIEWLKNGGPSSGAPPPS (SEQ ID n° 1)), Ex4(x-y)-Kₙ (1)
où Ex4(x-y) représente la séquence d'acides aminés de SEQ ID n°1 entre des positions x et y, x est un entier de 1 à 9, y est un entier de 30 à 39, K représente de la lysine et n est 0 ou 1,
le groupe α-amino au niveau de la terminaison N du polypeptide ExP est non modifié ou est modifié avec un groupe de modification n'ayant aucune charge électrique,
le groupe carboxyle au niveau de la terminaison C du polypeptide ExP est amidé, et
le groupe -L-Z représente un groupe représenté par la formule suivante (II) qui est lié à une chaîne latérale d'acide aminé d'une lysine qui correspond à la position 12 ou 40 du polypeptide représenté par la séquence d'acides aminés de formule (1), 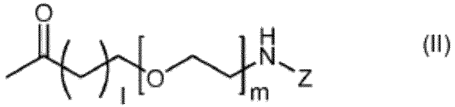 où I est 0, 1 ou 2 et m est un entier de 3 à 30 quand n dans la formule (1) est 1, I est un entier de 0 à 8 et m est un entier de 2 à 8 ou 4 à 30 quand n dans la formule (1) est 0, et Z représente un groupe de marquage contenant un radionucléide ou un isotope de ce dernier.

2. Dérivé de peptide selon la revendication 1, dans lequel Ex4(x-y)-Kₙ est n'importe laquelle des séquences d'acides aminés des formules suivantes (2) à (5) :
HGEGTFTSDLSKQMEEEAVRLFIEWLKNGGPSSGAPPPS (2) (SEQ ID n° 2)
HGEGTFTSDLSKQMEEEAVRLFIEWLKNGGPSSGAPPPSK (3) (SEQ ID n° 3)
DLSKQMEEEAVRLFIEWLKNGGPSSGAPPPS (4) (SEQ ID n° 4)
DLSKQMEEEAVRLFIEWLKNGGPSSGAPPPSK (5) (SEQ ID n° 5).

3. Dérivé de peptide selon la revendication 1 ou 2, dans lequel Z est un groupe représenté par la formule suivante (III), 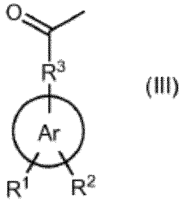 où Ar représente un groupe hydrocarbure aromatique ou un groupe hétérocyclique aromatique, de préférence un groupe hydrocarbure aromatique ayant un nombre de carbones de 6 à 18, R¹ représente un substituant contenant un radionucléide ou un isotope de ce dernier, R² représente un atome d'hydrogène ou un ou plusieurs substituants différents du substituant représenté par R¹, de préférence un atome d'hydrogène, et R³ représente une liaison, un groupe alkylène ayant de 1 à 6 atomes de carbone ou un groupe oxyalkylène ayant de 1 à 6 atomes de carbone, de préférence une liaison, un groupe méthylène, ou un groupe éthylène.

4. Dérivé de peptide selon l'une quelconque des revendications 1 à 3, dans lequel Z représente un groupe de marquage contenant un radionucléide.

5. Dérivé de peptide selon l'une quelconque des revendications 1 à 4, dans lequel n dans la formule (1) est 0 et I est un entier de 0 à 8, de préférence 0 à 5 et m est un entier de 2 à 8 ou 4 à 30.

6. Composition comprenant le dérivé de peptide selon l'une quelconque des revendications 1 à 5.

7. Utilisation du dérivé de peptide selon l'une quelconque des revendications 1 à 5 pour prendre une image d'îlots pancréatiques, de préférence pour prendre une image de cellules β pancréatiques.

8. Procédé pour prendre une image de cellules β pancréatiques, comprenant la détection d'un signal d'un radionucléide du dérivé de peptide selon la revendication 4 à partir d'un analyte auquel le dérivé de peptide a été administré à l'avance, dans lequel de préférence l'analyte est un humain.

9. Procédé selon la revendication 8, comprenant en outre la reconfiguration du signal détecté pour transformer le signal en une image et l'affichage de l'image.

10. Procédé pour déterminer la quantité d'îlots pancréatiques, le procédé comprenant :
la détection d'un signal du dérivé de peptide selon la revendication 4 à partir d'un analyte auquel le dérivé de peptide a été administré ; et
le calcul de la quantité d'îlots pancréatiques à partir du signal détecté du dérivé de peptide, dans lequel de préférence l'analyte est un humain.

11. Procédé selon la revendication 10, comprenant en outre la présentation de la quantité calculée d'îlots pancréatiques.

12. Dérivé de peptide selon la revendication 4 à utiliser dans le diagnostic de diabète, dans lequel, dans ledit diagnostic, ledit dérivé de peptide est utilisé dans l'imagerie d'îlots pancréatiques, dans lequel de préférence ledit dérivé de peptide est utilisé dans l'imagerie comme décrit dans la revendication 8 ou 9.

13. Procédé pour produire un dérivé de peptide selon l'une quelconque des revendications 1 à 5, ledit procédé comprenant le marquage d'un dérivé de peptide représenté par la formule générale (IV) suivante, 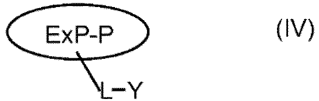 où ExP-P représente un polypeptide qui est représenté par une séquence d'acides aminés de la formule suivante (1) et qui contient complètement ou partiellement la séquence d'acides aminés d'exendin-4
(HGEGTFTSDLSKQMEEEAVRLFIEWLKNGGPSSGAPPPS (SEQ ID n° 1)), Ex4(x-y)-Kₙ (1)
où Ex4(x-y) représente la séquence d'acides aminés de SEQ ID n°1 entre des positions x et y, x est un entier de 1 à 9, y est un entier de 30 à 39, K représente de la lysine et n est 0 ou 1,
le groupe α-amino au niveau de la terminaison N du polypeptide ExP-P est lié à un groupe de protection, modifié avec un groupe de modification n'ayant aucune charge électrique, ou non modifié,
le groupe carboxyle au niveau de la terminaison C du polypeptide ExP-P est amidé, et
le groupe -L-Y représente un groupe représenté par la formule suivante (V) qui est lié à une chaîne latérale d'acide aminé d'une lysine qui correspond à la position 12 ou 40 du polypeptide représenté par la formule (1), 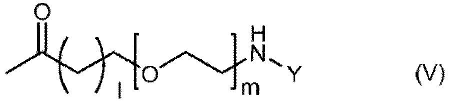 où I est 0, 1 ou 2 et m est un entier de 3 à 30 quand n dans la formule (1) est 1, I est un entier de 0 à 8 et m est un entier de 2 à 8 ou 4 à 30 quand n dans la formule (1) est 0, et Y représente un atome d'hydrogène ou un groupe d'introduction de marquage radioactif.

14. Procédé selon la revendication 13, dans lequel le groupe de marquage radioactif est un site de chélation sélectionné à partir du groupe constitué d'acide diéthylènetriaminopentacétique (DTPA), d'acide 6-hydrazinopyridine-3-carboxylique (HYNIC), d'acide tétraazacyclododécanetétraacétique (DOTA), de dithiosemicarbazone (DTS), de diaminedithiol (DADT), de mercaptoacétylglycylglycylglycine (MAG3), de monoamidemonoaminedithiol (MAMA), de diamidedithiol (DADS) et de dioxime de propylène diamine (PnAO), ou un groupe représenté par la formule (VI), 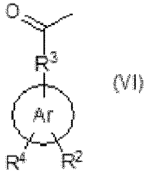 où Ar représente un groupe hydrocarbure aromatique ou un groupe hétérocyclique aromatique, R⁴ représente un substituant ayant un groupe mésylate, un groupe tosylate, un groupe triflate, ⁸⁰Br, ¹²⁷I, un atome de chlore, un groupe nitro, un groupe triméthylammonium, un atome d'étain, un groupe alkylétain, ou un groupe alkylesilicium, R² représente un atome d'hydrogène ou un ou plusieurs substituants différents du substituant représenté par R⁴, et R³ représente une liaison, un groupe alkylène ayant de 1 à 6 atomes de carbone ou un groupe oxyalkylène ayant de 1 à 6 atomes de carbone.

15. Procédé selon la revendication 13 ou 14, dans lequel Ex4(x-y)-Kₙ est n'importe laquelle des séquences d'acides aminés des formules suivantes (2) à (5) :
HGEGTFTSDLSKQMEEEAVRLFIEWLKNGGPSSGAPPPS (2) (SEQ ID n° 2)
HGEGTFTSDLSKQMEEEAVRLFIEWLKNGGPSSGAPPPSK (3) (SEQ ID n° 3)
DLSKQMEEEAVRLFIEWLKNGGPSSGAPPPS (4) (SEQ ID n° 4)
DLSKQMEEEAVRLFIEWLKNGGPSSGAPPPSK (5) (SEQ ID n° 5).

16. Kit pour produire le dérivé de peptide selon la revendication 4, comprenant le dérivé de peptide tel que défini selon l'une quelconque des revendications 13 à 15, et
une substance sélectionnée dans le groupe constitué par un composé de marquage pour marquer le dérivé de peptide et des composés qui sont des matières de départ du composé de marquage, dans lequel le composé de marquage contient un radionucléide.


### SEQUENCE LISTING

<110> Kyoto University
   ARKRAY, Inc.
<120> PEPTIDE DERIVATIVE AND USE OF THE SAME
<130> H3723-04
<160> 39
<170> PatentIn version 3.5
<210> 1
   <211> 39
   <212> PRT
   <213> Heloderma suspectum
<400> 1
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35
<210> 2
   <211> 39
   <212> PRT
   <213> Artificial
<220>
   <223> A peptide derivate
<400> 2
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35
<210> 3
   <211> 40
   <212> PRT
   <213> Artificial Sequence
<220>
   <223> A peptide derivate
<400> 3
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser Lys
           35                  40
<210> 4
   <211> 31
   <212> PRT
   <213> Artificial Sequence
<220>
   <223> A peptide derivate
<400> 4
   Asp Leu Ser Lys Gln Met Glu Glu Glu Ala Val Arg Leu Phe Ile Glu
   1               5                   10                  15
   Trp Leu Lys Asn Gly Gly Pro Ser Ser Gly Ala Pro Pro Pro Ser
               20                  25                  30
<210> 5
   <211> 32
   <212> PRT
   <213> Artificial Sequence
<220>
   <223> A pepitde derivate
<400> 5
   Asp Leu Ser Lys Gln Met Glu Glu Glu Ala Val Arg Leu Phe Ile Glu
   1               5                   10                  15
   Trp Leu Lys Asn Gly Gly Pro Ser Ser Gly Ala Pro Pro Pro Ser Lys
               20                  25                  30
<210> 6
   <211> 31
   <212> PRT
   <213> Artificial Sequence
<220>
   <223> A peptide derivate of formula (6)
<220>
   <221> MOD_RES
   <222> (4)..(4)
   <223> An amino group of a side chain is bound to [1251] IB-PEG3.
<220>
   <221> MOD_RES
   <222> (31)..(31)
   <223> A carboxyl group of a C-terminus is amidated.
<400> 6
   Asp Leu Ser Lys Gln Met Glu Glu Glu Ala Val Arg Leu Phe Ile Glu
   1               5                   10                  15
   Trp Leu Lys Asn Gly Gly Pro Ser Ser Gly Ala Pro Pro Pro Ser
               20                  25                  30
<210> 7
   <211> 31
   <212> PRT
   <213> Artificial Sequence
<220>
   <223> A protected peptide resin A
<220>
   <221> MOD_RES
   <222> (1)..(1)
   <223> An alpha-amino group of an N-terminus is protected by a Fmoc. A functional group of a side chain is protected by a OBu.
<220>
   <221> MOD_RES
   <222> (3)..(3)
   <223> A functional group of a side chain is protected by a OBu.
<220>
   <221> MOD_RES
   <222> (4)..(4)
   <223> An amino group of a side chain is bound to Boc-PEG3.
<220>
   <221> MOD_RES
   <222> (5)..(5)
   <223> A functional group of a side chain is protected by a Trt.
<220>
   <221> MOD_RES
   <222> (7)..(9)
   <223> A functional group of a side chain is protected by a OBu.
<220>
   <221> MOD_RES
   <222> (12)..(12)
   <223> A functional group of a side chain is protected by a Pdf.
<220>
   <221> MOD_RES
   <222> (16)..(16)
   <223> A functional group of a side chain is protected by a OBu.
<220>
   <221> MOD_RES
   <222> (17)..(17)
   <223> A functional group of a side chain is protected by a Boc.
<220>
   <221> MOD_RES
   <222> (19)..(19)
   <223> An amino group of a side chain is protected by a Fmoc
<220>
   <221> MOD_RES
   <222> (20)..(20)
   <223> A functional group of a side chain is protected by a Trt.
<220>
   <221> MOD_RES
   <222> (24)..(25)
   <223> A functional group of a side chain is protected by a OBu.
<220>
   <221> MOD_RES
   <222> (31)..(31)
   <223> A functional group of a side chain is protected by a OBu. A carboxyl group of a C-terminus is bound to Rink Amide MBHA.
<400> 7
   Asp Leu Ser Lys Gln Met Glu Glu Glu Ala Val Arg Leu Phe Ile Glu
   1               5                   10                  15
   Trp Leu Lys Asn Gly Gly Pro Ser Ser Gly Ala Pro Pro Pro Ser
               20                  25                  30
<210> 8
   <211> 27
   <212> PRT
   <213> Artificial Sequence
<220>
   <223> A protected peptide resin A1
<220>
   <221> MOD_RES
   <222> (1)..(1)
   <223> An alpha-amino group of an N-terminus is protected by a Fmoc. A functional group of a side chain is protected by a Trt.
<220>
   <221> MOD_RES
   <222> (3)..(5)
   <223> A functional group of a side chain is protected by a OBu.
<220>
   <221> MOD_RES
   <222> (8)..(8)
   <223> A functional group of a side chain is protected by a Pdf.
<220>
   <221> MOD_RES
   <222> (13)..(13)
   <223> A functional group of a side chain is protected by a OBu.
<220>
   <221> MOD_RES
   <222> (14)..(14)
   <223> A functional group of a side chain is protected by a Boc.
<220>
   <221> MOD_RES
   <222> (16)..(16)
   <223> An amino group of a side chain is protected by a Mmt.
<220>
   <221> MOD_RES
   <222> (17)..(17)
   <223> A functional group of a side chain is protected by a Trt.
<220>
   <221> MOD_RES
   <222> (20)..(21)
   <223> A functional group of a side chain is protected by a OBu.
<220>
   <221> MOD_RES
   <222> (27)..(27)
   <223> A functional group of a side chain is protected by a OBu. A carboxyl group of a C-terminus is bound to Rink Amide MBHA
<400> 8
   Gln Met Glu Glu Glu Ala Val Arg Leu Phe Ile Glu Trp Leu Lys Asn
   1               5                   10                  15
   Gly Gly Pro Ser Ser Gly Ala Pro Pro Pro Ser
               20                  25
<210> 9
   <211> 31
   <212> PRT
   <213> Artificial Sequence
<220>
   <223> A protected peptide resin A2
<220>
   <221> MOD_RES
   <222> (1)..(1)
   <223> An alpha-amino group of an N-terminus is protected by a Fmoc. A functional group of a side chain is protected by a OBu.
<220>
   <221> MOD_RES
   <222> (3)..(3)
   <223> A functional group of a side chain is protected by a OBu.
<220>
   <221> MOD_RES
   <222> (4)..(4)
   <223> An amino group of a side chain is bound to Boc-PEG3.
<220>
   <221> MOD_RES
   <222> (5)..(5)
   <223> A functional group of a side chain is protected by a Trt.
<220>
   <221> MOD_RES
   <222> (7)..(9)
   <223> A functional group of a side chain is protected by a OBu.
<220>
   <221> MOD_RES
   <222> (12)..(12)
   <223> A functional group of a side chain is protected by a Pdf.
<220>
   <221> MOD_RES
   <222> (16)..(16)
   <223> A functional group of a side chain is protected by a OBu.
<220>
   <221> MOD_RES
   <222> (17)..(17)
   <223> A functional group of a side chain is protected by a Boc.
<220>
   <221> MOD_RES
   <222> (19)..(19)
   <223> An amino group of a side chain is protected by a Mmt.
<220>
   <221> MOD_RES
   <222> (20)..(20)
   <223> A functional group of a side chain is protected by a Trt.
<220>
   <221> MOD_RES
   <222> (24)..(25)
   <223> A functional group of a side chain is protected by a OBu.
<220>
   <221> MOD_RES
   <222> (31)..(31)
   <223> A functional group of a side chain is protected by a OBu. A carboxyl group of a C-terminus is bound to Rink Amide MBHA.
<400> 9
   Asp Leu Ser Lys Gln Met Glu Glu Glu Ala Val Arg Leu Phe Ile Glu
   1               5                   10                  15
   Trp Leu Lys Asn Gly Gly Pro Ser Ser Gly Ala Pro Pro Pro Ser
               20                  25                  30
<210> 10
   <211> 31
   <212> PRT
   <213> Artificial Sequence
<220>
   <223> A peptide derivate of formula (10)
<220>
   <221> MOD_RES
   <222> (1)..(1)
   <223> An amino group of an N-terminus is protected by a Fmoc
<220>
   <221> MOD_RES
   <222> (4)..(4)
   <223> An amino group of a side chain is bound to PEG3.
<220>
   <221> MOD_RES
   <222> (19)..(19)
   <223> An amino group of a side chain is bound to Fmoc.
<220>
   <221> MOD_RES
   <222> (31)..(31)
   <223> A carboxyl group of a C-terminus is amidated.
<400> 10
   Asp Leu Ser Lys Gln Met Glu Glu Glu Ala Val Arg Leu Phe Ile Glu
   1 5 10 15
   Trp Leu Lys Asn Gly Gly Pro Ser Ser Gly Ala Pro Pro Pro Ser
   20 25 30
<210> 11
   <211> 31
   <212> PRT
   <213> Artificial Sequence
<220>
   <223> A peptide derivate of reference production example 1
<220>
   <221> MOD_RES
   <222> (4)..(4)
   <223> An amino group of a side chain is labeled by [125I] 3-iodobenzoyl.
<220>
   <221> MOD_RES
   <222> (31)..(31)
   <223> A carboxyl group of a C-terminus is amidated.
<400> 11
   Asp Leu Ser Lys Gln Met Glu Glu Glu Ala Val Arg Leu Phe Ile Glu
   1 5 10 15
   Trp Leu Lys Asn Gly Gly Pro Ser Ser Gly Ala Pro Pro Pro Ser
   20 25 30
<210> 12
   <211> 31
   <212> PRT
   <213> Artificial Sequence
<220>
   <223> A precursor of a peptide derivate of reference production example 1
<220>
   <221> MOD_RES
   <222> (1)..(1)
   <223> An amino group of an N-terminus is protected by a Fmoc
<220>
   <221> MOD_RES
   <222> (19)..(19)
   <223> An amino group of a side chain is protected by a Fmoc.
<220>
   <221> MOD_RES
   <222> (31)..(31)
   <223> A carboxyl group of a C-terminus is amidated.
<400> 12
   Asp Leu Ser Lys Gln Met Glu Glu Glu Ala Val Arg Leu Phe Ile Glu
   1               5                   10                  15
   Trp Leu Lys Asn Gly Gly Pro Ser Ser Gly Ala Pro Pro Pro Ser
               20                  25                  30
<210> 13
   <211> 32
   <212> PRT
   <213> Artificial Sequence
<220>
   <223> A peptide derivate of formula (13)
<220>
   <221> MOD_RES
   <222> (32)..(32)
   <223> An amino group of a side chain is bound to [1251] IB-PEG3. A carboxyl group of a C-terminus is amidated.
<400> 13
   Asp Leu Ser Lys Gln Met Glu Glu Glu Ala Val Arg Leu Phe Ile Glu
   1               5                   10                  15
   Trp Leu Lys Asn Gly Gly Pro Ser Ser Gly Ala Pro Pro Pro Ser Lys
               20                  25                  30
<210> 14
   <211> 32
   <212> PRT
   <213> Artificial Sequence
<220>
   <223> A protected peptide resin B
<220>
   <221> MOD_RES
   <222> (1)..(1)
   <223> An alpha-amino group of an N-terminus is protected by a Fmoc. A functional group of a side chain is protected by a OBu.
<220>
   <221> MOD_RES
   <222> (3)..(3)
   <223> A functional group of a side chain is protected by a OBu.
<220>
   <221> MOD_RES
   <222> (4)..(4)
   <223> An amino group of a side chain is protected by a Fmoc.
<220>
   <221> MOD_RES
   <222> (5)..(5)
   <223> A functional group of a side chain is protected by a Trt.
<220>
   <221> MOD_RES
   <222> (7)..(9)
   <223> A functional group of a side chain is protected by a OBu.
<220>
   <221> MOD_RES
   <222> (12)..(12)
   <223> A functional group of a side chain is protected by a Pdf.
<220>
   <221> MOD_RES
   <222> (16)..(16)
   <223> A functional group of a side chain is protected by a OBu.
<220>
   <221> MOD_RES
   <222> (17)..(17)
   <223> A functional group of a side chain is protected by a Boc.
<220>
   <221> MOD_RES
   <222> (19)..(19)
   <223> An amino group of a side chain is protected by a Fmoc.
<220>
   <221> MOD_RES
   <222> (20)..(20)
   <223> A functional group of a side chain is protected by a Trt.
<220>
   <221> MOD_RES
   <222> (24)..(25)
   <223> A functional group of a side chain is protected by a OBu.
<220>
   <221> MOD_RES
   <222> (32)..(32)
   <223> An amino group of a side chain is bound to Boc-PEG3. A carboxyl group of a C-terminus is bound to Rink Amide MBHA.
<400> 14
   Asp Leu Ser Lys Gln Met Glu Glu Glu Ala Val Arg Leu Phe Ile Glu
   1               5                   10                  15
   Trp Leu Lys Asn Gly Gly Pro Ser Ser Gly Ala Pro Pro Pro Ser Lys
               20                  25                  30
<210> 15
   <211> 32
   <212> PRT
   <213> Artificial Sequence
<220>
   <223> A peptide derivate of formula (15)
<220>
   <221> MOD_RES
   <222> (1)..(1)
   <223> An amino group of an N-terminus is protected by a Fmoc
<220>
   <221> MOD_RES
   <222> (4)..(4)
   <223> An amino group of a side chain is bound to a Fmoc.
<220>
   <221> MOD_RES
   <222> (19)..(19)
   <223> An amino group of a side chain is bound to a Fmoc.
<220>
   <221> MOD_RES
   <222> (32)..(32)
   <223> An amino group of a side chain is bound to PEG3. A carboxyl group of a C-terminus is amidated.
<400> 15
   Asp Leu Ser Lys Gln Met Glu Glu Glu Ala Val Arg Leu Phe Ile Glu
   1               5                   10                  15
   Trp Leu Lys Asn Gly Gly Pro Ser Ser Gly Ala Pro Pro Pro Ser Lys
               20                  25                  30
<210> 16
   <211> 32
   <212> PRT
   <213> Artificial Sequence
<220>
   <223> A peptide derivate of reference production example 2
<220>
   <221> MOD_RES
   <222> (32)..(32)
   <223> An amino group of a side chain is labeled by [125I]3-iodobenzoyl. A carboxyl group of a C-terminus is amidated.
<400> 16
   Asp Leu Ser Lys Gln Met Glu Glu Glu Ala Val Arg Leu Phe Ile Glu
   1               5                   10                  15
   Trp Leu Lys Asn Gly Gly Pro Ser Ser Gly Ala Pro Pro Pro Ser Lys
               20                  25                  30
<210> 17
   <211> 32
   <212> PRT
   <213> Artificial Sequence
<220>
   <223> A precursor of a peptide derivate of reference production example 2
<220>
   <221> MOD_RES
   <222> (1)..(1)
   <223> An amino group of an N-terminus is protected by a Fmoc
<220>
   <221> MOD_RES
   <222> (4)..(4)
   <223> An amino group of a side chain is protected by a Fmoc.
<220>
   <221> MOD_RES
   <222> (19)..(19)
   <223> An amino group of a side chain is protected by a Fmoc.
<220>
   <221> MOD_RES
   <222> (32)..(32)
   <223> A carboxyl group of a C-terminus is amidated.
<400> 17
   Asp Leu Ser Lys Gln Met Glu Glu Glu Ala Val Arg Leu Phe Ile Glu
   1               5                   10                  15
   Trp Leu Lys Asn Gly Gly Pro Ser Ser Gly Ala Pro Pro Pro Ser Lys
               20                  25                  30
<210> 18
   <211> 31
   <212> PRT
   <213> Artificial Sequence
<220>
   <223> A peptide derivate of formula (18)
<220>
   <221> MOD_RES
   <222> (4)..(4)
   <223> An amino group of a side chain is bound to [123I]IB-PEG3.
<220>
   <221> MOD_RES
   <222> (31)..(31)
   <223> A carboxyl group of a C-terminus is amidated.
<400> 18
   Asp Leu Ser Lys Gln Met Glu Glu Glu Ala Val Arg Leu Phe Ile Glu
   1               5                   10                  15
   Trp Leu Lys Asn Gly Gly Pro Ser Ser Gly Ala Pro Pro Pro Ser
               20                  25                  30
<210> 19
   <211> 32
   <212> PRT
   <213> Artificial Sequence
<220>
   <223> A peptide derivate of formula (19)
<220>
   <221> MOD_RES
   <222> (32)..(32)
   <223> An amino group of a side chain is bound to [123I]IB-PEG3. A carboxyl group of a C-terminus is amidated.
<400> 19
   Asp Leu Ser Lys Gln Met Glu Glu Glu Ala Val Arg Leu Phe Ile Glu
   1               5                   10                  15
   Trp Leu Lys Asn Gly Gly Pro Ser Ser Gly Ala Pro Pro Pro Ser Lys
               20                  25                  30
<210> 20
   <211> 31
   <212> PRT
   <213> Heloderma suspectum
<220>
   <221> MOD_RES
   <222> (31)..(31)
   <223> A carboxyl group of a C-terminus is amidated.
<400> 20
   Asp Leu Ser Lys Gln Met Glu Glu Glu Ala Val Arg Leu Phe Ile Glu
   1               5                   10                  15
   Trp Leu Lys Asn Gly Gly Pro Ser Ser Gly Ala Pro Pro Pro Ser
               20                  25                  30
<210> 21
   <211> 39
   <212> PRT
   <213> Artificial Sequence
<220>
   <223> A peptide derivate of formula (21)
<220>
   <221> MOD_RES
   <222> (12)..(12)
   <223> An amino group of a side chain is bound to [18F]FB-PEG3.
<220>
   <221> MOD_RES
   <222> (39)..(39)
   <223> A carboxyl group of a C-terminus is amidated.
<400> 21
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35
<210> 22
   <211> 39
   <212> PRT
   <213> Artificial Sequence
<220>
   <223> A peptide derivate of formula (22)
<220>
   <221> MOD_RES
   <222> (1)..(1)
   <223> An amino group of an N-terminus is protected by a Fmoc
<220>
   <221> MOD_RES
   <222> (12)..(12)
   <223> An amino group of a side chain is bound to PEG3.
<220>
   <221> MOD_RES
   <222> (27)..(27)
   <223> An amino group of a side chain is bound to Fmoc.
<220>
   <221> MOD_RES
   <222> (39)..(39)
   <223> A carboxyl group of a C-terminus is amidated.
<400> 22
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35
<210> 23
   <211> 39
   <212> PRT
   <213> Artificial Sequence
<220>
   <223> A precursor of peptide derivate of reference production example 3
<220>
   <221> MOD_RES
   <222> (1)..(1)
   <223> An amino group of an N-terminus is protected by a Fmoc
<220>
   <221> MOD_RES
   <222> (27)..(27)
   <223> An amino group of a side chain is bound to Fmoc.
<220>
   <221> MOD_RES
   <222> (39)..(39)
   <223> A carboxyl group of a C-terminus is amidated.
<400> 23
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35
<210> 24
   <211> 39
   <212> PRT
   <213> Artificial Sequence
<220>
   <223> A peptide derivate of reference production example 3
<220>
   <221> MOD_RES
   <222> (12)..(12)
   <223> An amino group of a side chain is bound to [18F]FB.
<220>
   <221> MOD_RES
   <222> (39)..(39)
   <223> A carboxyl group of a C-terminus is amidated.
<400> 24
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 25
   <211> 39
   <212> PRT
   <213> Artificial Sequence
<220>
   <223> A peptide derivate of formula (25)
<220>
   <221> MOD_RES
   <222> (12)..(12)
   <223> An amino group of a side chain is bound to [125I]IB-PEG3.
<220>
   <221> MOD_RES
   <222> (39)..(39)
   <223> A carboxyl group of a C-terminus is amidated.
<400> 25
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 26
   <211> 39
   <212> PRT
   <213> Artificial Sequence
<220>
   <223> A peptide derivate of reference production example 4
<220>
   <221> MOD_RES
   <222> (12)..(12)
   <223> An amino group of a side chain is bound to [125I]IB.
<220>
   <221> MOD_RES
   <222> (39)..(39)
   <223> A carboxyl group of a C-terminus is amidated.
<400> 26
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35
<210> 27
   <211> 40
   <212> PRT
   <213> Artificial Sequence
<220>
   <223> A peptide derivate of formula (27)
<220>
   <221> MOD_RES
   <222> (40)..(40)
   <223> An amino group of a side chain is bound to [1251]IB-PEG3. A carboxyl group of a C-terminus is amidated.
<400> 27
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser Lys
           35                  40
<210> 28
   <211> 40
   <212> PRT
   <213> Artificial Sequence
<220>
   <223> A peptide derivate of formula (28)
<220>
   <221> MOD_RES
   <222> (1)..(1)
   <223> An amino group of an N-terminus is protected by a Fmoc
<220>
   <221> MOD_RES
   <222> (12)..(12)
   <223> An amino group of a side chain is bound to a Fmoc.
<220>
   <221> MOD_RES
   <222> (27)..(27)
   <223> An amino group of a side chain is bound to a Fmoc.
<220>
   <221> MOD_RES
   <222> (40)..(40)
   <223> An amino group of a side chain is bound to PEG3. A carboxyl group of a C-terminus is amidated.
<400> 28
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser Lys
           35                  40
<210> 29
   <211> 40
   <212> PRT
   <213> Artificial Sequence
<220>
   <223> A peptide derivate of formula (29)
<220>
   <221> MOD_RES
   <222> (1)..(1)
   <223> An amino group of an N-terminus is protected by a Fmoc
<220>
   <221> MOD_RES
   <222> (12)..(12)
   <223> An amino group of a side chain is bound to a Fmoc.
<220>
   <221> MOD_RES
   <222> (27)..(27)
   <223> An amino group of a side chain is bound to a Fmoc.
<220>
   <221> MOD_RES
   <222> (40)..(40)
   <223> A carboxyl group of a C-terminus is amidated.
<400> 29
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser Lys
   35 40
<210> 30
   <211> 40
   <212> PRT
   <213> Artificial Sequence
<220>
   <223> A peptide derivate of formula (30)
<220>
   <221> MOD_RES
   <222> (40)..(40)
   <223> An amino group of a side chain is bound to [125I]IB. A carboxyl group of a C-terminus is amidated.
<400> 30
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser Lys
   35 40
<210> 31
   <211> 31
   <212> PRT
   <213> Artificial Sequence
<220>
   <223> A peptide derivate of formula (31)
<220>
   <221> MOD_RES
   <222> (4)..(4)
   <223> An amino group of a side chain is bound to IB-PEG3.
<220>
   <221> MOD_RES
   <222> (31)..(31)
   <223> A carboxyl group of a C-terminus is amidated.
<400> 31
   Asp Leu Ser Lys Gln Met Glu Glu Glu Ala Val Arg Leu Phe Ile Glu
   1               5                   10                  15
   Trp Leu Lys Asn Gly Gly Pro Ser Ser Gly Ala Pro Pro Pro Ser
               20                  25                  30
<210> 32
   <211> 32
   <212> PRT
   <213> Artificial Sequence
<220>
   <223> A peptide derivate of formula (32)
<220>
   <221> MOD_RES
   <222> (32)..(32)
   <223> An amino group of a side chain is bound to IB-PEG3. A carboxyl group of a C-terminus is amidated.
<400> 32
   Asp Leu Ser Lys Gln Met Glu Glu Glu Ala Val Arg Leu Phe Ile Glu
   1               5                   10                  15
   Trp Leu Lys Asn Gly Gly Pro Ser Ser Gly Ala Pro Pro Pro Ser Lys
               20                  25                  30
<210> 33
   <211> 39
   <212> PRT
   <213> Artificial Sequence
<220>
   <223> A peptide derivate of formula (33)
<220>
   <221> MOD_RES
   <222> (12)..(12)
   <223> An amino group of a side chain is bound to IB-PEG3.
<220>
   <221> MOD_RES
   <222> (39)..(39)
   <223> A carboxyl group of a C-terminus is amidated.
<400> 33
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35
<210> 34
   <211> 40
   <212> PRT
   <213> Artificial Sequence
<220>
   <223> A peptide derivate of formula (34)
<220>
   <221> MOD_RES
   <222> (40)..(40)
   <223> An amino group of a side chain is bound to IB-PEG3. A carboxyl group of a C-terminus is amidated.
<400> 34
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser Lys
           35                  40
<210> 35
   <211> 39
   <212> PRT
   <213> Artificial Sequence
<220>
   <223> A peptide derivate of formula (35)
<220>
   <221> MOD_RES
   <222> (12)..(12)
   <223> An amino group of a side chain is bound to [123I]IB-PEG3.
<220>
   <221> MOD_RES
   <222> (39)..(39)
   <223> A carboxyl group of a C-terminus is amidated.
<400> 35
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35
<210> 36
   <211> 40
   <212> PRT
   <213> Artificial Sequence
<220>
   <223> A peptide derivate of formula (36)
<220>
   <221> MOD_RES
   <222> (40)..(40)
   <223> An amino group of a side chain is bound to [123I]IB-PEG3. A carboxyl group of a C-terminus is amidated.
<400> 36
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser Lys
           35                  40
<210> 37
   <211> 39
   <212> PRT
   <213> Artificial Sequence
<220>
   <223> A peptide derivate of formula (37)
<220>
   <221> MOD_RES
   <222> (12)..(12)
   <223> An amino group of a side chain is bound to [125I]IB-ePEG12.
<220>
   <221> MOD_RES
   <222> (39)..(39)
   <223> A carboxyl group of a C-terminus is amidated.
<400> 37
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35
<210> 38
   <211> 39
   <212> PRT
   <213> Artificial Sequence
<220>
   <223> A peptide derivate of formula (38)
<220>
   <221> MOD_RES
   <222> (1)..(1)
   <223> An amino group of an N-terminus is protected by a Fmoc
<220>
   <221> MOD_RES
   <222> (12)..(12)
   <223> An amino group of a side chain is bound to ePEG12.
<220>
   <221> MOD_RES
   <222> (27)..(27)
   <223> An amino group of a side chain is bound to Fmoc.
<220>
   <221> MOD_RES
   <222> (39)..(39)
   <223> A carboxyl group of a C-terminus is amidated.
<400> 38
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35
<210> 39
   <211> 39
   <212> PRT
   <213> Artificial Sequence
<220>
   <223> A peptide derivate of formula (39)
<220>
   <221> MOD_RES
   <222> (12)..(12)
   <223> An amino group of a side chain is bound to [123I]IB-ePEG12.
<220>
   <221> MOD_RES
   <222> (39)..(39)
   <223> A carboxyl group of a C-terminus is amidated.
<400> 39
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35